(19) Europäisches Patentamt
European Patent Office
Office européen des brevets

(11) EP 3 916 110 A1

(12) EUROPEAN PATENT APPLICATION

(43) Date of publication:
01.12.2021 Bulletin 2021/48

(21) Application number: 21185798.2

(22) Date of filing: 14.07.2015

(51) Int Cl.:
*C12Q 1/6886 (2018.01)* *G01N 33/574 (2006.01)*
*C40B 40/08 (2006.01)*

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 14.07.2014 US 201462024456 P
12.05.2015 US 201562160403 P

(62) Document number(s) of the earlier application(s) in accordance with Art. 76 EPC:
15822338.8 / 3 169 814

(71) Applicant: Veracyte, Inc.
South San Francisco, CA 94080 (US)

(72) Inventors:
- WHITNEY, Duncan H.
Sudbury, MA 01776 (US)
- ELASHOFF, Michael
Redwood City, CA 94065 (US)

(74) Representative: Avidity IP
Broers Building
Hauser Forum
21 JJ Thomson Avenue
Cambridge CB3 0FA (GB)

Remarks:
This application was filed on 15.07.2021 as a divisional application to the application mentioned under INID code 62.

(54) METHODS FOR EVALUATING LUNG CANCER STATUS

(57) The disclosure in some aspects provides methods of determining the likelihood that a subject has lung cancer based on the expression of informative-genes. In other aspects, the disclosure provides methods for determining an appropriate diagnostic intervention plan for a subject based on the expression of informative-genes. Related compositions and kits are provided in other aspects of the disclosure.

FIGURE 1

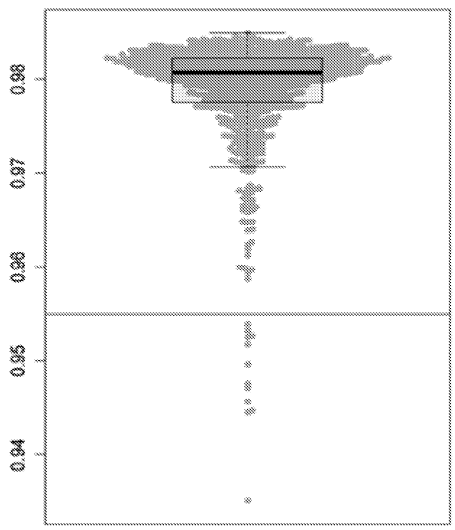


EP 3 916 110 A1

SEQUENCE LISTING

<110> Whitney, Duncan H
Elashoff, Michael

<120> METHODS FOR EVALUATING LUNG CANCER STATUS

<130> VRCT-008/02US

<150> US 62/024,456
<151> 2014-07-14

<150> US 62/160,403
<151> 2015-05-12

<160> 46

<170> PatentIn version 3.5

<210> 1
<211> 25
<212> DNA
<213> Artificial Sequence

<220>
<223> TSPAN2 Probe Sequence

<400> 1
tcaacattaa gaagtcttaa ttcag 25

<210> 2
<211> 25
<212> DNA
<213> Artificial Sequence

<220>
<223> MCAM Probe Sequence

<400> 2
gctttaatcc ccatgaagga cagtg 25

<210> 3
<211> 25
<212> DNA
<213> Artificial Sequence

<220>
<223> ATP12A Probe Sequence

<400> 3
gcggtggaga taccggcggc ggcgc 25

<210> 4
<211> 25
<212> DNA
<213> Artificial Sequence

<220>
<223> NOVA1 Probe Sequence

<400> 4
gacgaaattc agacatggag catca 25

<210> 5
<211> 25
<212> DNA
<213> Artificial Sequence

<220>
<223> MIA Probe Sequence

<400> 5
atgacaccaa ggcacaccag ggacc 25

<210> 6
<211> 25
<212> DNA
<213> Artificial Sequence

<220>
<223> CD177.1 Probe Sequence

<400> 6
gacccgtctg tggctggtaa tctct 25

<210> 7
<211> 25
<212> DNA
<213> Artificial Sequence

<220>
<223> EPHX3 Probe Sequence

<400> 7
gctccactgg aagagaggta taccc 25

<210> 8
<211> 25
<212> DNA
<213> Artificial Sequence

<220>
<223> CD177.2 Probe Sequence

<400> 8
ttcctgtgtc ccattgagca ggttg 25

<210> 9
<211> 25
<212> DNA
<213> Artificial Sequence

<220>
<223> CGREF1

<400> 9
tagggtacag cacttaacgc aatct 25

<210> 10
<211> 25
<212> DNA
<213> Artificial Sequence

<220>
<223> BST1 Probe Sequence

<400> 10
tgtttgccgt ttcccgttcc agaca 25

<210> 11
<211> 25
<212> DNA
<213> Artificial Sequence

<220>
<223> RNF150 Probe Sequence

<400> 11
tggttaatcc aagccgcagc ctggt 25

<210> 12
<211> 25
<212> DNA
<213> Artificial Sequence

<220>
<223> CLDN22 Probe Sequence

<400> 12
ggtgtttctc gtctccagtt cttga 25

<210> 13
<211> 25
<212> DNA
<213> Artificial Sequence

<220>
<223> GABBR1 Probe Sequence

<400> 13
tacggagcca ttacctgggc agtgc 25

<210> 14
<211> 25
<212> DNA
<213> Artificial Sequence

<220>
<223> SDC2 Probe Sequence

<400> 14
tgagcctgct tctccgggct cccct 25

<210> 15
<211> 25

<212> DNA
<213> Artificial Sequence

<220>
<223> NKX3-1 Probe Sequence

<400> 15
tttgtgctgg ctagtactcc ggtcg 25

<210> 16
<211> 25
<212> DNA
<213> Artificial Sequence

<220>
<223> LYPD2 Probe Sequence

<400> 16
ttcttcaagg cattcggggc tgggc 25

<210> 17
<211> 25
<212> DNA
<213> Artificial Sequence

<220>
<223> CDR1

<400> 17
aacaactccg ggtcttccag cgact 25

<210> 18
<211> 25
<212> DNA
<213> Artificial Sequence

<220>
<223> RPS4Y1 Probe Sequence

<400> 18
taaaccgcag gaagtcagat gagtg 25

<210> 19
<211> 25
<212> DNA
<213> Artificial Sequence

<220>
<223> SLC7A11

<400> 19
ggttgaagca actagaagcg tgaca 25

<210> 20
<211> 25
<212> DNA
<213> Artificial Sequence

<220>
<223>  CLDN10 Probe Sequence

<400>  20
gacagcgttt catgctcgga tggcc                                          25

<210>  21
<211>  25
<212>  DNA
<213>  Artificial Sequence

<220>
<223>  TKT Probe Sequence

<400>  21
ggtttattct ctccagacgg tcagg                                          25

<210>  22
<211>  25
<212>  DNA
<213>  Artificial Sequence

<220>
<223>  RUNX1T1 Probe Sequence

<400>  22
taacagggag gaggtcaaat ctatc                                          25

<210>  23
<211>  25
<212>  DNA
<213>  Artificial Sequence

<220>
<223>  AKR1C2 Probe Sequence

<400>  23
tagctgtagc ttactgaagt cgcca                                          25

<210>  24
<211>  25
<212>  DNA
<213>  Artificial Sequence

<220>
<223>  CD177.1 Probe Sequence

<400>  24
gacgtgaacc agaccaatga agggc                                          25

<210>  25
<211>  25
<212>  DNA
<213>  Artificial Sequence

<220>
<223>  CD177.1 Probe Sequence

<400> 25
tcccataggg caagtccgga tgctc 25

<210> 26
<211> 25
<212> DNA
<213> Artificial Sequence

<220>
<223> CD177.1 Probe Sequence

<400> 26
agcaggaagg gcaaaccact cccca 25

<210> 27
<211> 25
<212> DNA
<213> Artificial Sequence

<220>
<223> CD177.1 Probe Sequence

<400> 27
ccaagtgaga gactccaggc cctca 25

<210> 28
<211> 25
<212> DNA
<213> Artificial Sequence

<220>
<223> CD177.1 Probe Sequence

<400> 28
aggaggctgc acatcacgct tctca 25

<210> 29
<211> 25
<212> DNA
<213> Artificial Sequence

<220>
<223> CD177.1 Probe Sequence

<400> 29
ggaggctgca catcacgctt ctcac 25

<210> 30
<211> 25
<212> DNA
<213> Artificial Sequence

<220>
<223> CD177.1 Probe Sequence

<400> 30
acaagaagct ggaaggttgg gccac 25

<210> 31
<211> 25
<212> DNA
<213> Artificial Sequence

<220>
<223> CD177.1 Probe Sequence

<400> 31
aatgctcatt ttggtggaca gccca 25

<210> 32
<211> 25
<212> DNA
<213> Artificial Sequence

<220>
<223> CD177.1 Probe Sequence

<400> 32
ctacatctag gagcagcagc gtctc 25

<210> 33
<211> 25
<212> DNA
<213> Artificial Sequence

<220>
<223> CD177.1 Probe Sequence

<400> 33
agcagcgtct cctgacacac ctgcc 25

<210> 34
<211> 25
<212> DNA
<213> Artificial Sequence

<220>
<223> CD177.1 Probe Sequence

<400> 34
tcttgagcca agtttccgtg tgtca 25

<210> 35
<211> 25
<212> DNA
<213> Artificial Sequence

<220>
<223> CD177.1 Probe Sequence

<400> 35
tccgtgtgtc ataatggtgg tcccc 25

<210> 36
<211> 25

<212> DNA
<213> Artificial Sequence

<220>
<223> CD177.1 Probe Sequence

<400> 36
gttaacgagg ttgttgcaga agtcc 25

<210> 37
<211> 25
<212> DNA
<213> Artificial Sequence

<220>
<223> CD177.1 Probe Sequence

<400> 37
ttggagagca ccaggctcac ttggg 25

<210> 38
<211> 25
<212> DNA
<213> Artificial Sequence

<220>
<223> CD177.1 Probe Sequence

<400> 38
tctcaatgag catcaacgtg tcctg 25

<210> 39
<211> 25
<212> DNA
<213> Artificial Sequence

<220>
<223> CD177.1 Probe Sequence

<400> 39
gcaggtcgga caccttccac acatg 25

<210> 40
<211> 25
<212> DNA
<213> Artificial Sequence

<220>
<223> CD177.1 Probe Sequence

<400> 40
agggccagca gtaataccgc gctca 25

<210> 41
<211> 25
<212> DNA
<213> Artificial Sequence

<220>
<223> CD177.1 Probe Sequence

<400> 41
gggccagcag taataccgcg ctcat 25

<210> 42
<211> 25
<212> DNA
<213> Artificial Sequence

<220>
<223> CD177.1 Probe Sequence

<400> 42
gacccgtctg tggctggtaa tctct 25

<210> 43
<211> 25
<212> DNA
<213> Artificial Sequence

<220>
<223> CD177.2 Probe Sequence

<400> 43
ttcctgtgtc ccattgagca ggttg 25

<210> 44
<211> 25
<212> DNA
<213> Artificial Sequence

<220>
<223> CD177.2 Probe Sequence

<400> 44
tcccattgag caggttgcaa actgg 25

<210> 45
<211> 25
<212> DNA
<213> Artificial Sequence

<220>
<223> CD177.2 Probe Sequence

<400> 45
cctgaggagg ccatcataac agtgt 25

<210> 46
<211> 25
<212> DNA
<213> Artificial Sequence

<220>
<223> CD177.2 Probe Sequence

<400> 46
aggccatcat aacagtgtgt ggtcc 25

## Description

### CROSS REFERENCE TO RELATED APPLICATIONS

The present application claims priority to U.S. Provisional Application Nos. 62/024,456, filed on July 14, 2014, and 62/160,403, filed May 12, 2015, the entire contents of which are hereby incorporated by reference in their entirety for all purposes.

### DESCRIPTION OF THE TEXT FILE SUBMITTED ELECTRONICALLY

The contents of the text file submitted electronically are incorporated herein by reference in their entirety: A computer readable format copy of the Sequence Listing (filename: VRCT-008_02WO_ST25.txt, date recorded: July 14, 2015; file size: 9 kilobytes).

### FIELD OF THE DISCLOSURE

The present disclosure generally relates to methods and compositions for assessing cancer using gene expression information.

### BACKGROUND OF THE DISCLOSURE

A challenge in diagnosing lung cancer, particularly at an early stage where it can be most effectively treated, is gaining access to cells to diagnose disease. Early stage lung cancer is typically associated with small lesions, which may also appear in the peripheral regions of the lung airway, which are particularly difficult to reach by standard techniques such as bronchoscopy.

### SUMMARY OF THE INVENTION

Provided herein are methods for establishing appropriate diagnostic intervention plans and/or treatment plans for subjects, and for aiding healthcare providers in establishing appropriate diagnostic intervention plans and/or treatment plans. In some embodiments, the methods are based on an airway field of injury concept. In some embodiments, the methods involve establishing lung cancer risk scores based on expression levels of informative-genes that are useful for assessing the likelihood that a subject has cancer. In some embodiments, methods provided herein involve making an assessment based on expression levels of informative-genes in a biological sample obtained from a subject during a routine cell or tissue sampling procedure. In some embodiments, the biological sample comprises histologically normal cells. In some embodiments, aspects of the disclosure are based, at least in part, on a determination that expression levels of certain informative-genes in apparently histologically normal cells obtained from a first airway locus can be used to evaluate the likelihood of cancer at a second locus in the airway (for example, at a locus in the airway that is remote from the locus at which the histologically normal cells were sampled). In some embodiments, sampling of histologically normal cells (e.g., cells of the bronchus) is advantageous because tissues containing such cells are generally readily available, and thus it is possible to reproducibly obtain useful samples compared with procedures that involve obtaining tissues of suspicious lesions which may be much less reproducibly sampled. In some embodiments, the methods involve making a lung cancer assessment based on expression levels of informative-genes in cytologically normal appearing cells collected from the bronchi of a subject. In some embodiments, informative-genes useful for predicting the likelihood of lung cancer are provided in Tables 1, 11, and 26.

According to some aspects of the disclosure methods are provided of determining the likelihood that a subject has lung cancer that involve subjecting a biological sample obtained from a subject to a gene expression analysis, in which the gene expression analysis comprises determining mRNA expression levels in the biological sample of one or more informative-genes that relate to lung cancer status (e. g., an informative gene selected from Table 11). In some embodiments, the methods comprise determining mRNA expression levels in the biological sample of one or more genomic correlate genes that relate to one or more self-reportable characteristics of the subject. In some embodiments, the methods further comprise transforming expression levels determined above into a lung cancer risk-score that is indicative of the likelihood that the subject has lung cancer. In some embodiments, the one or more self-reportable characteristics of the subject are selected from: smoking pack years, smoking status, age and gender. In some embodiments, a lung cancer-risk score is determined according to a model having a Negative Predictive Value (NPV) of greater than 90% for ruling out lung cancer in an intended use population. In some embodiments, a lung cancer-risk score is determined according to a model having a Negative Predictive Value (NPV) of greater than 85% for subjects diagnosed with COPD.

In some embodiments, appropriate diagnostic intervention plans are established based at least in part on the lung cancer risk scores. In some embodiments, the methods assist health care providers with making early and accurate diagnoses. In some embodiments, the methods assist health care providers with establishing appropriate therapeutic interventions early on in patient clinical evaluations. In some embodiments, the methods involve evaluating biological samples obtained during bronchoscopic procedures. In some embodiments, the methods are beneficial because they enable health care providers to make informative decisions regarding patient diagnosis and/or treatment from otherwise uninformative bronchoscopies. In some embodiments, the risk or likelihood assessment leads to appropriate surveillance for monitoring low risk lesions. In some embodiments, the risk or likelihood assessment leads to faster diagnosis, and thus, faster therapy for certain cancers.

Certain methods described herein, alone or in combination with other methods, provide useful information for health care providers to assist them in making diagnostic and therapeutic decisions for a patient. Certain methods disclosed herein are employed in instances where other methods have failed to provide useful information regarding the lung cancer status of a patient. Certain methods disclosed herein provide an alternative or complementary method for evaluating or diagnosing cell or tissue samples obtained during routine bronchoscopy procedures, and increase the likelihood that the procedures will result in useful information for managing a patient's care. The methods disclosed herein are highly sensitive, and produce information regarding the likelihood that a subject has lung cancer from cell or tissue samples (e.g., histologically normal tissue) that may be obtained from positions remote from malignant lung tissue. Certain methods described herein can be used to assess the likelihood that a subject has lung cancer by evaluating histologically normal cells or tissues obtained during a routine cell or tissue sampling procedure (e.g., ancillary bronchoscopic procedures such as brushing, such as by cytobrush; biopsy; lavage; and needle-aspiration). However, it should be appreciated that any suitable tissue or cell sample can be used. Often the cells or tissues that are assessed by the methods appear histologically normal. In some embodiments, the subject has been identified as a candidate for bronchoscopy and/or as having a suspicious lesion in the respiratory tract.

In some embodiments, the methods disclosed herein are useful because they enable health care providers to determine appropriate diagnostic intervention and/or treatment plans by balancing the risk of a subject having lung cancer with the risks associated with certain invasive diagnostic procedures aimed at confirming the presence or absence of the lung cancer in the subject. In some embodiments, an objective is to align subjects with low probability of disease with interventions that may not be able to rule out cancer but are lower risk.

According to some aspects of the disclosure, methods are provided for evaluating the lung cancer status of a subject using gene expression information that involve one or more of the following acts: (a) obtaining a biological sample from the respiratory tract of a subject, wherein the subject has been referred for bronchoscopy (e.g., has been identified as having a suspicious lesion in the respiratory tract and therefore referred for bronchoscopy to evaluate the lesion), (b) subjecting the biological sample to a gene expression analysis, in which the gene expression analysis comprises determining the expression levels of a plurality of informative-genes in the biological sample, (c) computing a lung cancer risk score based on the expression levels of the plurality of informative-genes, (d) determining that the subject is in need of a first diagnostic intervention to evaluate lung cancer status, if the level of the lung cancer risk score is beyond (e.g., above) a first threshold level, and (e) determining that the subject is in need of a second diagnostic intervention to evaluate lung cancer status, if the level of the lung cancer risk score is beyond (e.g., below) a second threshold level. In some embodiments, the methods further comprise (f) determining that the subject is in need of a third diagnostic intervention to evaluate lung cancer status, if the level of the lung cancer risk score is between the first threshold and the second threshold levels.

In particular embodiments, the approaches herein may be used when a subject was referred for bronchoscopy and the bronchoscopy procedure resulted in indeterminate or non-diagnostic information. Accordingly, disclosed herein are methods for assigning such subjects to a low-risk, including one or more of steps (a) obtaining a biological sample from the respiratory tract of the subject, wherein the subject has undergone a non-diagnostic bronchoscopy procedure, (b) subjecting the biological sample to a gene expression analysis, in which the gene expression analysis comprises determining the expression levels of a plurality of informative-genes in the biological sample, (c) computing a lung cancer risk score based on the expression levels of the plurality of informative-genes, and (d) determining that the subject is a low risk of lung cancer, if the level of the lung cancer risk score is beyond (e.g., below) a first threshold level, and optionally, (e) assigning the low-risk subjects to one or more non-invasive follow-up procedures; CT surveillance, for example. Such approaches allow a population of subjects to avoid subsequent invasive approaches. For subjects who are not below the threshold level, traditional approaches following a non-diagnostic bronchoscopy may be followed.

In some embodiments, the first diagnostic intervention comprises performing a transthoracic needle aspiration, mediastinoscopy or thoracotomy. In some embodiments, the second diagnostic intervention comprises engaging in watchful waiting (e.g., periodic monitoring). In some embodiments, watchful waiting comprises periodically imaging the respiratory tract to evaluate the suspicious lesion. In some embodiments, watchful waiting comprises periodically imaging the respiratory tract to evaluate the suspicious lesion for up to one year, two years, four years, five years or more. In some embodiments, watchful waiting comprises imaging the respiratory tract to evaluate the suspicious lesion at least once per year. In some embodiments, watchful waiting comprises imaging the respiratory tract to evaluate the suspicious lesion at least twice per year. In some embodiments, watchful waiting comprises periodic monitoring of a subject unless and until the subject is diagnosed as being free of cancer. In some embodiments, watchful waiting comprises periodic monitoring of a subject unless and until the subject is diagnosed as having cancer. In some embodiments, watchful waiting comprises periodically repeating one or more of steps (a) to (f) noted in the preceding paragraph. In some embodiments, the third diagnostic intervention comprises performing a bronchoscopy procedure. In some embodiments, the third diagnostic intervention comprises repeating steps (a) to (e) noted in the preceding paragraph. In certain embodiments, the third diagnostic intervention comprises repeating steps (a) to (e) within six months of determining that the lung cancer risk score is between the first threshold and the second threshold levels. In certain embodiments, the third diagnostic intervention comprises repeating steps (a) to (e) within three months of determining that the lung cancer risk score is between the first threshold and the second threshold levels. In some embodiments, the third diagnostic intervention comprises repeating steps (a) to (e) within one month of determining that the lung cancer risk score is between the first threshold and the second threshold levels.

In some embodiments, the plurality of informative-genes is selected from the group of genes in Table 11. In some embodiments, the expression levels of a subset of these genes are evaluated and compared to reference expression levels (e.g., for normal patients that do not have cancer). In some embodiments, the subset includes a) genes for which an increase in expression is associated with lung cancer or an increased risk for lung cancer, b) genes for which a decrease in expression is associated with lung cancer or an increased risk for lung cancer, or both. In some embodiments, at least 5%, at least 10%, at least 20%, at least 30%, at least 40%, or about 50% of the genes in a subset have an increased level of expression in association with an increased risk for lung cancer. In some embodiments, at least 5%, at least 10%, at least 20%, at least 30%, at least 40%, or about 50% of the genes in a subset have a decreased level of expression in association with an increased risk for lung cancer. In some embodiments, an expression level is evaluated (e.g., assayed or otherwise interrogated) for each of 10-80 or more genes (e.g., 5-10, 10-20, 20-30, 30-40, 40-50, 50-60, 60-70, 70-80, about 10, about 15, about 17, about 25, about 35, about 45, about 55, about 65, about 75, or more genes) selected from the genes in Table 11. In some embodiments, expression levels for one or more control genes also are evaluated (e.g., 1, 2, 3, 4, or 5 control genes). It should be appreciated that an assay can also include other genes, for example reference genes or other gene (regardless of how informative they are). However, if the expression profile for any of the informative-gene subsets described herein is indicative of an increased risk for lung cancer, then an appropriate therapeutic or diagnostic recommendation can be made as described herein.

In some embodiments, the identification of changes in expression level of one or more subsets of genes from Table 11 can be provided to a physician or other health care professional in any suitable format. In some embodiments, these gene expression profiles and/or results of a prediction model disclosed herein alone may be sufficient for making a diagnosis, providing a prognosis, or for recommending further diagnosis or a particular treatment. However, in some embodiments gene expression profiles and/or results of a prediction model disclosed herein may assist in the diagnosis, prognosis, and/or treatment of a subject along with other information (e.g., other expression information, and/or other physical or chemical information about the subject, including family history).

In some embodiments, a subject is identified as having a suspicious lesion in the respiratory tract by imaging the respiratory tract. In certain embodiments, imaging the respiratory tract comprises performing computer-aided tomography, magnetic resonance imaging, ultrasonography or a chest X-ray.

Methods are provided, in some embodiments, for obtaining biological samples from patients. Expression levels of informative-genes in these biological samples provide a basis for assessing the likelihood that the patient has lung cancer. Methods are provided for processing biological samples. In some embodiments, the processing methods ensure RNA quality and integrity to enable downstream analysis of informative-genes and ensure quality in the results obtained. Accordingly, various quality control steps (e.g., RNA size analyses) may be employed in these methods. Methods are provided for packaging and storing biological samples. Methods are provided for shipping or transporting biological samples, e.g., to an assay laboratory where the biological sample may be processed and/or where a gene expression analysis may be performed. Methods are provided for performing gene expression analyses on biological samples to determine the expression levels of informative-genes in the samples. Methods are provided for analyzing and interpreting the results of gene expression analyses of informative-genes. Methods are provided for generating reports that summarize the results of gene expression analyses, and for transmitting or sending assay results and/or assay interpretations to a health care provider (e.g., a physician). Furthermore, methods are provided for making treatment decisions based on the gene expression assay results, including making recommendations for further treatment or invasive diagnostic procedures.

In some embodiments, aspects of the disclosure relate to determining the likelihood that a subject has lung cancer, by subjecting a biological sample obtained from a subject to a gene expression analysis, wherein the gene expression analysis comprises determining expression levels in the biological sample of at least one informative-genes (e.g., at least two genes selected from Table 11), and using the expression levels to assist in determining the likelihood that the subject has lung cancer.

In some embodiments, the step of determining comprises transforming the expression levels into a lung cancer risk-score that is indicative of the likelihood that the subject has lung cancer. In some embodiments, the lung cancer risk-score is the combination of weighted expression levels. In some embodiments, the lung cancer risk-score is the sum of weighted expression levels. In some embodiments, the expression levels are weighted by their relative contribution to predicting increased likelihood of having lung cancer

In some embodiments, aspects of the disclosure relate to determining a treatment course for a subject, by subjecting a biological sample obtained from the subject to a gene expression analysis, wherein the gene expression analysis comprises determining the expression levels in the biological sample of at least two informative-genes (e.g., at least two mRNAs selected from Table 11), and determining a treatment course for the subject based on the expression levels. In some embodiments, the treatment course is determined based on a lung cancer risk-score derived from the expression levels. In some embodiments, the subject is identified as a candidate for a lung cancer therapy based on a lung cancer risk-score that indicates the subject has a relatively high likelihood of having lung cancer. In some embodiments, the subject is identified as a candidate for an invasive lung procedure based on a lung cancer risk-score that indicates the subject has a relatively high likelihood of having lung cancer. In some embodiments, the invasive lung procedure is a transthoracic needle aspiration, mediastinoscopy or thoracotomy. In some embodiments, the subject is identified as not being a candidate for a lung cancer therapy or an invasive lung procedure based on a lung cancer risk-score that indicates the subject has a relatively low likelihood of having lung cancer. In some embodiments, a report summarizing the results of the gene expression analysis is created. In some embodiments, the report indicates the lung cancer risk-score.

In some embodiments, aspects of the disclosure relate to determining the likelihood that a subject has lung cancer by subjecting a biological sample obtained from a subject to a gene expression analysis, wherein the gene expression analysis comprises determining the expression levels in the biological sample of at least one informative-gene (e.g., at least one informative-mRNA selected from Table 11), and determining the likelihood that the subject has lung cancer based at least in part on the expression levels.

In some embodiments, aspects of the disclosure relate to determining the likelihood that a subject has lung cancer, by subjecting a biological sample obtained from the respiratory epithelium of a subject to a gene expression analysis, wherein the gene expression analysis comprises determining the expression level in the biological sample of at least one informative-gene (e.g., at least one informative-mRNA selected from Table 11), and determining the likelihood that the subject has lung cancer based at least in part on the expression level, wherein the biological sample comprises histologically normal tissue.

In some embodiments, aspects of the disclosure relate to a computer-implemented method for processing genomic information, by obtaining data representing expression levels in a biological sample of at least two informative-genes (e.g., at least two informative-mRNAs from Table 11), wherein the biological sample was obtained of a subject, and using the expression levels to assist in determining the likelihood that the subject has lung cancer. A computer-implemented method can include inputting data via a user interface, computing (e.g., calculating, comparing, or otherwise analyzing) using a processor, and/or outputting results via a display or other user interface.

In some embodiments, the step of determining comprises calculating a risk-score indicative of the likelihood that the subject has lung cancer. In some embodiments, computing the risk-score involves determining the combination of weighted expression levels (e.g., expression levels of one or more informative-genes alone or together with one of more genomic correlate genes), in which the expression levels are weighted by their relative contribution to predicting increased likelihood of having lung cancer. In some embodiments, genomic correlate genes are genes related to or correlated with specific clinical variables (e.g., self-reportable variables). In some embodiments, such clinical variables are correlated with cancer, e.g., lung cancer. In some embodiments, rather than using expression levels of genes, groups of related genes that vary collinearly (e.g., are correlated with one another) within a population of subjects may be combined or collapsed into a single value (e.g., the mean value of a group of related genes). In some embodiments, a computer-implemented method comprises generating a report that indicates the risk-score. In some embodiments, the report is transmitted to a health care provider of the subject.

In some embodiments, a computer-implemented method comprises obtaining data representting expression levels in a biological sample of at least 2, 3, 4, 5, 6, 7, 8, 9, or 10 genes selected from the set of genes identified in cluster 1, 2, 3, 4, 5, 6, 7, 8, 9, 10, or 11 in table 11. In some embodiments, the genes comprise MYOT.

It should be appreciated that in any embodiment or aspect described herein, a biological sample can be obtained from the respiratory epithelium of the subject. The respiratory epithelium can be of the mouth, nose, pharynx, trachea, bronchi, bronchioles, or alveoli. However, other sources of respiratory epithelium also can be used. The biological sample can comprise histologically normal tissue. The biological sample can be obtained using bronchial brushings, such as cytobrush or histobrush; broncho-alveolar lavage; bronchial biopsy; oral washings; touch preps; fine needle aspirate; or sputum collection. The subject can exhibit one or more symptoms of lung cancer and/or have a lesion that is observable by computer-aided tomography or chest X-ray. In some cases, the subject has not been diagnosed with primary lung cancer prior to being evaluating by methods disclosed herein.

In any of the embodiments or aspects described herein, the expression levels can be determined using a quantitative reverse transcription polymerase chain reaction, a bead-based nucleic acid detection assay or an oligonucleotide array assay (e.g., a microarray assay) or other technique.

In any of the embodiments or aspects described herein, the lung cancer can be a adenocarcinoma, squamous cell carcinoma, small cell cancer or non-small cell cancer.

In some embodiments, aspects of the disclosure relate to a composition consisting essentially of at least one nucleic acid probe, wherein each of the at least one nucleic acid probes specifically hybridizes with an informative-gene (e.g., at least one informative-mRNA selected from Table 11).

In some embodiments, aspects of the disclosure relate to a composition comprising up to 5, up to 10, up to 25, up to 50, up to 100, or up to 200 nucleic acid probes, wherein each of the nucleic acid probes specifically hybridizes with an informative-gene (e.g., at least one informative-mRNA selected from Table 1 or 11).

In some embodiments, a composition comprises at least 1, 2, 3, 4, 5, 6, 7, 8, 9, or 10 nucleic acid probes. In some embodiments, at least 2, 3, 4, 5, 6, 7, 8, 9, or 10 of the nucleic acid probes hybridize with an mRNA expressed from a different gene selected from clusters 1, 2, 3, 4, 5, 6, 7, 8, 9, 10, or 11 of Table 11.

In some embodiments, nucleic acid probes are conjugated directly or indirectly to a bead. In some embodiments, the bead is a magnetic bead. In some embodiments, the nucleic acid probes are immobilized to a solid support. In some embodiments, the solid support is a glass, plastic or silicon chip.

In some embodiments, aspects of the disclosure relate to a kit comprising at least one container or package housing any nucleic acid probe composition described herein.

In some embodiments, expression levels are determined using a quantitative reverse transcription polymerase chain reaction.

In some embodiments, aspects of the disclosure relate to genes for which expression levels can be used to determine the likelihood that a subject (e.g., a human subject) has lung cancer. In some embodiments, the expression levels (e.g., mRNA levels) of one or more genes described herein can be determined in airway samples (e.g., epithelial cells or other samples obtained during a bronchoscopy or from an appropriate bronchial lavage samples). In some embodiments, the patterns of increased and/or decreased mRNA expression levels for one or more subsets of informative-genes (e.g., 1-5, 5-10, 10-15, 15-20, 20-25, 25-50, 50-80, or more genes) described herein can be determined and used for diagnostic, prognostic, and/or therapeutic purposes. It should be appreciated that one or more expression patterns described herein can be used alone, or can be helpful along with one or more additional patient-specific indicia or symptoms, to provide personalized diagnostic, prognostic, and/or therapeutic predictions or recommendations for a patient. In some embodiments, sets of informative-genes that distinguish smokers (current or former) with and without lung cancer are provided that are useful for predicting the risk of lung cancer with high accuracy. In some embodiments, the informative-genes are selected from Table 1 or 11.

In some embodiments, methods provided herein for determining the likelihood that a subject has lung cancer involve subjecting a biological sample obtained from a subject to a gene expression analysis that comprises determining mRNA expression levels in the biological sample of one or more informative-genes that relate to lung cancer status (e.g., an informative gene selected from Table 1 or 11). In some embodiments, the methods comprise determining mRNA expression levels in the biological sample of one or more genomic correlate genes that relate to one or more self-reportable characteristics of the subject. In some embodiments, the methods further comprise transforming the expression levels determined above into a lung cancer risk-score that is indicative of the likelihood that the subject has lung cancer. In some embodiments, the one or more self-reportable characteristics of the subject are selected from: smoking pack years, smoking status, age and gender. In some embodiments, the lung cancer risk-score is determined according to the follow equation:

$$\text{Score} = \frac{ex^{Score1\ or\ Score\ 2}}{(1+ex^{Score\ 1\ or\ Score\ 2})}$$

wherein:

$$X^{\text{score 1}} = W_0 + W_1 \text{ x } GG + W_2 \text{ x } G\text{S} + W_3 \text{ x } GPY + W_4 \text{ x } GA + W_5 \text{ x } C1A + W_6 \text{ x } C1B + W_7 \text{ x } C2 + W_8 \text{ x } C3 + W_9 \text{ x } C4A + W_{10} \text{ x } C4B$$

and

$$x^{score\ 2} = W_0 + W_1 \text{ x } GG + W_2 \text{ x } GS + W_3 \text{ x } GPY + W_4 \text{ x } Reported\ Age + W_5 \text{ x } C1A + W_6 \text{ x } C1B + W_7 \text{ x } C2 + W_8 \text{ x } C3 + W_9 \text{ x } C4A + W_{10} \text{ x } C4B,$$

and in which *GG, GS, GPY, GA, C1A, C1B, C2, C3, C4A* and *C4B* are determined according to the equations disclosed herein.

In some embodiments, informative-genes are selected from Table 1 or 11. In some embodiments, groups of related genes that vary collinearly (e.g., are correlated with one another) within a population of subjects may be combined or collapsed into a single value (e.g., the mean value of a group of related genes). In some embodiments, groups of related genes are correlated because they are associated with the same cellular and/or molecular pathways. In some embodiments, at least 2, at least 3, at least 4, at least 5 or more related genes (e.g., correlated genes, genes within a common cluster) are combined together in a single value. In some embodiments, groups of related genes are identified by performing a cluster analysis of expression levels obtained from multiple subjects (e.g., 2 to 100, 2 to 500, 2 to 1000 or more subjects). Any appropriate cluster analysis may be used to identify such related genes including, for example, centroid based clustering (e.g., k-means clustering), connectivity based clustering (e.g., hierarchical clustering) and other suitable approaches. Non-limiting examples of such clusters are identified in Table 11 with the values in column 2 specifying the cluster within which each gene resides such that related genes (e.g., correlated genes) are within the same cluster. In some embodiments, a value reflecting the expression status of a set of related genes is the mean expression level of the set of related genes. For example, one or more of the following values may be used: CIA, C1B, C2, C3, C4A, and C4B in a model for predicting the likelihood that a subject has cancer, in which

CIA = mean of (BST1, CD177.1, CD177.2),
C1B = mean of (ATP12A, TSPAN2),
C2 = mean of (GABBRI, MCAM, NOVA1, SDC2),
C3 = mean of (CDR1, CGREF1, CLND22, NKX3-1),
C4A = mean of (EPHX3, LYPD2), and
C4B = mean of (MIA, RNF150).

In some embodiments genes within a cluster can be substituted for each other. Thus, in some embodiments, all genes within a cluster need to be evaluated or used in a prediction model. In some embodiments, only 1, 2, 3, 4, 5, 6, 7, 8, 9, or 10 genes within a cluster are independently selected for analysis as described herein. In some embodiments, at least 1, 2, 3, 4, 5, 6, 7, 8, 9, or 10 genes within a cluster of table 11 are identified.

In some embodiments, one or more informative-genes are selected from the set of genes identified as cluster 1 in Table 11. In some embodiments, one or more informative-genes are selected from the set of genes identified as cluster 2 in Table 11. In some embodiments, one or more informative-genes are selected from the set of genes identified as cluster 3 in Table 11. In some embodiments, one or more informative-genes are selected from the set of genes identified as cluster 4 in Table 11. In some embodiments, one or more informative-genes are selected from the set of genes identified as cluster 5 in Table 11. In some embodiments, one or more informative-genes are selected from the set of genes identified as cluster 6 in Table 11. In some embodiments, one or more informative-genes are selected from the set of genes identified as cluster 7 in Table 11. In some embodiments, one or more informative-genes are the set of genes identified as cluster 8 in Table 11. In some embodiments, one or more informative-genes are selected from the set of genes identified as cluster 9 in Table 11. In some embodiments, one or more informative-genes are selected from the set of genes identified as cluster 10 in Table 11. In some embodiments, one or more informative-genes are selected from the set of genes identified as cluster 11 in Table 11. In some embodiments, the informative-genes comrise MYOT. In some embodiments, genes selected from a cluster are reduced to a single value, such as, for example, the mean, median, mode or other summary statistic of the expression levels of the selected genes.

In some embodiments, provided herein are methods for establishing appropriate diagnostic intervention plans and/or treatment plans for subjects and for aiding healthcare providers in establishing appropriate diagnostic intervention plans and/or treatment plans. In some embodiments, methods are provided that involve making a risk assessment based on expression levels of informative-genes in a biological sample obtained from a subject during a routine cell or tissue sampling procedure. In some embodiments, methods are provided that involve establishing lung cancer risk scores based on expression levels of informative genes. In some embodiments, appropriate diagnostic intervention plans are established based at least in part on the lung cancer risk scores. In some embodiments, methods provided herein assist health care providers with making early and accurate diagnoses. In some embodiments, methods provided herein assist health care providers with establishing appropriate therapeutic interventions early on in patients' clinical evaluations. In some embodiments, methods provided herein involve evaluating biological samples obtained during bronchoscopies procedure. In some embodiments, the methods are beneficial because they enable health care providers to make informative decisions regarding patient diagnosis and/or treatment from otherwise uninformative bronchoscopies. In some embodiments, the risk assessment leads to appropriate surveillance for monitoring low risk lesions. In some embodiments, the risk assessment leads to faster diagnosis, and thus, faster therapy for certain cancers.

Provided herein are methods for determining the likelihood that a subject has lung cancer, such as adenocarcinoma, squamous cell carcinoma, small cell cancer or non-small cell cancer. The methods alone or in combination with other methods provide useful information for health care providers to assist them in making diagnostic and therapeutic decisions for a patient. The methods disclosed herein are often employed in instances where other methods have failed to provide useful information regarding the lung cancer status of a patient. For example, approximately 50% of bronchoscopy procedures result in indeterminate or non-diagnostic information. There are multiple sources of indeterminate results, and may depend on the training and procedures available at different medical centers. However, in certain embodiments, molecular methods in combination with bronchoscopy are expected to improve cancer detection accuracy.

In some embodiments, provided herein are methods of determining the likelihood that a subject has lung cancer. In some embodiments, methods are provided that involve subjecting a biological sample obtained from a subject to a gene expression analysis, wherein the gene expression analysis comprises measuring cDNA levels of one or more informative-genes that relate to lung cancer status, and measuring cDNA levels of ore or more genomic correlate genes that relate to one or more self-reportable characteristics of the subject; and determining a lung cancer risk-score based on the cDNA levels determined in (a) and (b), that is indicative of the likelihood that the subject has lung cancer; wherein the cDNA is prepared from mRNA from the biological sample.

In some embodiments, the methods of the present disclosure include the conversion of mRNA into cDNA. In further embodiments, cDNA is amplified.

These and other aspects are described in more detail herein and are illustrated by the non-limiting figures and examples.

## BRIEF DESCRIPTION OF THE DRAWINGS

**FIG. 1** is a non-limiting example of a plot of correlation coefficients from pairwise correlation of all gene expression data of all qualified AEGIS I samples; samples with a correlation coefficient <0.955 were identified as outliers and excluded from further analysis; a total of 597 samples were retained;

**FIG. 2** is a non-limiting example of ROC curves for prediction models based on a set of training samples; and

**FIG. 3** is a non-limiting example of ROC curves for prediction models based on a set of bronchoscopy negative training samples.

**FIG. 4** depicts the following color-coding: patients that met inclusion criteria of the study (blue); patients who were excluded (yellow); patients who were included in the final analysis (green).

**FIG. 5** depicts ROC curves for total patients (light gray) and the subset of patients with a non-diagnostic bronchoscopy (black) in the AEGIS 1 (left) and AEGIS 2 (right) cohorts is shown. In AEGIS 1 the AUC=0.78 (95%CI, 0.73 to 0.83) and AUC=0.76 (95%CI, 0.68 to 0.83) for the two groups, respectively (p=0.31). In AEGIS 2 the AUC=0.74 (95%CI, 0.68 to 0.80) and AUC=0.75 (95%CI, 0.68 to 0.82) in the two groups (p=0.85). The AUC was also not significantly different for patients with a non-diagnostic bronchoscopy comparing AEGIS 1 and AEGIS 2 (p=0.61).

**FIG. 6A** and **FIG. 6B. FIG. 6A** depicts a post-test POM related to pre-test POM based on a negative classifier call (solid line; adjusted using the negative likelihood ratio) and a positive classifier call (dotted line; adjusted using the positive likelihood ratio) calculated for the classifier in combination with bronchoscopy. The negative classifier call curve shows that for patients with a pre-test POM of <66%, the post-test POM is <10% when bronchoscopy is negative and the classifier is negative. For patients with a negative bronchoscopy and a positive classifier score, the post-test likelihood of cancer is > 10% when the pre-test likelihood is greater than 5%. **FIG. 6B** depicts post test probability of cancer based on the pretest probability and the negative likelihood ratio of the classifier and bronchoscopy. The posttest probability of lung cancer is shown in relation to the pretest probability based on a nondiagnostic bronchoscopic examination and a negative classifier score (adjusted with the use of the negative likelihood ratio). The curve shows that for patients with a pretest probability of cancer of less than 66% (short vertical line), the posttest probability is less than 10% (broken line) when bronchoscopic findings are negative and the classifier score is negative.

**FIG. 7** depicts a pairwise correlation of genes with cancer-associated gene expression. The correlation between all possible pairs of genes with cancer-associated gene expression (n=232) were assessed to identify groups of genes that share a similar pattern of gene expression. Unsupervised hierarchical clustering was used to group correlated genes into 11 clusters, with the dendrogram threshold level to establish clusters indicated on the y-axis (green line). Genes were selected from the clusters in a parsimonious manner to predict lung cancer status using linear regression. The classifier genes came from specific clusters (outlined in blue), using 2-4 genes from each cluster. Clusters 4 and 7 contain genes which were up-regulated in lung cancer, and clusters 1, 2, 9, and 10 were down-regulated in lung cancer.

**FIG. 8** depicts an ROC curve of patients with a non-diagnostic bronchoscopy in the test set. The AUC=0.81 for the 123 patients whose bronchoscopy did not result in a diagnosis of lung cancer (in which the prevalence of lung cancer = 31%).

**FIG. 9** depicts gene expression data corresponding to all patients in the training set (black line), and the subset of patients with a non-diagnostic bronchoscopy (grey line) were analyzed using the locked classifier. The AUC was calculated as 0.78 (95% CI, 0.73-0.82) and 0.78 (95% CI, 0.71-0.85), for the two groups respectively.

**FIG. 10A** and **FIG. 10B** depicts nucleic acid probes used in hybridizing to nucleic acid sequences represented by gene classifier CD177. **Fig. 10A** discloses the 19 nucleic acid probes in CD177.1 (SEQ ID NOs:24-42 in order from top to bottom) and **Fig. 10B** discloses the 4 nucleic acid probes in CD177.2 (SEQ ID NOs:43-46 from top to bottom).

## DETAILED DESCRIPTION OF THE INVENTION

Methods disclosed herein provide alternative or complementary approaches for evaluating cell or tissue samples obtained by bronchoscopy procedures (or other procedures for evaluating respiratory tissue), and increase the likelihood that the procedures will result in useful information for managing the patient's care. The methods disclosed herein are highly sensitive, and produce information regarding the likelihood that a subject has lung cancer from cell or tissue samples (e.g., bronchial brushings of airway epithelial cells), which are often obtained from regions in the airway that are remote from malignant lung tissue. In general, the methods disclosed herein involve subjecting a biological sample obtained from a subject to a gene expression analysis to evaluate gene expression levels. However, in some embodiments, the likelihood that the subject has lung cancer is determined in further part based on the results of a histological examination of the biological sample or by considering other diagnostic indicia such as protein levels, mRNA levels, imaging results, chest X-ray exam results etc.

The term "subject," as used herein, generally refers to a mammal. Typically the subject is a human. However, the term embraces other species, e.g., pigs, mice, rats, dogs, cats, or other primates. In certain embodiments, the subject is an experimental subject such as a mouse or rat.

The subject may be a male or female. The subject may be an infant, a toddler, a child, a young adult, an adult or a geriatric. The subject may be a smoker, a former smoker or a non-smoker. The subject may have a personal or family history of cancer. The subject may have a cancer-free personal or family history. The subject may exhibit one or more symptoms of lung cancer or other lung disorder (e.g., emphysema, COPD). For example, the subject may have a new or persistent cough, worsening of an existing chronic cough, blood in the sputum, persistent bronchitis or repeated respiratory infections, chest pain, unexplained weight loss and/or fatigue, or breathing difficulties such as shortness of breath or wheezing. The subject may have a lesion , which may be observable by computer-aided tomography or chest X-ray. The subject may be an individual who has undergone a bronchoscopy or who has been identified as a candidate for bronchoscopy (e.g., because of the presence of a detectable lesion or suspicious imaging result). In some embodiments, a subject has or has been diagnosed with chronic obstructive pulmonary disease (COPD). In some embodiments, a subject does not have or has not been diagnosed with COPD. A subject under the care of a physician or other health care provider may be referred to as a "patient."

The term "about", as used herein, refers to plus or minus ten percent of the object that "about" modifies. Thus, the phrase "about 10, 20, or 30" encompasses 8-11, 18-22, 27-33, respectively.

### Informative-genes

The expression levels of genes of the present disclosure have been identified as providing useful information regarding the lung cancer status of a subject. These genes are referred to herein as "informative-genes." Informative-genes include protein coding genes and non-protein coding genes. It will be appreciated by the skilled artisan that the expression levels of informative-genes may be determined by evaluating the levels of appropriate gene products (e.g., mRNAs, miRNAs, proteins etc.) Accordingly, the expression levels of certain mRNAs have been identified as providing useful information regarding the lung cancer status of a subject. These mRNAs are referred to herein as "informative-mRNAs."

Table 11 provides a listing of informative-genes that are differentially expressed in cancer. In some embodiments, informative-genes that are differentially expressed in lung cancer are selected from: BST1, CD177.1, CD177.2, ATP12A, TSPAN2, GABBR1, MCAM, NOVA1, SDC2, CDR1, CGREF1, CLND22, NKX3-1, EPHX3, LYPD2, MIA, RNF150. In some embodiments, informative-genes that are differentially expressed in lung cancer are selected from: TMEM51, CR1L, PDZKIIP1, MICAL2, VWA5A, ACAD8, SAA4, GLYATL2, ETV6, CD177, CEACAM7, QPCT, CASP10, PI3, BST1, MTNR1A, STARD4, CFB, SLC26A8, VNN2, HDAC9, SLC26A4, and LCN2. In some embodiments, informative-genes that are differentially expressed in lung cancer are selected from: CCDC18, FAM72D, NUF2, FBXO28, GPR137B, STIL, DEPDC1, TSPAN2, ASPM, KIF14, KIF20B, RAD51AP1, GAS2L3, SPIC, SMAGP, ATP12A, BRCA2, BORA, SKA3, DLGAP5, CASC5, LRRC28, PYCARD, TXNL4B, EFCAB5, SPAG5, ABCAI2, AURKA, SGOL1, BANK1,

CENPE, CASP6, MAD2L1, CCNA2, CCNB1, KIF20A, CENPK, ERAP1, FAM54A, PHTF2, CLDN12, BPGM, PCMTD1, MELK, and MST4. In some embodiments, informative-genes that are differentially expressed in lung cancer are selected from: CR1, GOS2, CSF3R, S100Al2, SELL, NCF2, LIPN, ZNF438, NAMPT, CBL, CASP5, CARD16, CARD17, CLEC4A, LRRK2, HMGN2P46, AQP9, BCL2A1, ITGAX, GPR97, CCL4, PSTPIP2, IFI30, FFAR2, EMR3, FPR1, LILRA5, PLEK, MXD1, TNFAIP6, CXCR2, IL1B, CXCR1, SIRPB1, NCF4, IRAK2, PROK2, TLR2, TREM1, SOD2, CREB5, TNFRSF10C, CSGALNACT1, and ASAP 1. In some embodiments, informative-genes that are differentially expressed in lung cancer are selected from: PLA2G2A, NFYC, RASSF10, GLB1L3, TRIM3, MCAM, MSRB3, SLITRK5, GAS6, NOVA1, GABRG3, ABCA3, LPO, FSCN2, RASD1, HILS1, SDK2, NTN5, KCNA7, ATOH8, KCNIP3, INHBB, VSTM2L, ZNRF3, PLEKHG4B, GNMT, GABBR1, ARHGEF10, SDC2, CRB2, GAS1, PNPLA7, and RAI2.

Certain methods disclosed herein involve determining expression levels in the biological sample of at least one informative-gene. However, in some embodiments, the expression analysis involves determining the expression levels in the biological sample of at least 2, at least 3, at least 4, at least 5, at least 6, at least 7, at least 8, at least 9, at least 10, at least 20, at least 30, at least 40, at least 50, at least 60, at least 70, or least 80 informative-genes. In some embodiments, the expression analysis involves determining expression levels in the biological sample of 1 to 5, 1 to 10, 5 to 10, 5 to 15, 10 to 15, 10 to 20, 15 to 20, 15 to 25, 20 to 30, 25 to 50, 25 to 75, 50 to 100, 50 to 200 or more informative-genes, such as those in table 11. In some embodiments, the expression analysis involves determining expression levels in the biological sample to at least 1 to 5, 1 to 10, 2 to 10, 5 to 10, 5 to 15, 10 to 15, 10 to 20, 15 to 20, 15 to 25, 20 to 30, 25 to 50, 25 to 75, 50 to 100, 50 to 200 or more informative-genes, such as those in table 11.

In some embodiments, the number of informative-genes for an expression analysis are sufficient to provide a level of confidence in a prediction outcome that is clinically useful. This level of confidence (e.g., strength of a prediction model) may be assessed by a variety of performance parameters including, but not limited to, the accuracy, sensitivity specificity, and area under the curve (AUC) of the receiver operator characteristic (ROC) curve. These parameters may be assessed with varying numbers of features (e.g., number of genes, mRNAs) to determine an optimum number and set of informative-genes. An accuracy, sensitivity or specificity of at least 60%, 70%, 80%, 90%, may be useful when used alone or in combination with other information.

Any appropriate system or method may be used for determining expression levels of informative-genes. Gene expression levels may be determined through the use of a hybridization-based assay. As used herein, the term, "hybridization-based assay" refers to any assay that involves nucleic acid hybridization. A hybridization-based assay may or may not involve amplification of nucleic acids. Hybridization-based assays are well known in•the art and include, but are not limited to, array-based assays (e.g., oligonucleotide arrays, microarrays), oligonucleotide conjugated bead assays (e.g., Multiplex Bead-based Luminex® Assays), molecular inversion probe assays, and quantitative RT-PCR assays. Multiplex systems, such as oligonucleotide arrays or bead-based nucleic acid assay systems are particularly useful for evaluating levels of a plurality of genes simultaneously. Other appropriate methods for determining levels of nucleic acids will be apparent to the skilled artisan.

As used herein, a "level" refers to a value indicative of the amount or occurrence of a substance, e.g., an mRNA. A level may be an absolute value, e.g., a quantity of mRNA in a sample, or a relative value, e.g., a quantity of mRNA in a sample relative to the quantity of the mRNA in a reference sample (control sample). The level may also be a binary value indicating the presence or absence of a substance. For example, a substance may be identified as being present in a sample when a measurement of the quantity of the substance in the sample, e.g., a fluorescence measurement from a PCR reaction or microarray, exceeds a background value. Similarly, a substance may be identified as being absent from a sample (or undetectable in the sample) when a measurement of the quantity of the molecule in the sample is at or below background value. It should be appreciated that the level of a substance may be determined directly or indirectly.

Further non-limiting examples of informative mRNAs are disclosed in, for example, the following patent applications, the contents of which are incorporated herein by reference in their entirety for all purposes: U.S. Patent Publication No. US2007/148650, filed on May 12, 2006, entitled ISOLATION OF NUCLEIC ACID FROM MOUTH EPITHELIAL CELLS; U.S. Patent Publication No. US2009/311692, filed January 9, 2009, entitled ISOLATION OF NUCLEIC ACID FROM MOUTH EPITHELIAL CELLS; U.S. Application No. 12/884,714, filed September 17, 2010, entitled ISOLATION OF NUCLEIC ACID FROM MOUTH EPITHELIAL CELLS; U.S. Patent Publication No. US2006/154278, filed December 6, 2005, entitled DETECTION METHODS FOR DISORDER OF THE LUNG; U.S. Patent Publication No. US2010/035244, filed February 8, 2008, entitled, DIAGNOSTIC FOR LUNG DISORDERS USING CLASS PREDICTION; U.S. Application No. 12/869,525, filed August 26, 2010, entitled, DIAGNOSTIC FOR LUNG DISORDERS USING CLASS PREDICTION; U.S. Application No. 12/234,368, filed September 19, 2008, entitled, BIOMARKERS FOR SMOKE EXPOSURE; U.S. Application No. 12/905,897, filed October 154, 2010, entitled BIOMARKERS FOR SMOKE EXPOSURE; U.S. Patent Application No. US2009/186951, filed September 19, 2008, entitled IDENTIFICATION OF NOVEL PATHWAYS FOR DRUG DEVELOPMENT FOR LUNG DISEASE; U.S. Publication No. US2009/061454, filed September 9, 2008, entitled, DIAGNOSTIC AND PROGNOSTIC METHODS FOR LUNG DISORDERS USING GENE EXPRESSION PROFILES; U.S. Application No. 12/940,840, filed November 5, 2010, entitled, DIAGNOSTIC AND PROGNOSTIC METHODS FOR LUNG DISORDERS USING GENE EXPRESSION PROFILES; U.S. Publication No. US2010/055689, filed March 30, 2009, entitled, MULTIFACTORIAL METHODS FOR DETECTING LUNG DISORDERS; and International Patent Application No. PCT/US13/38449, filed April 26, 2013, entitled METHODS FOR EVALUATING LUNG CANCER STATUS.

**cDNA**

cDNA molecules are non-naturally occurring polynucleotide sequences that are synthesized from mRNA molecules by one possessing ordinary skill in the art. In some embodiments, cDNA molecules of the present invention are obtained or acquired. The conversion of RNA to cDNA utilizing a reverse transcriptase enzyme creates cDNA, a non-naturally occurring molecule that lacks introns. Methods that rely on cDNA are necessarily relying on an artificial molecule that does not naturally occur in nature, e.g. protein expression of cDNA molecules or hybridization of cDNA molecules.

In certain aspects, mRNA in a biological sample is used to produce cDNA from a sample by reverse transcription of mRNA using at least one primer; amplifying the cDNA using polynucleotides as sense and antisense primers to amplify cDNAs therein; and detecting the presence of the amplified cDNA. In further aspects, the sequence of the amplified cDNA can be determined by any suitable method.

In one embodiment, once the mRNA is obtained from a sample, it is converted to complementary DNA (cDNA). cDNA does not exist *in vivo* and therefore is a non-natural molecule. In a further embodiment, the cDNA is then amplified, for example, by the polymerase chain reaction (PCR) or other amplification method known to those of ordinary skill in the art. The product of this amplification reaction, *i.e.*, amplified cDNA is necessarily a non-natural product. As mentioned above, cDNA is a non-natural molecule. Second, in the case of PCR, the amplification process serves to create hundreds of millions of cDNA copies for every individual cDNA molecule of starting material. The number of copies generated are far removed from the number of copies of mRNA that are present *in vivo.*

In one embodiment, cDNA is amplified with primers that introduce an additional DNA sequence (adapter sequence) onto the fragments (with the use of adapter-specific primers). Amplification therefore serves to create non-natural double stranded molecules from the non-natural single stranded cDNA, by introducing barcode, adapter and/or reporter sequences onto the already non-natural cDNA. In one embodiment, during amplification with the adapter-specific primers, a detectable label, *e.g.*, a fluorophore, is added to single strand cDNA molecules. Amplification therefore also serves to create DNA complexes that do not occur in nature, at least because (i) cDNA does not exist *in vivo,* (i) adapter sequences are added to the ends of cDNA molecules to make DNA sequences that do not exist *in vivo,* (ii) the error rate associated with amplification further creates DNA sequences that do not exist *in vivo,* (iii) the disparate structure of the cDNA molecules as compared to what exists in nature and (iv) the chemical addition of a detectable label to the cDNA molecules.

In one embodiment, the synthesized cDNA (for example, amplified cDNA) is immobilized on a solid surface via hybridization with a probe, e.g., via a microarray. In another embodiment, cDNA products are detected via real-time polymerase chain reaction (PCR) via the introduction of fluorescent probes that hybridize with the cDNA products. For example, in one embodiment, biomarker detection is assessed by quantitative fluorogenic RT-PCR (e.g., with TaqMan® probes). For PCR analysis, well known methods are available in the art for the determination of primer sequences for use in the analysis.

In one embodiment, to synthesize and amplify cDNAs, the 5'Ampli FINDER RACE kit (Manufactured by Clontech) and the 5'-RACE method using PCR (Frohman, M.A. et al., Proc. Natl. Acad. Sci. USA (1988) 85:8998-9002; Belyaysky, A. et al., Nucleic Acids Research. (1989) 17:2919-2932) can be used. In the process of such cDNA synthesis, restriction enzyme sites can be introduced into both ends of the cDNA.

**Genomic correlates**

As disclosed herein, the expression levels of certain genes have been identified as being related to (correlated with) certain self-reportable characteristics of a subject. Such genes are referred to herein as "genomic correlate genes" or "genomic correlates" and are useful because they provide a surrogate marker for characteristics of a subject that could otherwise be incorrectly and/or inaccurately reported. For example, in some embodiments, a subject may incorrectly estimate information such as pack years, smoking status or age (e.g., by providing an underestimate of such information). In such embodiments, the use of a prediction model based on genomic correlate genes can reduce or eliminate variability associated with incorrect reporting because it is based on the expression of the genomic correlate genes rather than a subject's decision making about what information to report and/or a subject's recollection of circumstances. It will be appreciated by the skilled artisan that the expression levels of such genomic correlate genes may be determined by evaluating the levels of appropriate gene products (e.g., mRNAs, miRNAs, proteins etc.) Expression levels of genomic correlate genes may be determined in parallel with informative-genes of lung cancer status (e.g., an informative gene selected from Table 11) or independently of such genes.

In some embodiments, genomic correlates reflect a response of an individual to an environmental hazard (e.g., cigarette smoke). In some embodiments, genomic correlates reflect exposure to a hazard.

In some embodiment gender of a subject is determined based on one or more genomic correlate genes. In some embodiments, a genomic correlate gene related to gender is RPS4Y1. In some embodiments, if the expression of RPS4Y1 is below a threshold then the subject is identified as being a male and if the expression of RPS4Y1 is above the threshold the subject is identified as being a female. In some embodiments, a threshold is a relative expression level that accurately differentiates males and females for the gene(s) of interest.

In some embodiment smoking status (e.g., current or former) of a subject is determined based on one or more genomic correlate genes. In some embodiments, a genomic correlate gene related to smoking status is SLC7A11, CLND10 or TKT. In some embodiments, the smoking status of a subject is determined according to the following model: smoking status (also, referred to as Genomic Smoking (GS)) = exp(x)/(1+exp(x)), in which $x = \beta\frac{GS}{0} + \beta\frac{GS}{1} * SLC7A11 + \beta\frac{GS}{2} * CLND10 + \beta\frac{GS}{3} * TKT$, in which $\beta\frac{GS}{n}$ are regression weights for the regression model and gene symbols represent the relative expression intensity of each respective gene. In some embodiments, a smoker is a subject who has smoked at least 100 cigarettes in a lifetime. In some embodiments, a former smoker is a subject who quit or who has not smoked a cigarette within 1 month prior to bronchoscopy.

In some embodiment, smoking history of a subject is determined based on one or more genomic correlate genes. In some embodiments, a genomic correlate gene related to smoking history is AKR1C2 or RUNX1T1. In some embodiments, the smoking history of a subject is determined according to the following model: smoking history (also, referred to as Genomic Pack Years (GPY)) = exp(x)/(1+exp(x)), in which $x = \beta\frac{GPY}{0} + \beta\frac{GPY}{1} * RUNX1T1 + \beta\frac{GPY}{2} * AKR1C2$, in which $\beta\frac{GPY}{n}$, are regression weights for the model and gene symbols represent the relative expression intensity of each respective gene.

In some embodiment, age of a subject is determined based on one or more genomic correlate genes. In some embodiments, a genomic correlate gene related to age is CD52, SYT8, TNNT3, ALX1, KLRK1, RASA3, CERS3, ASPA, GRP, APOC1, EPHX3, REEP1, FAM198B, PCDHB4, PCDHB16, FOXD1, SPARC, NKAPL, or GPR110. In some embodiments, the age of a subject is determined according to the following model: age (also referred to as genomic age (GA)) = $\beta\frac{GA}{0} + \beta\frac{GA}{1} * CD52 + \beta\frac{GA}{2} * SYT8 + \beta\frac{GA}{3} * TNNT3 + \beta\frac{GA}{4} * ALX1 + \beta\frac{GA}{5} * KLRK1 + \beta\frac{GA}{6} * RASA3 + \beta\frac{GA}{7} * CERS3 + \beta\frac{GA}{8} * ASPA + \beta\frac{GA}{9} * GRP + \beta\frac{GA}{10} * APOC1 + \beta\frac{GA}{11} * EPHX3 + \beta\frac{GA}{12} * REEP1 + \beta\frac{GA}{13} * FAM198B + \beta\frac{GA}{14} * PCDHB4 + \beta\frac{GA}{15} * PCDHB16 + \beta\frac{GA}{16} * FOXD1 + \beta\frac{GA}{17} * SPARC\ \beta\frac{GA}{18} * NKAPL + \beta\frac{GA}{19} * GPR110$, in which $\beta\frac{GA}{n}$ are regression weights for the model and gene symbols represent the relative expression intensity of each respective gene.

### Biological Samples

The methods generally involve obtaining a biological sample from a subject. As used herein, the phrase "obtaining a biological sample" refers to any process for directly or indirectly acquiring a biological sample from a subject. For example, a biological sample may be obtained (e.g., at a point-of-care facility, a physician's office, a hospital) by procuring a tissue or fluid sample from a subject. Alternatively, a biological sample may be obtained by receiving the sample (e.g., at a laboratory facility) from one or more persons who procured the sample directly from the subject.

The term "biological sample" refers to a sample derived from a subject, e.g., a patient. A biological sample typically comprises a tissue, cells and/or biomolecules. In some embodiments, a biological sample is obtained on the basis that it is histologically normal, e.g., as determined by endoscopy, e.g., bronchoscopy. In some embodiments, biological samples are obtained from a region, e.g., the bronchus or other area or region, that is not suspected of containing cancerous cells. In some embodiments, a histological or cytological examination is performed. However, it should be appreciated that a histological or cytological examination may be optional. In some embodiments, the biological sample is a sample of respiratory epithelium. The respiratory epithelium may be of the mouth, nose, pharynx, trachea, bronchi, bronchioles, or alveoli of the subject. The biological sample may comprise epithelium of the bronchi. In some embodiments, the biological sample is free of detectable cancer cells, e.g., as determined by standard histological or cytological methods. In some embodiments, histologically normal samples are obtained for evaluation. Often biological samples are obtained by scrapings or brushings, e.g., bronchial brushings. However, it should be appreciated that other procedures may be used, including, for example, brushings, scrapings, broncho-alveolar lavage, a bronchial biopsy or a transbronchial needle aspiration.

It is to be understood that a biological sample may be processed in any appropriate manner to facilitate determining expression levels. For example, biochemical, mechanical and/or thermal processing methods may be appropriately used to isolate a biomolecule of interest, e.g., RNA, from a biological sample. Accordingly, a RNA or other molecules may be isolated from a biological sample by processing the sample using methods well known in the art.

## Lung Cancer Assessment

Methods disclosed herein may involve comparing expression levels of informative-genes with one or more appropriate references. An "appropriate reference" is an expression level (or range of expression levels) of a particular informative-gene that is indicative of a known lung cancer status. An appropriate reference can be determined experimentally by a practitioner of the methods or can be a pre-existing value or range of values. An appropriate reference represents an expression level (or range of expression levels) indicative of lung cancer. For example, an appropriate reference may be representative of the expression level of an informative-gene in a reference (control) biological sample obtained from a subject who is known to have lung cancer. When an appropriate reference is indicative of lung cancer, a lack of a detectable difference (e.g., lack of a statistically significant difference) between an expression level determined from a subject in need of characterization or diagnosis of lung cancer and the appropriate reference may be indicative of lung cancer in the subject. When an appropriate reference is indicative of lung cancer, a difference between an expression level determined from a subject in need of characterization or diagnosis of lung cancer and the appropriate reference may be indicative of the subject being free of lung cancer.

Alternatively, an appropriate reference may be an expression level (or range of expression levels) of a gene that is indicative of a subject being free of lung cancer. For example, an appropriate reference may be representative of the expression level of a particular informative-gene in a reference (control) biological sample obtained from a subject who is known to be free of lung cancer. When an appropriate reference is indicative of a subject being free of lung cancer, a difference between an expression level determined from a subject in need of diagnosis of lung cancer and the appropriate reference may be indicative of lung cancer in the subject. Alternatively, when an appropriate reference is indicative of the subject being free of lung cancer, a lack of a detectable difference (e.g., lack of a statistically significant difference) between an expression level determined from a subject in need of diagnosis of lung cancer and the appropriate reference level may be indicative of the subject being free of lung cancer.

In some embodiments, the reference standard provides a threshold level of change, such that if the expression level of a gene in a sample is within a threshold level of change (increase or decrease depending on the particular marker) then the subject is identified as free of lung cancer, but if the levels are above the threshold then the subject is identified as being at risk of having lung cancer.

In some embodiments, the methods involve comparing the expression level of an informative-gene to a reference standard that represents the expression level of the informative-gene in a control subject who is identified as not having lung cancer. This reference standard may be, for example, the average expression level of the informative-gene in a population of control subjects who are identified as not having lung cancer.

The magnitude of difference between a expression level and an appropriate reference that is statistically significant may vary. For example, a significant difference that indicates lung cancer may be detected when the expression level of an informative-gene in a biological sample is at least 1%, at least 5%, at least 10%, at least 25%, at least 50%, at least 100%, at least 250%, at least 500%, or at least 1000% higher, or lower, than an appropriate reference of that gene. Similarly, a significant difference may be detected when the expression level of informative-gene in a biological sample is at least 1.1-fold, 1.2-fold,1.5-fold, 2-fold, at least 3-fold, at least 4-fold, at least 5-fold, at least 6-fold, at least 7-fold, at least 8-fold, at least 9-fold, at least 10-fold, at least 20-fold, at least 30-fold, at least 40-fold, at least 50-fold, at least 100-fold, or more higher, or lower, than the appropriate reference of that gene. In some embodiments, at least a 20% to 50% difference in expression between an informative-gene and appropriate reference is significant. Significant differences may be identified by using an appropriate statistical test. Tests for statistical significance are well known in the art and are exemplified in Applied Statistics for Engineers and Scientists by Petruccelli, Chen and Nandram 1999 Reprint Ed.

It is to be understood that a plurality of expression levels may be compared with plurality of appropriate reference levels, e.g., on a gene-by-gene basis, in order to assess the lung cancer status of the subject. The comparison may be made as a vector difference. In such cases, Multivariate Tests, e.g., Hotelling's T2 test, may be used to evaluate the significance of observed differences. Such multivariate tests are well known in the art and are exemplified in Applied Multivariate Statistical Analysis by Richard Arnold Johnson and Dean W. Wichern Prentice Hall; 6th edition (April 2, 2007).

## Classification Methods

The methods may also involve comparing a set of expression levels (referred to as an expression pattern or profile) of informative-genes in a biological sample obtained from a subject with a plurality of sets of reference levels (referred to as reference patterns), each reference pattern being associated with a known lung cancer status, identifying the reference pattern that most closely resembles the expression pattern, and associating the known lung cancer status of the reference pattern with the expression pattern, thereby classifying (characterizing) the lung cancer status of the subject.

The methods may also involve building or constructing a prediction model, which may also be referred to as a classifier or predictor, that can be used to classify the disease status of a subject. As used herein, a "lung cancer-classifier" is a prediction model that characterizes the lung cancer status of a subject based on expression levels determined in a biological sample obtained from the subject. Typically the model is built using samples for which the classification (lung cancer status) has already been ascertained. Once the model (classifier) is built, it may then be applied to expression levels obtained from a biological sample of a subject whose lung cancer status is unknown in order to predict the lung cancer status of the subject. Thus, the methods may involve applying a lung cancer-classifier to the expression levels, such that the lung cancer-classifier characterizes the lung cancer status of a subject based on the expression levels. The subject may be further treated or evaluated, e.g., by a health care provider, based on the predicted lung cancer status.

The classification methods may involve transforming the expression levels into a lung cancer risk-score that is indicative of the likelihood that the subject has lung cancer. In some embodiments, such as, for example, when a linear discriminant classifier is used, the lung cancer risk-score may be obtained as the combination (e.g., sum, product, or other combination) of weighted expression levels, in which the expression levels are weighted by their relative contribution to predicting increased likelihood of having lung cancer.

It should be appreciated that a variety of prediction models known in the art may be used as a lung cancer-classifier. For example, a lung cancer-classifier may comprises an algorithm selected from logistic regression, partial least squares, linear discriminant analysis, quadratic discriminant analysis, neural network, naive Bayes, C4.5 decision tree, k-nearest neighbor, random forest, support vector machine, or other appropriate method.

The lung cancer-classifier may be trained on a data set comprising expression levels of the plurality of informative-genes in biological samples obtained from a plurality of subjects identified as having lung cancer. For example, the lung cancer-classifier may be trained on a data set comprising expression levels of a plurality of informative-genes in biological samples obtained from a plurality of subjects identified as having lung cancer based histological findings. The training set will typically also comprise control subjects identified as not having lung cancer. As will be appreciated by the skilled artisan, the population of subjects of the training data set may have a variety of characteristics by design, e.g., the characteristics of the population may depend on the characteristics of the subjects for whom diagnostic methods that use the classifier may be useful. For example, the population may consist of all males, all females or may consist of both males and females. The population may consist of subjects with history of cancer, subjects without a history of cancer, or subjects from both categories. The population may include subjects who are smokers, former smokers, and/or non-smokers.

A class prediction strength can also be measured to determine the degree of confidence with which the model classifies a biological sample. This degree of confidence may serve as an estimate of the likelihood that the subject is of a particular class predicted by the model.

Accordingly, the prediction strength conveys the degree of confidence of the classification of the sample and evaluates when a sample cannot be classified. There may be instances in which a sample is tested, but does not belong, or cannot be reliably assigned to, a particular class. This may be accomplished, for example, by utilizing a threshold, or range, wherein a sample which scores above or below the determined threshold, or within the particular range, is not a sample that can be classified (e.g., a "no call").

Once a model is built, the validity of the model can be tested using methods known in the art. One way to test the validity of the model is by cross-validation of the dataset. To perform cross-validation, one, or a subset, of the samples is eliminated and the model is built, as described above, without the eliminated sample, forming a "cross-validation model." The eliminated sample is then classified according to the model, as described herein. This process is done with all the samples, or subsets, of the initial dataset and an error rate is determined. The accuracy the model is then assessed. This model classifies samples to be tested with high accuracy for classes that are known, or classes have been previously ascertained. Another way to validate the model is to apply the model to an independent data set, such as a new biological sample having an unknown lung cancer status.

As will be appreciated by the skilled artisan, the strength of the model may be assessed by a variety of parameters including, but not limited to, the accuracy, sensitivity and specificity. Methods for computing accuracy, sensitivity and specificity are known in the art and described herein (See, e.g., the Examples). The lung cancer-classifier may have an accuracy of at least 60%, at least 65%, at least 70%, at least 75%, at least 80%, at least 85%, at least 90%, at least 95%, at least 99%, or more. The lung cancer-classifier may have an accuracy in a range of about 60% to 70%, 70% to 80%, 80% to 90%, or 90% to 100%. The lung cancer-classifier may have a sensitivity of at least 60%, at least 65%, at least 70%, at least 75%, at least 80%, at least 85%, at least 90%, at least 95%, at least 99%, or more. The lung cancer-classifier may have a sensitivity in a range of about 60% to 70%, 70% to 80%, 80% to 90%, or 90% to 100%. The lung cancer-classifier may have a specificity of at least 60%, at least 65%, at least 70%, at least 75%, at least 80%, at least 85%, at least 90%, at least 95%, at least 99%, or more. The lung cancer-classifier may have a specificity in a range of about 60% to 70%, 70% to 80%, 80% to 90%, or 90% to 100%.

The Negative Predictive Value (NPV) may be greater than 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% for ruling out lung cancer in an intended use population.

The intended use population may have a prevalence of cancer at or about 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%.

## Clinical Treatment/Management

In certain aspects, methods are provided for determining a treatment course for a subject. The methods typically involve determining the expression levels in a biological sample obtained from the subject of one or more informative-genes, and determining a treatment course for the subject based on the expression levels. Often the treatment course is determined based on a lung cancer risk-score derived from the expression levels. The subject may be identified as a candidate for a lung cancer therapy based on a lung cancer risk-score that indicates the subject has a relatively high likelihood of having lung cancer. The subject may be identified as a candidate for an invasive lung procedure (e.g., transthoracic needle aspiration, mediastinoscopy, or thoracotomy) based on a lung cancer risk-score that indicates the subject has a relatively high likelihood of having lung cancer (e.g., greater than 60%, greater than 70%, greater than 80%, greater than 90%). The subject may be identified as not being a candidate for a lung cancer therapy or an invasive lung procedure based on a lung cancer risk-score that indicates the subject has a relatively low likelihood (e.g., less than 50%, less than 40%, less than 30%, less than 20%) of having lung cancer. In some cases, an intermediate risk-score is obtained and the subject is not indicated as being in the high risk or the low risk categories. In some embodiments, a health care provider may engage in "watchful waiting" and repeat the analysis on biological samples taken at one or more later points in time, or undertake further diagnostics procedures to rule out lung cancer, or make a determination that cancer is present, soon after the risk determination was made. In a particular example, a subject is identified as intermediate risk due to a non-diagnostic bronchoscopy and is reassigned to non-invasive monitoring (such as CT surveillance) following a determination, using the methods herein, that the patient is at low-risk of cancer. In another particular example, the samples assayed as described herein may be used The methods may also involve creating a report that summarizes the results of the gene expression analysis. Typically the report would also include an indication of the lung cancer risk-score.

## Computer Implemented Methods

Methods disclosed herein may be implemented in any of numerous ways. For example, certain embodiments may be implemented using hardware, software or a combination thereof. When implemented in software, the software code can be executed on any suitable processor or collection of processors, whether provided in a single computer or distributed among multiple computers. Such processors may be implemented as integrated circuits, with one or more processors in an integrated circuit component. Though, a processor may be implemented using circuitry in any suitable format.

Further, it should be appreciated that a computer may be embodied in any of a number of forms, such as a rack-mounted computer, a desktop computer, a laptop computer, or a tablet computer. Additionally, a computer may be embedded in a device not generally regarded as a computer but with suitable processing capabilities, including a Personal Digital Assistant (PDA), a smart phone or any other suitable portable or fixed electronic device.

Also, a computer may have one or more input and output devices. These devices can be used, among other things, to present a user interface. Examples of output devices that can be used to provide a user interface include printers or display screens for visual presentation of output and speakers or other sound generating devices for audible presentation of output. Examples of input devices that can be used for a user interface include keyboards, and pointing devices, such as mice, touch pads, and digitizing tablets. As another example, a computer may receive input information through speech recognition or in other audible format.

Such computers may be interconnected by one or more networks in any suitable form, including as a local area network or a wide area network, such as an enterprise network or the Internet. Such networks may be based on any suitable technology and may operate according to any suitable protocol and may include wireless networks, wired networks or fiber optic networks.

Also, the various methods or processes outlined herein may be coded as software that is executable on one or more processors that employ any one of a variety of operating systems or platforms. Additionally, such software may be written using any of a number of suitable programming languages and/or programming or scripting tools, and also may be compiled as executable machine language code or intermediate code that is executed on a framework or virtual machine.

In this respect, aspects of the disclosure may be embodied as a computer readable medium (or multiple computer readable media) (e.g., a computer memory, one or more floppy discs, compact discs (CD), optical discs, digital video disks (DVD), magnetic tapes, flash memories, circuit configurations in Field Programmable Gate Arrays or other semiconductor devices, or other non-transitory, tangible computer storage medium) encoded with one or more programs that, when executed on one or more computers or other processors, perform methods that implement the various embodiments of the disclosure discussed above. The computer readable medium or media can be transportable, such that the program or programs stored thereon can be loaded onto one or more different computers or other processors to implement various aspects of the present disclosure as discussed above. As used herein, the term "non-transitory computer-readable storage medium" encompasses only a computer-readable medium that can be considered to be a manufacture (i.e., article of manufacture) or a machine.

The terms "program" or "software" are used herein in a generic sense to refer to any type of computer code or set of computer-executable instructions that can be employed to program a computer or other processor to implement various aspects of the present disclosure as discussed above. Additionally, it should be appreciated that according to one aspect of this embodiment, one or more computer programs that when executed perform methods of the present disclosure need not reside on a single computer or processor, but may be distributed in a modular fashion amongst a number of different computers or processors to implement various aspects of the present disclosure.

As used herein, the term "database" generally refers to a collection of data arranged for ease and speed of search and retrieval. Further, a database typically comprises logical and physical data structures. Those skilled in the art will recognize the methods described herein may be used with any type of database including a relational database, an object-relational database and an XML-based database, where XML stands for "eXtensible-Markup¬Language". For example, the gene expression information may be stored in and retrieved from a database. The gene expression information may be stored in or indexed in a manner that relates the gene expression information with a variety of other relevant information (e.g., information relevant for creating a report or document that aids a physician in establishing treatment protocols and/or making diagnostic determinations, or information that aids in tracking patient samples). Such relevant information may include, for example, patient identification information, ordering physician identification information, information regarding an ordering physician's office (e.g., address, telephone number), information regarding the origin of a biological sample (e.g., tissue type, date of sampling), biological sample processing information, sample quality control information, biological sample storage information, gene annotation information, lung-cancer risk classifier information, lung cancer risk factor information, payment information, order date information, etc.

Computer-executable instructions may be in many forms, such as program modules, executed by one or more computers or other devices. Generally, program modules include routines, programs, objects, components, data structures, etc. that perform particular tasks or implement particular abstract data types. Typically the functionality of the program modules may be combined or distributed as desired in various embodiments.

In some aspects of the disclosure, computer implemented methods for processing genomic information are provided. The methods generally involve obtaining data representing expression levels in a biological sample of one or more informative-genes and determining the likelihood that the subject has lung cancer based at least in part on the expression levels. Any of the statistical or classification methods disclosed herein may be incorporated into the computer implemented methods. In some embodiments, the methods involve calculating a risk-score indicative of the likelihood that the subject has lung cancer. Computing the risk-score may involve a determination of the combination (e.g., sum, product or other combination) of weighted expression levels, in which the expression levels are weighted by their relative contribution to predicting increased likelihood of having lung cancer. The computer implemented methods may also involve generating a report that summarizes the results of the gene expression analysis, such as by specifying the risk-score. Such methods may also involve transmitting the report to a health care provider of the subject.

**Affymetrix Array**

In some aspects, the Affymetrix Human Gene 1.0 ST array (Affymetrix Cat. # 901087) is used to identify the mRNA or cDNA in a biological sample. The Affymetrix Human Gene 1.0 ST array utilizes numerous probes that are disclosed at www.affymetrix.com/site/include/byproduct.affx?product=hugene-1_0-st-v1. Multiple probes are present that correspond to segments of specific genes, which is to say that that is not a 1:1 ratio of one probe per gene, rather multiple probes are present that correspond to multiple segments of a single gene. In one example, the LYPD2 gene is represented by three probe sets in the Human Gene 1.0 ST array (Release 32), probeset IDs 8153343, 8153344, and 8153345 as disclosed in the Affymetrix Human Gene 1.0 ST array (HuGene-1_0-st-v1 Probeset Annotations. Exemplary suitable builds of the array include release 32 (09/30/2011), release 33 (03/27/2013), release 34 04/07/2014), release 35 on (04/15/2015), and release 36. Additional releases, including future releases may also be used. Information correlating probe-sets and nucleic acid sequences can be found at Affymetrix.com, including at www.affymetrix.com/site/include/byproduct.affx?product=hugene-1_0-st-v1. Furthermore, data sets are available on the NCBI Gene Expression Omnibus website under Platform GPL6244 detailing the probes and genes that may be utilized in practicing the methods of the present disclosure at www.ncbi.nlm.nih.gov/geo/query/acc.cgi?acc=GPL6244. These documents, including those correlating the probesets and gene symbols are incorporated herein by reference.

**Compositions and Kits**

In some aspects, compositions and related methods are provided that are useful for determining expression levels of informative-genes. For example, compositions are provided that consist essentially of nucleic acid probes that specifically hybridize with informative-genes or with nucleic acids having sequences complementary to informative-genes. These compositions may also include probes that specifically hybridize with control genes or nucleic acids complementary thereto. These compositions may also include appropriate buffers, salts or detection reagents. The nucleic acid probes may be fixed directly or indirectly to a solid support (e.g., a glass, plastic or silicon chip) or a bead (e.g., a magnetic bead). The nucleic acid probes may be customized for used in a bead-based nucleic acid detection assay.

In some embodiments, compositions are provided that comprise up to 5, up to 10, up to 25, up to 50, up to 100, or up to 200 nucleic acid probes. In some cases, each of the nucleic acid probes specifically hybridizes with an mRNA selected from Table 11 or with a nucleic acid having a sequence complementary to the mRNA. In some embodiments, probes that detect informative-mRNAs are also included. In some cases, each of at least 2, at least 3, at least 4, at least 5, at least 6, at least 7, at least 8, at least 9, at least 10, or at least 20 of the nucleic acid probes specifically hybridizes with an mRNA selected from Table llor with a nucleic acid having a sequence complementary to the mRNA. In some embodiments, the compositions are prepared for detecting different genes in biochemically separate reactions, or for detecting multiple genes in the same biochemical reactions. In some embodiments, the compositions are prepared for performing a multiplex reaction.

Also provided herein are oligonucleotide (nucleic acid) arrays that are useful in the methods for determining levels of multiple informative-genes simultaneously. Such arrays may be obtained or produced from commercial sources. Methods for producing nucleic acid arrays are also well known in the art. For example, nucleic acid arrays may be constructed by immobilizing to a solid support large numbers of oligonucleotides, polynucleotides, or cDNAs capable of hybridizing to nucleic acids corresponding to genes, or portions thereof. The skilled artisan is referred to Chapter 22 "Nucleic Acid Arrays" of Current Protocols In Molecular Biology (Eds. Ausubel et al. John Wiley and #38; Sons NY, 2000) or Liu CG, et al., An oligonucleotide microchip for genome-wide microRNA profiling in human and mouse tissues. Proc Nall Acad Sci USA. 2004 Jun 29;101(26):9740-4, which provide non-limiting examples of methods relating to nucleic acid array construction and use in detection of nucleic acids of interest. In some embodiments, the arrays comprise, or consist essentially of, binding probes for at least 2, at least 5, at least 10, at least 20, at least 50, at least 60, at least 70 or more informative-genes. In some embodiments, the arrays comprise, or consist essentially of, binding probes for up to 2, up to 5, up to 10, up to 20, up to 50, up to 60, up to 70 or more informative-genes. In some embodiments, an array comprises or consists of 1, 2, 3, 4, 5, 6, 7, 8, 9, or 10 of the mRNAs selected from Table 11. In some embodiments, an array comprises or consists of 4, 5, or 6 of the mRNAs selected from Table 11. Kits comprising the oligonucleotide arrays are also provided. Kits may include nucleic acid labeling reagents and instructions for determining expression levels using the arrays.

The compositions described herein can be provided as a kit for determining and evaluating expression levels of informative-genes. The compositions may be assembled into diagnostic or research kits to facilitate their use in diagnostic or research applications. A kit may include one or more containers housing the components of the disclosure and instructions for use. Specifically, such kits may include one or more compositions described herein, along with instructions describing the intended application and the proper use of these compositions. Kits may contain the components in appropriate concentrations or quantities for running various experiments.

The kit may be designed to facilitate use of the methods described herein by researchers, health care providers, diagnostic laboratories, or other entities and can take many forms. Each of the compositions of the kit, where applicable, may be provided in liquid form (e.g., in solution), or in solid form, (e.g., a dry powder). In certain cases, some of the compositions may be constitutable or otherwise processable, for example, by the addition of a suitable solvent or other substance, which may or may not be provided with the kit. As used herein, "instructions" can define a component of instruction and/or promotion, and typically involve written instructions on or associated with packaging of the disclosure. Instructions also can include any oral or electronic instructions provided in any manner such that a user will clearly recognize that the instructions are to be associated with the kit, for example, audiovisual (e.g., videotape, DVD, etc.), Internet, and/or web-based communications, etc. The written instructions may be in a form prescribed by a governmental agency regulating the manufacture, use or sale of diagnostic or biological products, which instructions can also reflect approval by the agency.

A kit may contain any one or more of the components described herein in one or more containers. As an example, in one embodiment, the kit may include instructions for mixing one or more components of the kit and/or isolating and mixing a sample and applying to a subject. The kit may include a container housing agents described herein. The components may be in the form of a liquid, gel or solid (e.g., powder). The components may be prepared sterilely and shipped refrigerated. Alternatively they may be housed in a vial or other container for storage. A second container may have other components prepared sterilely.

As used herein, the terms "approximately" or "about" in reference to a number are generally taken to include numbers that fall within a range of 1%, 5%, 10%, 15%, or 20% in either direction (greater than or less than) of the number unless otherwise stated or otherwise evident from the context (except where such number would be less than 0% or exceed 100% of a possible value).

All references described herein are incorporated by reference for the purposes described herein.

Exemplary embodiments of the disclosure will be described in more detail by the following examples. These embodiments are exemplary of the disclosure, which one skilled in the art will recognize is not limited to the exemplary embodiments.

## EXAMPLES

### Example 1: Development of a Microarray Based Prediction Model

#### Introduction

This example describes a method for developing a prediction algorithm. A final optimized model is described, including the combination of genes used in the model. The method uses Clinical Factor Genomic Correlates (CFGC) to aid in the selection of a cancer-specific signature.

The objectives were to develop and characterize a new cancer prediction model that effectively predicts lung cancer status after accounting gene expression signal attributed more specifically to clinical factors by deriving genomic correlates. Genomic correlates are defined herein as gene expression algorithms to predict the specific clinical characteristics, such as subject gender, smoking status, and smoking history.

An objective was to develop a prediction algorithm that meets the following specific performance criteria:

Negative Predictive Value (NPV) of greater than 90% for ruling out lung cancer in an intended use population, and NPV of greater than 85% for subjects diagnosed with COPD.

#### Materials and Methods

#### Sample Processing and Analysis

Clinical specimens were collected from patients scheduled to undergo bronchoscopy for the suspicion of lung cancer. Bronchial epithelial cells (BECs) were collected from the mainstem bronchus of subjects using standard bronchial brushes during a scheduled bronchoscopy procedure.

Samples were analyzed on gene-expression microarrays using Gene 1.0 ST microarrays (Affymetrix). A pairwise correlation analysis of the array data was conducted to identify outliers (described herein). A total of 597 samples were retained as the final data set. The data set were then split into equivalently sized Training and Validation sets in a randomized manner.

Microarray CEL files were used for the development of a prediction algorithm. Subjects were first designated to independent Training and Validation sets, and gene selection and optimization of the prediction model was conducted within the Training set. The model was optimized and locked prior to predicting the cancer status of validation set samples.

#### Normalization /Batch Adjustment

RMA was used to compute gene expression values from Gene 1.0 ST (Affymetrix) CEL files. ComBat batch adjustment was used to correct for batch effects. All samples were analyzed within 5 separate microarray experiments (i.e., batches). Training and test samples were combined in RMA and ComBat pre-processing. Subsequent development was restricted to the Training set as previously described. CEL files corresponding to samples with a RIN score less than 4 were excluded, as were samples with average pairwise correlation less than .955 (see FIG. 1).

## Genomic Correlates

Genomic correlates were established as a gene expression signature that accurately predict the corresponding clinical characteristic. The genomic correlates, in combination with separate genes to predict cancer status, are combined in a prediction algorithm. Correlates for the following clinical characteristics were developed and evaluated:

Gender
Smoking status (current versus former)
Smoking history (pack-year; PY)
Age

The genomic correlates were developed by selecting top-ranked genes differentiating the clinical characteristic of interest and fitting those genes to a model using logistic regression. Scoring of clinical characteristics was based on the gene expression of those selected genes.
Two models were developed in parallel to be tested simultaneously in a Validation set. A first model (Score 1) was based on the methods described herein and factoring the reported Age into the prediction algorithm as well as the genomic signal. A second model (Score 2) was developed using a genomic correlate for age, which was then incorporated into the prediction algorithm.

## Initial Gene Selection

A clinical factor model was developed, using logistic regression of cancer status (0/1) on age, gender, smoking status, and pack years. The residuals from the clinical factor model were used to select genes using an empirical Bayes linear model to test association of each gene with the residuals. The top 232 genes were selected based on the p-value and fold change from this model. The top 232 genes are listed in Table 11.

## *Clustering/Final Gene Selection*

Gene selection and model fitting were conducted in an automated cross-validation approach in order to minimize bias during the selection process and to provide a robust final selection. The gene selection consisted of the following steps. Hierarchical clustering was used to divide the genes into 11 clusters. The cluster membership of each gene is identified in column 2 of the Table 11. For each of the clusters, cluster means were computed using all of the genes within each cluster. A combination of LASSO and backwards selection were used in repeated random subsets of the data to identify six clusters that were consistently selected to have independent predictive association with cancer status. Cross validation was then used to determine the approximate number of genes within each cluster that would retain the predictive strength of the cluster means.
A gene titration analysis was done to determine the sensitivity of the models to increased numbers of genes from the selected clusters within the final model. This was included as part of the optimization to determine if complementary genes could add additional clinical sensitivity to the model.

## Software

R (Version 3.01) was used for the analysis, including the packages rms, limma, verification, and sva.

## Results

## Derivation of Genomic Correlates

Genes were selected to represent clinical characteristics using the whole Training set. These "genomic correlate genes" were based on a minimal set of genes to represent the clinical factor as accurately as possible. Genomic Gender (GG) was defined with 100% accuracy using a single gene. The values are set forth as follows: GG = 1 (male) if RPS4Y1 < threshold, and GG = 0 (female) otherwise.
For genomic smoking status, genes were screened based on an empirical Bayes t-test. The top genes by p-value were included in a logistic regression model where smoking status was the dependent variable. The resulting predicted genomic smoking (GS) value was derived from this model (using gene symbols to represent the relative expression intensity), where, $x= \beta_{0}^{GS} + \beta_{1}^{GS}{*}SLC7A11 + \beta_{2}^{GS}{*}CLND10 + \beta_{3}^{GS}{*}TKT,$ in which $\beta_{n}^{GS}$ are regression weights for the genomic regression model, and GS = exp(x)/(1+exp(x)).

For genomic pack years, genes were screened based on an empirical Bayes t-test. The top genes by p-value were included in a logistic regression model where pack years < 10 was the dependent variable. The resulting predicted genomic pack years (GPY) value was derived from this model, where $x=\beta_{0}^{GPY} + \beta_{1}^{GPY}{*}RUNX1T1 + \beta_{2}^{GPY}{*}AKR1C2,$ in which $\beta_{n}^{GPY}$ are regression weights for the genomic pack years regression model, and GPY = exp(x)/(1+exp(x)).

For genomic age, genes were screened based on an empirical Bayes linear model. The top genes by p-value were included in a penalized linear regression model (LASSO) where age (in years) was the dependent variable. The resulting predicted genomic age (GA) value was derived from this model, where,

$$GA = \beta_{0}^{GA} + \beta_{1}^{GA}{*}CD52 + \beta_{2}^{GA}{*}SYT8 + \beta_{3}^{GA}{*}TNNT3 + \beta_{4}^{GA}{*}ALX1 + \beta_{5}^{GA}{*}KLRK1 + \beta_{6}^{GA}{*}RASA3 + \beta_{7}^{GA}{*}CERS3 + \beta_{8}^{GA}{*}ASPA + \beta_{9}^{GA}{*}GRP + \beta_{10}^{GA}{*}APOC1 + \beta_{11}^{GA}{*}EPHX3 + \beta_{12}^{GA}{*}REEP1 + \beta_{13}^{GA}{*}FAM198B + \beta_{14}^{GA}{*}PCDHB4 + \beta_{15}^{GA}{*}PCDHB16 + \beta_{16}^{GA}{*}FOXD1 + \beta_{17}^{GA}{*}SPARC\ \beta_{18}^{GA}{*}NKAPL + \beta_{19}^{GA}{*}GPR110,$$

in which $\beta_{n}^{GA}$ are regression weights for the genomic age regression model.

### Derivation of the Cancer Genes

The top 232 gene were selected initially, followed by a down-selection of genes using the clustering analysis described in the methods section.

Cross validation was then used to determine the approximate number of genes within each cluster that would retain the predictive strength of the cluster means. This number was found to be between 2 and 4 genes per cluster. Final gene selection within each cluster was based on p-value, fold change, and strength of evidence for cancer association from the literature. Cluster means were recomputed using the reduced gene sets within each cluster, as follows:

CIA= mean of (BST1, CD177.1, CD177.2)
C1B= mean of (ATP 12A, TSPAN2)
C2 = mean of (GABBRI, MCAM, NOVA1, SDC2)
C3 = mean of (CDR1, CGREF1, CLND22, NKX3-1) C4A = mean of (EPHX3, LYPD2)
C4B = mean of (MIA, RNF150).

### Description of the finalized model

To estimate the final model coefficients, a penalized logistic regression model was used, with Age (in years), genomic gender (GG), genomic smoking status (GS), genomic pack years (GPY), and the six reduced gene cluster means (labeled CIA, C1B, C2, C3, C4A, C4B) as the independent predictors and cancer status (0/1) as the dependent variable. The penalization factor (lambda) was 0 for the clinical/genomic correlates and 10 for each of the gene expression clusters. The second model was built using the same approach, but replacing Age with genomic age (GA) as defined above. The model coefficients were then re-estimated.

The final classification algorithm was of the form, $x^{score1} = W_0 + W_1 \times GG + W_2 \times GS + W_3 \times GPY + W_4 \times Reported\ Age + W_5 \times C1A + W_6 \times C1B + W_7 \times C2 + W_8 \times C3 + W_9 \times C4A + W_{10} \times C4B$ $X^{score2} = W_0 + W_1 \times GG + W_2 \times GS + W_3 \times GPY + W_4 \times GA + W_5 \times C1A + W_6 \times C1B + W_7 \times C2 + W_8 \times C3 + W_9 \times C4A + W_{10} \times C4B.$

The logistic regression score is then converted to a prediction score, ranging from 0 to 1, using the equation

$$Prediction\ Score = \frac{ex^{Score1\ or\ Score\ 2}}{(1+ex^{Score\ 1\ or\ Score\ 2})}$$

**Evaluation of Prediction Models**

A cross-validation approach was used in which the Training set samples were randomly split (90:10) into training and testing groups, respectively. The clinical accuracy was recorded and the process was repeated 100-fold. Scores for the training set were reported as the average of the total iterations for each sample. Results were reported as ROC curves, AUC, and sensitivity and specificity after defining the score threshold to yield 50% specificity and to maximize the clinical sensitivity.

The scores for all training set samples were generated and compared to recorded clinical status. ROC curves for Score 1 and Score 2 are shown in for all samples in FIG. 2, and for bronchoscopy-negative samples only in FIG. 3. The AUC's were calculated as, Score 1 = 0.803, and Score 2 = 0.785, for all samples, and Score 1 = 0.808, and Score 2 = 0.778 for the bronch-negative set. Table 1 provides a list of informative genes used in the prediction score models. Table 1.1 provides a non-limiting list of probe sequences for detecting expression of such genes.

Table 1: Gene list and function

| Gene Symbol | Function | Gene Symbol ' | Function |
|---|---|---|---|
| RPS4Y1 | GG | TSPAN2 | CA |
| SLC7A11 | GS | MCAM | CA |
| CLDN10 | GS | ATP12A | CA |
| TKT | GS | NOVA1 | CA |
| RUNX1T1 | GPY | MIA | CA |
| AKR1C2 | GPY | CD177.1 | CA |
| CD52 | GA | EPHX3 | CA |
| SYT8 | GA | CD177.2 | CA |
| TNNT3 | GA | CGREF1 | CA |
| ALX1 | GA | BST1 | CA |
| KLRK1 | GA | RNF150 | CA |
| RASA3 | GA | CLND22 | CA |
| CERS3 | GA | GABBR1 | CA |
| ASPA | GA | SDC2 | CA |
| GRP | GA | NKX3-1 | CA |
| APOC1 | GA | LYPD2 | CA |
| EPHX3 | GA, CA | CDR1 | CA |
| REEP1 | GA | | |
| FAM198B | GA | | |
| PCDHB4 | GA | | |
| PCDHB16 | GA | | |
| FOXD1 | GA | | |
| SPARC | GA | | |
| NKAPL | GA | | |
| GPR110 | GA | | |

Table 1.1: Non-limiting examples of probe sequences for detecting informative-genes.

| Gene: Cancer | Probe Sequence | SEQ ID NO: |
|---|---|---|
| TSPAN2 | TCAACATTAAGAAGTCTTAATTCAG | 1 |
| MCAM | GCTTTAATCCCCATGAAGGACAGTG | 2 |
| ATP12A | GCGGTGGAGATACCGGCGGCGGCGC | 3 |
| NOVA1 | GACGAAATTCAGACATGGAGCATCA | 4 |
| MIA | ATGACACCAAGGCACACCAGGGACC | 5 |
| CD177.1 | GACCCGTCTGTGGCTGGTAATCTCT | 6 |
| EPHX3 | GCTCCACTGGAAGAGAGGTATACCC | 7 |

(continued)

| Gene: Cancer | Probe Sequence | SEQ ID NO: |
|---|---|---|
| CD177.2 | TTCCTGTGTCCCATTGAGCAGGTTG | 8 |
| CGREF1 | TAGGGTACAGCACTTAACGCAATCT | 9 |
| BST1 | TGTTTGCCGTTTCCCGTTCCAGACA | 10 |
| RNF150 | TGGTTAATCCAAGCCGCAGCCTGGT | 11 |
| CLDN22 | GGTGTTTCTCGTCTCCAGTTCTTGA | 12 |
| GABBR1 | TACGGAGCCATTACCTGGGCAGTGC | 13 |
| SDC2 | TGAGCCTGCTTCTCCGGGCTCCCCT | 14 |
| NKX3-1 | TTTGTGCTGGCTAGTACTCCGGTCG | 15 |
| LYPD2 | TTCTTCAAGGCATTCGGGGCTGGGC | 16 |
| CDR1 | AACAACTCCGGGTCTTCCAGCGACT | 17 |
| Gene: Gender | Probe sequence | SEQ ID NO: |
| RP S4Y1 | TAAACCGCAGGAAGTCAGATGAGTG | 18 |
| Gene: Smoking | Probe sequence | SEQ ID NO: |
| SLC7A11 | GGTTGAAGCAACTAGAAGCGTGACA | 19 |
| CLDN10 | GACAGCGTTTCATGCTCGGATGGCC | 20 |
| TKT | GGTTTATTCTCTCCAGACGGTCAGG | 21 |
| Gene: PY | Probe sequence | SEQ ID NO: |
| RUNX1T1 | TAACAGGGAGGAGGTCAAATCTATC | 22 |
| AKR1 C2 | TAGCTGTAGCTTACTGAAGTCGCCA | 23 |

Table 2 below provides a summary of performance characteristics for the two models for bronchoscopy-negative subjects in the training set. The number of bronch-negative subjects (N) corresponds to CA+ subjects for sensitivity and CA- subjects for specificity.

| Table 2 | N | Score 1 | Score 2 |
|---|---|---|---|
| Sensitivity | 68 | 91.2% | 82.4% |
| Specificity | 76 | 56.6% | 48.7% |
| AUC | | 80.8% | 77.8% |
| NPV* | | 95.5% | 90.0% |
| *NPV is calculated assuming a 50% prevalence of cancer combined with the observed sensitivity and specificity of bronchoscopy and prediction model. | | | |

**Model Performance**

Calculation of the sensitivity and specificity of the prediction models (based on scores 1 and 2) was done using a specific score threshold, to differentiate prediction of CA+ and CA-samples. The same threshold that was selected for both models in the Training Set (Model Score = 0.65) was used in the Validation Set. Prediction accuracy was first determined in the bronchoscopy-negative samples (the intended-use cases) for both models and is summarized in

The sensitivity of the prediction model was also calculated for several subgroup categories within the Validation Set. Results are shown in Tables 3-9 for both models. Sub¬categories contain different numbers of samples which affect confidence intervals.

Table 3 - Sensitivity of prediction model as a function of the mass size of the observed lesion- Score 1

| Mass Size | N | Sens Sens. | Sens, | PM+BR Sens. |
|---|---|---|---|---|
| <1 | 13 | 100.00% | 83.30% | 100.00% |
| 1 to 2 | 35 | 87.50% | 50.00% | 100.00% |
| 2 to 3 | 41 | 90.60% | 53,10% | 96.90% |
| >3 | 155 | 85.10% | 79.40% | 95.00% |
| Infiltrate | 32 | 100.00% | 100.00% | 100.00% |
| Unknown | 21 | 100.00% | 80.00% | 100.00% |
| PM = Prediction Model; BR = Bronchoscopy | | | | |

Table 4 - Sensitivity of prediction model as a function of the mass size of the observed lesion- Score 2

| Mass Size | N | PMs. | Senens. | |
|---|---|---|---|---|
| BR Sens. | PM+BR S | | | |
| <1 | 13 | 66.70% | 83.30% | 100.00% |
| 1 to 2 | 35 | 87.50% | 50.00% | 93.80% |
| 2 to 3 | 41 | 81.30% | 53.10% | 93.80% |
| >3 | 155 | 83.00% | 79.40% | 95.70% |
| Infiltrate | 32 | 90.00% | 100.00% | 100.00% |
| Unknown | 21 | 100.00% | 80.00% | 100.00% |

Table 5 - Sensitivity of prediction model as a function of the location of the observed lesion in the lung (Score 1)

| Mass Size | N | PM Sens | BR Sens | PM+BR Sens |
|---|---|---|---|---|
| Central | 97 | 84.40% | 79.20% | 93.50% |
| Peripheral | 86 | 90.70% | 61.10% | 96.30% |
| Both | 90 | 90.50% | 78.40% | 100.00% |
| Unknown | 25 | 86.70% | 80.00% | 93.30% |

Table 6 - Sensitivity of prediction model as a function of the location of the observed lesion in the lung (Score 2)

| Mass Size | N | PM Sens. | BR Sens. | PM+BR Sens. |
|---|---|---|---|---|
| Central | 97 | 79.20% | 79.20% | 93.50% |
| Peripheral | 86 | 81.50% | 61.10% | 96.30% |
| Both | 90 | 89.20% | 78,40% | 98.60% |
| Unknown | 25 | 93.30% | 80.00% | 93.30% |

Table 7 - Sensitivity of prediction model as a function of the cancer sub-type as determined in pathology (Score 1)

| Cancer Type | N | P M Sens. | BR Sens. | PM+BR Sens. |
|---|---|---|---|---|
| AC | 67 | 83.60% | 70.10% | 94.00% |
| SCC | 72 | 94.40% | 75.00% | 95.80% |

(continued)

| Cancer Type | N | P M Sens. | BR Sens. | PM+BR Sens. |
|---|---|---|---|---|
| LC | 7 | 71.40% | 85.70% | 100.00% |
| NSCLC | 26 | 92.30% | 73.10% | 76.90% |
| SCLC | 37 | 89.20% | 86.50% | 97.30% |
| NSCLC/SCLC | 2 | 50.00% | 100.00% | 100,00% |
| Unknown | 86 | 75.00% | 37.50% | 100.00% |

Table 8 - Sensitivity of prediction model as a function of the cancer sub-type as determined in pathology (Score 2)

| Cancer Type | N | PM Sens. | BR Sens. | P M+BR Sens. |
|---|---|---|---|---|
| AC | 67 | 80,60% | 70.10% | 94,00% |
| SCC | 72 | 87.50% | 75.00% | 97.20% |
| LC | 7 | 85.70% | 85.70% | 100.00% |
| NSCLC | 26 | 69.20% | 73.10% | 80.80% |
| SCLC | 37 | 89.20% | 86.50% | 97.30% |
| NSCLC/SCLC | 2 | 0.00% | 100.00% | 100.00% |
| Unknown | 86 | 62.50% | 37.50% | 87.50% |
| PM = Prediction Model; BR = Bronchoscopy | | | | |

Table 9 - Sensitivity of prediction model as a function of cancer stage

| Score 1 | | | | |
|---|---|---|---|---|
| Stage | N | PM Sens. | BR Sens. | PM+B R |
| I | 13 | 92.3% | 38.5% | 100.0% |
| IIa | 2 | 100.0% | 0.0% | 100.0% |
| IIb | 13 | 92.3% | 76.9% | 100.0% |
| *Early* | *28* | *92.9%* | *53.6%* | *100.0%* |
| Ina | 27 | 92.6% | 74.1% | 96.3% |
| IIIb | 19 | 89.5% | 89.5% | 100.0% |
| IV | 47 | 87.2% | 89.4% | 100.0% |
| Extensive | 15 | 73.3% | 73.3% | 93.3% |
| Score 2 | | | | |
| Stage | N | PM Sens, | BR Sens. | PM+BR Sens. |
| I | 13 | 84.6% | 38.5% | 92.3% |
| IIa | 2 | 100.0% | 0.0% | 100.0% |
| Jib | 13 | 92.3% | 76.9% | 100.0% |
| *Early* | *28* | *89.3%* | *53.6%* | *96.4%* |
| Ina | 27 | 92.6% | 74.1% | 100.0% |
| IIIb | 19 | 78.9% | 89.5% | 94.7% |
| IV | 47 | 80.9% | 89.4% | 100.0% |

(continued)

| Score 2 | | | | |
|---|---|---|---|---|
| Stage | N | PM Sens, | BR Sens. | PM+BR Sens. |
| Extensive | 15 | 80.0% | 73.3% | 93.3% |

**Comparison of Performance in Training and Validation Sets**

The overall prediction accuracy of the prediction models in the Training and Validation Sets was compared in order to evaluate the reproducibility of the models in independent cohorts. Results are provided in Table 10.

Table 10 - Comparison of sensitivity, specificity and AUC for two models in Training and Validation sets

| | Score 1 | | | | Score 2 | | | |
|---|---|---|---|---|---|---|---|---|
| | Training | | Validation | | Training | | Validation | |
| | All samples | BR-neg samples | All samples | BR-neg samples | All samples | BR-neg samples | All samples | BR-neg samples |
| Sensitivity | 90.6% | 91.2% | 88.2% | 85.7% | 85.7% | 82.4% | 84.1% | 83.9% |
| Specificity | 56.6% | 56.6% | 47.4% | 47.4% | 48.7% | 48.7% | 47.4% | 47.4% |
| AUC | 0.803 | 0.808 | 0.793 | 0.764 | 0.785 | 0.778 | 0.751 | 0.739 |

Significant difference ($p>0.05$) in sensitivity or specificity were not observed between the Training and Validation Sets for either model. Likewise the small differences in AUC for each model between the two cohorts was not statistically significant (based on $p>0.05$). The sensitivity and specificity is also similar for the bronch-negative samples compared to all samples (bronch-neg and bronch-pos combined). The was a relatively small drop in overall performance between Score 1 and Score 2, with the latter showing a 2-4% point drop in AUC compared to score 1.

Table 11: 232 differentially expressed genes and cluster membership

| **Symbol** | **Cluster** | **Tstat** | **p.value** |
|---|---|---|---|
| TMEM51 | 1 | -3.73334 | 0.000227 |
| CR1L | 1 | -2.84036 | 0.00482 |
| PDZK1IP1 | 1 | -3.65419 | 0.000305 |
| MICAL2 | 1 | -3.08175 | 0.002252 |
| VWA5A | 1 | -3.01006 | 0.002838 |
| ACAD8 | 1 | -3.25793 | 0.001253 |
| SAA4 | 1 | -2.84375 | 0.00477 |
| GLYATL2 | 1 | -3.0263 8 | 0.002693 |
| ETV6 | 1 | -4.46414 | 1.15E-05 |
| CD177 | 1 | -3.68455 | 0.000273 |
| CEACAM7 | 1 | -5.40343 | 1.35E-07 |
| CD177 | 1 | -3.85704 | 0.000141 |
| QPCT | 1 | -3.16572 | 0.001709 |
| CASP10 | 1 | -3.07674 | 0.002289 |
| PI3 | 1 | -5.27363 | 2.59E-07 |
| BST1 | 1 | -4.29031 | 2.42E-05 |
| MTNRIA | 1 | -4.47628 | 1.09E-05 |
| STARD4 | 1 | -2.94146 | 0.003525 |

(continued)

| Symbol | Cluster | Tstat | p.value |
|---|---|---|---|
| CFB | 1 | -2.87447 | 0.004341 |
| SLC26A8 | 1 | -3.09768 | 0.002138 |
| VNN2 | 1 | -2.88831 | 0.00416 |
| HDAC9 | 1 | -3.27926 | 0.001165 |
| SLC26A4 | 1 | -3.93362 | 0.000104 |
| LCN2 | 1 | -3.64257 | 0.000319 |
| CFB | 1 | -2.86774 | 0.004432 |
| CCDC18 | 2 | -2.89401 | 0.004087 |
| FAM72D | 2 | -3.41712 | 0.000722 |
| NUF2 | 2 | -3.35293 | 0.000904 |
| FAM72D | 2 | -3.65638 | 0.000303 |
| FBXO28 | 2 | -2.93189 | 0.003633 |
| GPR137B | 2 | -3.29637 | 0.001099 |
| STIL | 2 | -3.05607 | 0.002448 |
| DEPDCI | 2 | -3.10412 | 0.002094 |
| TSPAN2 | 2 | -3.92967 | 0.000106 |
| FAM72D | 2 | -3.18098 | 0.001624 |
| ASPM | 2 | -3.21677 | 0.001441 |
| KIF14 | 2 | -3.09106 | 0.002185 |
| KIF20B | 2 | -2.95904 | 0.003336 |
| RAD51AP1 | 2 | -3.38028 | 0.000822 |
| GAS2L3 | 2 | -3.22465 | 0.001403 |
| SPIC | 2 | -3.00214 | 0.00291 |
| SMAGP | 2 | -3.38429 | 0.00081 |
| ATP12A | 2 | -3.49107 | 0.000555 |
| BRCA2 | 2 | -2.9535 | 0.003395 |
| BORA | 2 | -2.81443 | 0.005215 |
| SKA3 | 2 | -2.94422 | 0.003495 |
| DLGAP5 | 2 | -2.8962 | 0.00406 |
| CASC5 | 2 | -2.98473 | 0.003076 |
| LRRC28 | 2 | -3.78219 | 0.000188 |
| PYCARD | 2 | -3.0296 | 0.002666 |
| TXNL4B | 2 | -3.75986 | 0.000205 |
| EFCAB5 | 2 | -4.15228 | 4.31E-05 |
| SPAG5 | 2 | -3.28148 | 0.001157 |
| FAM72D | 2 | -3.66614 | 0.000292 |
| ABCAI2 | 2 | -3.25495 | 0.001266 |
| AURKA | 2 | -3.06413 | 0.002385 |

(continued)

| Symbol | Cluster | Tstat | p.value |
|---|---|---|---|
| SGOL1 | 2 | -2.87447 | 0.004341 |
| BANK1 | 2 | -3.22839 | 0.001385 |
| CENPE | 2 | -2.90302 | 0.003975 |
| CASP6 | 2 | -2.96202 | 0.003305 |
| MAD2L1 | 2 | -3.2685 | 0.001209 |
| CCNA2 | 2 | -3.12554 | 0.001952 |
| CCNB1 | 2 | -3.43884 | 0.000668 |
| KIF20A | 2 | -3.16017 | 0.001741 |
| CENPK | 2 | -3.38809 | 0.000799 |
| ERAP1 | 2 | -3.0003 | 0.002927 |
| FAM54A | 2 | -3.5307 | 0.000481 |
| PHTF2 | 2 | -2.82226 | 0.005093 |
| CLDN12 | 2 | -3.10361 | 0.002097 |
| BPGM | 2 | -2.89166 | 0.004117 |
| PCMTD1 | 2 | -2.92723 | 0.003686 |
| MELK | 2 | -2.90007 | 0.004011 |
| MST4 | 2 | -3.46215 | 0.000615 |
| CR1 | 3 | -3.1375 | 0.001876 |
| GOS2 | 3 | -3.08943 | 0.002197 |
| CSF3R | 3 | -3.326 | 0.000992 |
| S100Al2 | 3 | -2.93619 | 0.003584 |
| SELL | 3 | -2.81834 | 0.005154 |
| NCF2 | 3 | -2.8535 | 0.00463 |
| LIPN | 3 | -3.16551 | 0.00171 |
| ZNF438 | 3 | -3.31249 | 0.00104 |
| NAMPT | 3 | -2.85014 | 0.004678 |
| CBL | 3 | -3.77272 | 0.000195 |
| CASP5 | 3 | -2.96813 | 0.003242 |
| CARD 16 | 3 | -3.32829 | 0.000985 |
| CARD 17 | 3 | -2.96301 | 0.003295 |
| CLEC4A | 3 | -2.9666 | 0.003257 |
| LRRK2 | 3 | -3.38419 | 0.00081 |
| HMGN2P46 | 3 | -3.56798 | 0.00042 |
| AQP9 | 3 | -3.18953 | 0.001578 |
| BCL2A1 | 3 | -2.88499 | 0.004203 |
| ITGAX | 3 | -2.92718 | 0.003687 |
| GPR97 | 3 | -3.39828 | 0.000771 |
| CCL4 | 3 | -2.86648 | 0.004449 |

(continued)

| Symbol | Cluster | Tstat | p.value |
|---|---|---|---|
| PSTPIP2 | 3 | -2.88175 | 0.004245 |
| IFI30 | 3 | -2.81741 | 0.005168 |
| FFAR2 | 3 | -3.20036 | 0.001522 |
| EMR3 | 3 | -3.09579 | 0.002151 |
| FPR1 | 3 | -3.03905 | 0.002586 |
| LILRA5 | 3 | -2.87193 | 0.004375 |
| PLEK | 3 | -3.08569 | 0.002223 |
| MXD1 | 3 | -2.9859 | 0.003064 |
| TNFAIP6 | 3 | -3.05049 | 0.002492 |
| CXCR2 | 3 | -3.51316 | 0.000512 |
| IL1B | 3 | -3.27702 | 0.001174 |
| CXCR1 | 3 | -3.38462 | 0.000809 |
| SIRPB1 | 3 | -3.65291 | 0.000307 |
| NCF4 | 3 | -3.14344 | 0.00184 |
| IRAK2 | 3 | -3.27512 | 0.001182 |
| PROK2 | 3 | -3.43542 | 0.000677 |
| TLR2 | 3 | -2.82581 | 0.005038 |
| TREM1 | 3 | -2.96731 | 0.00325 |
| SOD2 | 3 | -3.16918 | 0.001689 |
| CREB 5 | 3 | -3.32746 | 0.000987 |
| NAMPT | 3 | -2.80465 | 0.005372 |
| TNFRSF10C | 3 | -2.80794 | 0.005318 |
| CSGALNACT1 | 3 | -3.54557 | 0.000455 |
| ASAP1 | 3 | -2.80888 | 0.005303 |
| PLA2G2A | 4 | 3.087887 | 0.002208 |
| NFYC | 4 | 3.20083 | 0.00152 |
| RASSF10 | 4 | 3.105541 | 0.002084 |
| GLB1L3 | 4 | 2.800517 | 0.005439 |
| TRIM3 | 4 | 3.207674 | 0.001485 |
| MCAM | 4 | 2.70666 | 0.007192 |
| MSRB3 | 4 | 3.432075 | 0.000685 |
| SLITRK5 | 4 | 3.963535 | 9.27E-05 |
| GAS6 | 4 | 2.820157 | 0.005125 |
| NOVA1 | 4 | 2.729315 | 0.006728 |
| GABRG3 | 4 | 2.904108 | 0.003961 |
| ABCA3 | 4 | 3.321624 | 0.001008 |
| LPO | 4 | 3.513723 | 0.000511 |
| FSCN2 | 4 | 2.781525 | 0.005759 |

(continued)

| Symbol | Cluster | Tstat | p.value |
|---|---|---|---|
| RASD1 | 4 | 3.198556 | 0.001531 |
| HILS1 | 4 | 3.002738 | 0.002905 |
| SDK2 | 4 | 3.45176 | 0.000638 |
| NTN5 | 4 | 3.159291 | 0.001746 |
| KCNA7 | 4 | 3.631462 | 0.000332 |
| ATOH8 | 4 | 3.062376 | 0.002398 |
| KCNIP3 | 4 | 2.994468 | 0.002982 |
| INHBB | 4 | 3.056753 | 0.002442 |
| VSTM2L | 4 | 3.520433 | 0.000499 |
| ZNRF3 | 4 | 3.592073 | 0.000384 |
| PLEKHG4B | 4 | 2.830968 | 0.00496 |
| GNMT | 4 | 3.274623 | 0.001184 |
| GABBR1 | 4 | 2.881256 | 0.004252 |
| ARHGEF10 | 4 | 3.270419 | 0.001201 |
| SDC2 | 4 | 2.847433 | 0.004717 |
| CRB2 | 4 | 3.452184 | 0.000637 |
| GAS1 | 4 | 3.470173 | 0.000598 |
| PNPLA7 | 4 | 2.715581 | 0.007006 |
| RAI2 | 4 | 3.25911 | 0.001248 |
| PLA2G2A | 4 | 2.899771 | 0.004015 |
| ID3 | 5 | 3.213565 | 0.001456 |
| PGLYRP4 | 5 | -3.64634 | 0.000314 |
| SFTPA1 | 5 | 3.189676 | 0.001578 |
| SFTPA1 | 5 | 3.189676 | 0.001578 |
| LIPK | 5 | -2.99802 | 0.002949 |
| SFTPA2 | 5 | 3.858853 | 0.00014 |
| SFTPA2 | 5 | 3.858853 | 0.00014 |
| ASCL3 | 5 | 2.764568 | 0.006059 |
| RPPH1 | 5 | 2.832278 | 0.00494 |
| CD209 | 5 | 3.331793 | 0.000973 |
| GPR32 | 5 | -2.98245 | 0.003098 |
| UGT2A3 | 5 | -2.84993 | 0.004681 |
| CD58 | 6 | -3.35673 | 0.000892 |
| LBR | 6 | -3.62457 | 0.000341 |
| ARHGDIB | 6 | -3.22018 | 0.001424 |
| SLC12A6 | 6 | -4.12377 | 4.85E-05 |
| LPCAT2 | 6 | -3.5142 | 0.00051 |
| PLEKHB2 | 6 | -3.02046 | 0.002745 |

(continued)

| Symbol | Cluster | Tstat | p.value |
|---|---|---|---|
| KYNU | 6 | -4.13269 | 4.68E-05 |
| ANKRD36B | 6 | -3.84654 | 0.000147 |
| ANKRD36B | 6 | -3.49272 | 0.000551 |
| ANKRD36B | 6 | -3.55319 | 0.000443 |
| SRD5A3 | 6 | -4.25485 | 2.81E-05 |
| NEIL3 | 6 | -3.23716 | 0.001345 |
| TLR1 | 6 | -2.90118 | 0.003997 |
| BCAP29 | 6 | -3.44601 | 0.000652 |
| MGAM | 6 | -3.11565 | 0.002016 |
| TPK1 | 6 | -3.21156 | 0.001466 |
| ATP6V1B2 | 6 | -3.57198 | 0.000414 |
| LYN | 6 | -3.1938 | 0.001556 |
| SDCBP | 6 | -2.80868 | 0.005307 |
| GK | 6 | -2.91501 | 0.003829 |
| GLA | 6 | -3.30241 | 0.001077 |
| ADRA2A | 7 | 3.550905 | 0.000447 |
| PRKCDBP | 7 | 2.741146 | 0.006496 |
| PRR4 | 7 | 3.898998 | 0.00012 |
| PRB4 | 7 | 2.988547 | 0.003039 |
| PRB3 | 7 | 3.690625 | 0.000266 |
| PRB1 | 7 | 2.976402 | 0.003158 |
| PRB2 | 7 | 3.201779 | 0.001515 |
| BMP4 | 7 | 2.886357 | 0.004185 |
| PRKCA | 7 | 3.80558 | 0.000172 |
| CYP1B1-AS1 | 7 | 2.720927 | 0.006897 |
| CGREF1 | 7 | 3.275505 | 0.00118 |
| RPRM | 7 | 2.944897 | 0.003488 |
| SDPR | 7 | 2.84669 | 0.004728 |
| BPIFB2 | 7 | 3.949534 | 9.80E-05 |
| BPIFB 6 | 7 | 2.98498 | 0.003073 |
| SNCA | 7 | 2.777053 | 0.005837 |
| CLDN22 | 7 | 3.502336 | 0.000533 |
| COBL | 7 | 3.1512 | 0.001793 |
| NKX3-1 | 7 | 2.92659 | 0.003693 |
| CDR1 | 7 | 4.307308 | 2.25E-05 |
| CH25H | 8 | 3.168911 | 0.001691 |
| FXCI | 8 | 3.17821 | 0.001639 |
| DLG2 | 8 | 2.948964 | 0.003443 |

(continued)

| Symbol | Cluster | Tstat | p.value |
|---|---|---|---|
| NRXN3 | 8 | 2.863338 | 0.004493 |
| CES1P1 | 8 | 3.630652 | 0.000333 |
| CES1 | 8 | 3.122943 | 0.001968 |
| KCNJ16 | 8 | 3.53821 | 0.000468 |
| APCDD1 | 8 | 3.103106 | 0.002101 |
| TMEM178 | 8 | 2.7868 | 0.005668 |
| MYRIP | 8 | 2.958247 | 0.003344 |
| FLNB | 8 | 2.911823 | 0.003867 |
| ENPP5 | 8 | 2.788207 | 0.005644 |
| SEMA3E | 8 | 2.940987 | 0.003531 |
| SLC7A2 | 8 | 3.321666 | 0.001007 |
| ARHGAP6 | 8 | 3.220932 | 0.001421 |
| ANO3 | 9 | -2.98289 | 0.003094 |
| SLC22A10 | 9 | -3.04806 | 0.002512 |
| UFM1 | 9 | -3.15354 | 0.001779 |
| EPHX3 | 9 | -3.73504 | 0.000225 |
| KLF7 | 9 | -2.84977 | 0.004683 |
| LGSN | 9 | -3.5566 | 0.000438 |
| LYPD2 | 9 | -2.93177 | 0.003634 |
| CES3 | 10 | -3.3613 | 0.000878 |
| MIA | 10 | -3.23844 | 0.001339 |
| RNF150 | 10 | -4.32839 | 2.06E-05 |
| SLC9A3 | 10 | -2.88577 | 0.004193 |
| MYOT | 11 | -3.40228 | 0.000761 |

**Example 2: Validation of Bronchial Genomic Classifier for Lung Cancer Patients Undergoaing Diagnostic Chronchoscopy**

**Introduction**

Bronchoscopy is frequently non-diagnostic in patients with pulmonary lesions suspicious for lung cancer. This often results in additional invasive testing, although many lesions are benign. We sought to validate a bronchial gene expression classifier that could improve the diagnostic performance of bronchoscopy.

Lesions suspicious for lung cancer are frequently identified on chest imaging. The decision of whether to pursue surveillance imaging or an invasive evaluation requiring tissue sampling is complex and requires assessment of the likelihood of malignancy, ability to biopsy, surgical risk, and patient preferences [1]. When a biopsy is required, the approach can include bronchoscopy, transthoracic needle biopsy (TTNB), or surgical lung biopsy (SLB). The choice between these modalities is usually determined by considerations such as lesion size and location, presence of adenopathy, risk of procedure and local expertise. Bronchoscopy is a safe procedure, with less than 1% complicated by pneumothorax [2]. There are approximately 500,000 bronchoscopies performed per year in the U.S. [3], of which roughly half are for the diagnostic workup of lung cancer. However, bronchoscopy is limited by its sensitivity, ranging between 34-88% depending on the location and size of the lesion [4]. Even with newer bronchoscopic guidance techniques, the sensitivity is only ~70% for peripheral lesions [5].

Patients with a non-diagnostic bronchoscopy often undergo further invasive testing to establish a definitive diagnosis. SLB is not the initial preferred approach given the inherent risks, with a complication rate of approximately of 5% and 30-day mortality of ~1% [6]. Importantly, 20-25% of SLBs are performed in patients ultimately diagnosed with benign lesions [7,8].Furthermore, TTNB is associated with significant morbidity including a 15% pneumothorax rate [9], of which 6% require chest tube drainage [10,11]. Given the pitfalls of invasive procedures, alternative approaches are needed to identify patients with lower likelihood of malignancy who are appropriate for imaging surveillance.

Classification of biological disease states, including cancer, using gene expression measurements of clinical specimens is well established [12]. In the setting of lung cancer, there are distinct cancer-associated gene expression patterns in cytologically-normal epithelium collected from the proximal airways of smokers [13]. Recently, we developed a gene expression classifier in bronchial epithelial cells (BECs) collected from the mainstem bronchus via bronchoscopy that distinguishes patients with and without lung cancer amongst current and former smokers (manuscript submitted). We undertook the present studies to validate this classifier in two prospective multicenter trials of patients undergoing bronchoscopy for suspected lung cancer and to assess how this classifier alters the diagnostic performance of bronchoscopy.

**Materials and Methods**

**Study Design, Population, and Protocol**

Current and former smokers undergoing bronchoscopy for suspicion of lung cancer were enrolled in AEGIS 1 and AEGIS 2, two independent, prospective, multicenter, observational studies (NCT01309087 and NCT00746759). Patients were enrolled at 28 sites in the U.S, Canada, and Ireland. Cytology brushes were used to collect normal appearing BECs from the mainstem bronchus and submerged in an RNA preservative. Results of the classifier were not reported to physicians or patients. Patients were screened prior to bronchoscopy to determine if they met the requirements of the study protocol. Exclusion criteria included subjects <21 year old, never smokers (defined as smoking < 100 cigarettes in lifetime), and patients with a concurrent cancer or a history of lung cancer. Patients who had been on a mechanical ventilator for >24 hours immediately prior to bronchoscopy, or could not consent or comply with the study, were excluded. Patients were followed until a final diagnosis was established or until 12 months post bronchoscopy. A diagnosis of lung cancer was established at the time of index bronchoscopy or by subsequent biopsy using TTNB, SLB, a second bronchoscopy, or other invasive procedures. The specific bronchoscopic methods used and subsequent surveillance imaging or procedures performed after non-diagnostic bronchoscopy was at the discretion of the treating physician. Patients diagnosed as cancer-free had a specific benign diagnosis or radiographic stability/resolution at 12 months of follow-up. Patients without a definitive diagnosis of cancer, a specific benign diagnosis or stability/resolution at 12 months of follow-up were excluded from further analysis. The treating physician assessed each patient's pre-test probability of malignancy (POM) prior to bronchoscopy using a five level scale (<10%, 10-39%, 40-60%, 61-85%, and >85%). The study protocol was approved by the institutional review board at each participating center, and all patients provided written informed consent before enrollment.

A total of 855 patients in AEGIS 1 and 502 in AEGIS 2 qualified and were enrolled between January 2009 and August 2012 (Figure 4) at twenty-eight medical centers in the United States, Canada, and Ireland (Table 12). The sites were a mix of tertiary/academic medical centers (n = 20), community based hospitals (n = 6), and Veteran's Administration hospitals (n = 2).

Additional patients were excluded from the study after enrollment based on the following criteria (see Figure 4). First, a total of 111 patients in AEGIS 1 and 71 patients in AEGIS 2 were ineligible due to the following protocol deviations: In AEGIS 1, 24 patients either did not meet the study enrollment criteria upon review, had specimens collected that did not meet the acceptance criteria in accessioning, or were enrolled but did not have a specimen collected. An additional 87 samples did not meet minimum QC criteria after RNA isolation, either due to a RIN score <4, or RNA yield < 1μg. In AEGIS 2, there were 3 ineligible patients due to having specimens collected that did not meet the acceptance criteria in accessioning, and 68 samples that did not meet the minimum QC criteria for RNA. Second, patients diagnosed with a non-primary lung cancer, including 40 patients in AEGIS 1 and 10 patients in AEGIS 2 were excluded. Additionally, upon review at 12 months, patients without a final diagnosis were also excluded from the study, including 91 subjects in AEGIS 1 and 71 patients in AEGIS 2. Approximately two thirds of these 162 patients (n=106) from AEGIS 1 and 2 were lost to follow-up after their initial bronchoscopy, while the remaining third (n=56) did not have a definitive diagnosis at 12 months post-bronchoscopy. Finally, we excluded 16 patients in AEGIS 1 and 9 patients in AEGIS 2 with gene expression data that failed the assay QC criteria (described below under Microarray Processing methods).

The AEGIS 1 cohort had previously been divided into equal training and test sets in a randomized manner. The training set (n=299 patients) was used to derive the gene expression classifier composed of 23 genes plus age and has been described elsewhere (manuscript submitted). In the training set, the classifier was found to have an AUC of 0.78 (95% CI, 0.71-0.85) in patients whose bronchoscopy did not lead to a diagnosis of lung cancer (n = 134), with a sensitivity of 93% and specificity of 57%. The current study is focused on validation of the locked classifier in two independent validation sets.

The baseline demographics and clinical characteristics of the final AEGIS 1 and AEGIS 2 validation sets are compared in Table 1. A separate comparison of patients diagnosed with cancer and benign disease within each cohort is provided in Table 12.

Table 12 - Characteristics of patients in the AEGIS 1 and AEGIS 2 test sets.

| | AEGIS 1 Test Set | | | AEGIS 2 Test Set | | |
|---|---|---|---|---|---|---|
| | Ca+ | Ca- | P | Ca+ | Ca- | P |
| N | 220 | 78 | | 267 | 74 | |
| Sex | | | 0.506 | | | 0.091 |
| Female | 95 | 30 | | 77 | 29 | |
| Male | 125 64 | 48 57 | | 190 65 | 45 60 | |
| Age (IQR)[a] | (15) | (14) | <0.001 | (13) | (18) | <0.001 |
| Race[b] | | | 0.878 | | | 0.426 |
| White | 166 | 60 | | 206 | 61 | |
| Black | 42 | 13 | | 55 | 11 | |
| Other/unknown | 12 | 5 | | 6 | 2 | |
| Smoking Status | | | 0.065 | | | 0.026 |
| Current | 115 | 31 | | 141 | 28 | |
| Former | 105 | 47 | | 126 | 46 | |
| Smoking History (PY) | 45 | 30 | | 50 | 20 | |
| (IQR) [a] | (30) | (36) | <0.001 | (35) | (30) | <0.001 |

a) Reported as the median value (and interquartile range; IQR). P-values calculated using the Mann-Whitney test.
b) P-value calculated for white vs. non-white.

Clinical and demographic data was collected for each patient and recorded on a study clinical report form (CRF). Additional pathology and radiology reports were maintained in the medical records of each patient and were available for review. All source documents were monitored and entered into databases maintained by the study sponsor. Size and location of the pulmonary lesions were obtained from the CT scan report. Subjects were followed up to twelve months post bronchoscopy to collect data for a clinical diagnosis. A diagnosis of lung cancer was based on results from pathology and copies of pathology reports were collected from the medical centers. The specimen leading to a clinical diagnosis of cancer was either obtained during bronchoscopy or from a subsequent invasive when bronchoscopy was non-diagnostic. Data on all clinical procedures performed after a non-diagnostic bronchoscopy was collected during the study. The subsequent evaluation of patients with non-diagnostic bronchoscopies was at the discretion of the treating physician at each study center. In some cases, more than one procedure after bronchoscopy was required to successfully render a diagnosis, but in all cases the procedure that led to the diagnosis of lung cancer was collected. Invasive follow-up procedures were defined as either a second bronchoscopy, TTNB, or surgical lung biopsy (SLB), which could include a mediastinoscopy, thoracoscopy, thoracotomy, or video-assisted thorascopic surgery (VATS).

The records of patients who were not diagnosed with cancer underwent an adjudication process in order to declare subjects as cancer-free. The process consisted of a review of the available medical records for each patient by a panel of five pulmonologists. Two pulmonologists from this panel independently reviewed each case and the patient was determined to be cancer-free if he/she met one of the following criteria: patient was diagnosed with an alternative diagnosis that explained the initial suspicious abnormality, the abnormality was determined to be stable, or the abnormality resolved. Patients whom did not meet these criteria at the completion of the 12-month follow-up period had no final diagnosis and were excluded from further analysis in the study.

Histology (Table 13) and cancer stage (Table 14) data was analyzed for patients diagnosed with primary lung cancer. Data was extracted from the study CRFs and were confirmed by review of complete medical records. Of the total cancers, 80% (175/220; 95% CI, 74-84%) in AEGIS 1 and 83% (222/267; 95% CI, 78-87%) in AEGIS 2 were NSCLC. There were 17% (38/220; 95% CI, 13-23%) and 16% (42/267 95% CI, 12-21%) SCLC cancers, respectively. Of the patients with NSCLC with known cancer stage data, early stage (stage I or 2) was present in 40% (53/139 95% CI, 32-48%) in AEGIS 1 and 37% (67/199; 95% CI, 30-44%) in AEGIS 2.

Table 13 - Summary demographics and clinical characteristics of study participants: A comparison of AEGIS 1 vs. AEGIS 2 patients was performed across all clinical characteristics. Significant differences are indicated by * p<.05, ** p <.001. The p-value for race was calculated for white versus non-white, and for lung cancer histology using NSCLC versus SCLC.

| | AEGIS 1 | AEGIS 2 |
|---|---|---|
| **N** | 298 | 341 |
| **Sex*** | | |
| **Female** | 125 | 106 |
| **Male** | 173 | 235 |
| **Age*,median(IQR)** | 62 (16) | 64 (15) |
| **Race** | | |
| **White** | 226 | 267 |
| **Black** | 55 | 66 |
| **Other** | 15 | 4 |
| **Unknown** | 2 | 4 |
| **Smoking Status** | | |
| **Current** | 146 | 169 |
| **Former** | 152 | 172 |
| **Tobacco Pack-years, median, (IQR)** | 40 (36) | 45 (38) |
| **Lesion Size**** | | |
| **<2 cm** | 48 | 83 |
| **2 to 3 cm** | 41 | 39 |
| **>3 cm** | 155 | 188 |
| **Infiltrate** | 32 | 28 |
| **Unknown** | 22 | 3 |
| **Lesion Location**** | | |
| **Central** | 98 | 127 |
| **Peripheral** | 86 | 108 |
| **Central & Peripheral** | 90 | 102 |
| **Unknown** | 24 | 4 |
| **Lung Cancer Histology** | 220 | 267 |
| **Small cell lung cancer** | 38 | 42 |
| **Nonsmall cell lung cancer** | 175 | 222 |
| ***Adenocarcinoma*** | *69* | *100* |
| ***Squamous*** | *72* | *81* |
| ***Large cell*** | *8* | *8* |
| ***NSCLC, not otherwise specified*** | *26* | *33* |
| **Unknown** | 7 | 3 |
| **Benign Diagnoses** | 78 | 74 |
| **Infection** | 18 | 14 |
| **Sarcoidosis** | 16 | 15 |
| **Resolution or Stability** | 27 | 24 |
| **Other** | 17 | 21 |

Table 14 - Stage data of patients diagnosed with primary lung cancer.

| Histology | Stage | AEGIS 1 | AEGIS 2 |
|---|---|---|---|
| **SCLC** | | 38 | 42 |

(continued)

| Histology | Stage | AEGIS 1 | AEGIS 2 |
|---|---|---|---|
| | *Limited* | 16 | 14 |
| | *Extensive* | 19 | 23 |
| | *Unknown* | 3 | 5 |
| **NSCLC** | | 175 | 222 |
| | *1* | 37 | 49 |
| | *2* | 16 | 18 |
| | *3* | 44 | 65 |
| | *4* | 42 | 67 |
| | *Unknown* | 36 | 23 |
| **Unknown** | | 7 | 3 |
| **Total** | | 220 | 267 |

Data about each of the benign diagnoses was extracted from medical records and summarized in Table 15. In both studies approximately two-thirds of cancer-free subjects had alternative diagnoses, with the specific alternative diagnoses enumerated in Table S4 for each study. Diagnosis of patients with abnormalities that were determined to have resolved or were stable was based on review of medical records, including follow-up imaging, at 12 months post-bronchoscopy, and approximately one-third of cancer-free patients were in this category in both studies.

Table 15 - Alternative diagnoses of patients with benign disease

| Category | AEGIS 1 | AEGIS 2 |
|---|---|---|
| **Resolution or Stability*** | 27 | 24 |
| **Alternative Diagnosis** | 51 | 50 |
| ***Sarcoidosis*** | *16* | *15* |
| ***Inflammation*** | *4* | *4* |
| ***Benign growth*** | *5* | *6* |
| ***Fibrosis*** | *4* | *5* |
| ***Other*** | *4* | *6* |
| ***Infection*** | *18* | *14* |
| ***Fungal*** | *8* | *3* |
| ***Mycobacteria*** | *5* | *4* |
| ***Bacterial*** | *5* | *7* |
| **Total** | 78 | 74 |

During the clinically indicated bronchoscopy, study participants underwent collection of mucosal brushings of the right or left main-stem bronchus using a standard, disposable cytology brush rubbed against the bronchial wall. Two brushings were obtained from each subject; physicians were trained to obtain brushings of normal appearing epithelial tissue and to avoid sampling tumor tissue or dysplastic cells. All samples were stored in an RNA preservative (RNAprotect; Qiagen) and were shipped to a central CLIA-certified laboratory for accessioning and processing. Centers were requested to store samples at 4°C for up to 14 days, and to ship at sub-ambient temperatures (4-20°C) using 2-day shipping and insulated shipping containers (NanoCool) provided by the sponsor.

**Laboratory Methods**

All BEC specimens were processed to isolate and analyze RNA for quality and yield prior to gene expression analysis; Only specimens with an RNA yield of at least 1 μg and RIN score of >4 were run on Gene-ST 1.0 microarrays. All microarray data has been deposited in GEO as GSE66499.

The samples were lysed and RNA was isolated using a column-based method (miRNeasy Kit; Qiagen) and the manufacturer's recommended protocol. The large RNA fraction was analyzed on a spectrophotometer (Nanodrop; ThermoFisher) to determine the concentration, purity, and yield. Samples were also analyzed for RNA integrity (Bioanalyzer), reported as the RIN score. Samples with a yield of <1μg and RIN score <4 were excluded from the study.

Approximately 10% of specimens in AEGIS 1 and 13% in AEGIS 2 were excluded due to insufficient RNA quality or quantity. The RNA was then stored frozen (-80°C) until further processing.

200ng of total RNA was converted to sense strand cDNA using Ambion WT Expression kit (Life technologies Cat. # 4440536), and subsequently labeled with Affymetrix GeneChip WT terminal labeling kit (Affymetrix Cat. #900671). For hybridization, a hybridization cocktail was prepared and added to the labeled cDNA target using the Hybridization, Wash and Stain kit (Affymetrix Cat. #900720), applied to Human Gene 1.0 ST arrays (Affymetrix Cat. # 901087), and incubated at 45°C for 16 hours. Following hybridization, arrays were washed and stained using standard Affymetrix procedures before they were scanned on the Affymetrix GeneChip Scanner. Data was extracted using Expression Console software (Affymetrix).

The Affymetrix Human Gene 1.0 ST array (Affymetrix Cat. # 901087) utilizes numerous probes that are disclosed at www.affymetrix.com/site/include/byproduct.affx?product=hugene-1_0-st-v1. Multiple probes are present that correspond to a segments of specific genes, which is to say that that is not a 1:1 ratio of one probe per gene, rather multiple probes are present that correspond to multiple segments of a single gene. In one example, the LYPD2 gene is represented by three probe sets in the Human Gene 1.0 ST array (Release 32), probeset IDs 8153343, 8153344, and 8153345 as disclosed in the Affymetrix Human Gene 1.0 ST array (HuGene-1_0-st-v1 Probeset Annotations, release 32 on 09/30/2011, release 33 on 03/27/2013, and release 34 on 04/07/2014). Information correlating probe-sets and nucleic acid sequences can be found at Affymetrix.com, including at www.affymetrix.com/site/include/byproduct.affx?product=hugene-1_0-st-v1. Furthermore, data sets are available on the NCBI Gene Expression Omnibus website under Platform GPL6244 detailing the probes and genes that may be utilized in practicing the methods of the present disclosure at www.ncbi.nlm.nih.gov/geo/query/acc.cgi?acc=GPL6244. These documents, including those correlating the probesets and gene symbols are incorporated herein by reference.

Microarray data (CEL files) corresponding to the samples in the final data set of each cohort were normalized using RMA [22]. The AEGIS 1 samples were run in a total of 5 batches and ComBat [23] was used to correct for batch effects. The AEGIS 2 samples were all run in a single microarray batch. After normalization, outliers were identified as having a genome-wide pairwise correlation of less than 0.955 in global gene expression. All microarray data has been deposited in GEO under accession # GSE66499.

Normalization and preprocessing of the microarray data are described in the Supplementary Appendix. Subjects enrolled in AEGIS 1 had previously been randomly assigned into independent training and validation sets (Figure 4) and the classifier algorithm was derived strictly within the AEGIS 1 training set and locked, as described previously. Scores for each sample in the AEGIS 1 validation set and for all AEGIS 2 samples were generated using this pre-specified classifier that was based on the expression of 23 genes and patient age. These scores were dichotomized as test-positive and test-negative using a pre-specified threshold value.

Performance of the classifier was evaluated using receiver operator characteristic (ROC) curves, calculation of area under the curve (AUC) [14], and estimates of sensitivity, specificity, negative predictive value (NPV), positive predictive value (PPV) and the negative likelihood ratio (NLR) which was defined as (1-sensitivity)/specificity. A Mann-Whitney non-parametric test was used for analysis of continuous variables and Fisher's exact test was used for categorical variables. All confidence intervals are reported as the two-sided binomial 95% confidence intervals (95%CI). Statistical analysis was performed using R software (Version 3.01).

Physicians were asked to assess the pre-test POM for all subjects enrolled based on expert opinion and available medical records prior to bronchoscopy, using one of the following categories: <10%, 10-39%, 40-60%, 61-85%, and >85%. The results were collapsed into categories of <10%, 10-60%, and >60%. We then calculated the actual prevalence of cancer in each of the POM categories in order to compare the actual prevalence of cancer in the stratified POM levels (Table 16). Additionally, we found that the prevalence of lung cancer post-non diagnostic bronchoscopy was 3%, 29% and 80%) in the <10%, 10-60% and >60% POM groups respectively.

Table 16 - Correlation of physician assessed POM and the prevalence of cancer.

| Pre-test POM[a] | CA+ patients | Total patients | Prevalence[b] |
|---|---|---|---|
| **<10%** | 3 | 62 | 5% |
| **10-60%** | 41 | 101 | 41% |
| **>60%** | 404 | 425 | 95% |
| **Unknown** | 39 | 51 | 77% |

a) POM = probability of malignancy; assessed by treating physicians prior to bronchoscopy.
b) Prevalence was calculated as the fraction diagnosed with lung cancer within each of the POM categories.

The prediction accuracy of the bronchial genomic classifier stratified by lesion size is summarized in Table 2, and by pre-test POM in Table 3. The sensitivity of the classifier is also reported for patients diagnosed with lung cancer stratified by stage and histology, in Tables S5 and S6. The classifier in combination with bronchoscopy leads to a sensitivity > 90% for all categories.

Table 17 - Sensitivity of the classifier, bronchoscopy, and the combined approaches according to radiological imaging characteristics. Sensitivity of bronchoscopy was determined for lung cancer patients in each category. Sensitivity of the classifier was determined for the patients with lung cancer who were not diagnosed during bronchoscopy. Sensitivity of the classifier combined with bronchoscopy was calculated for all lung cancer patients in each category.

| Size (mm) | $N_{total}$ | $N_{cancer}$ | Bronchoscopy (95% CI) | Classifier (95% CI) | Classifier & bronchoscopy combined (95% CI) |
|---|---|---|---|---|---|
| All patients | 639 | 487 | 75% (71-79) | 89% (82-94) | 97% (95-98) |
| Size (mm) | | | | | |
| <2 cm | 131 | 73 | 55% (43-66) | 91% (76-98) | 96% (88-99) |
| 2-3 cm | 80 | 60 | 58% (46-70) | 92% (74-99) | 97% (88-100) |
| >3 cm | 343 | 313 | 82% (78-86) | 85% (74-93) | 97% (95-99) |
| Infiltrate | 60 | 25 | 84% (65-94) | 100% (45-100) | 100% (84-100) |
| Unknown | 25 | 16 | 80% (54-94) | 100% (38-100) | 100% (76-100) |
| Location | | | | | |
| Central | 225 | 174 | 84% (78-89) | 81% (63-92) | 97% (93-99) |
| Peripheral | 194 | 133 | 55% (46-63) | 90% (80-96) | 95% (90-98) |
| Both | 192 | 164 | 82% (75-87) | 97% (82-100) | 99% (96-100) |
| Unknown | 28 | 16 | 81% (56-94) | 67% (20-94) | 94% (70-100) |

## Results

### Characteristics of the Study Participants

298 patients from AEGIS 1 served as a first validation set and all 341 patients from AEGIS 2 meeting study criteria were used as a second validation set (Figure 4 & Table 13). The prevalence of lung cancer was 74% and 78% for the AEGIS 1 and AEGIS 2 cohorts, respectively. Patients with lung cancer were older (p<0.001), had higher cumulative tobacco exposure compared to patients without cancer (p<0.001) and were more likely to be current smokers (p=0.07 in AEGIS 1 and p=0.03 in AEGIS 2; Table 12). A summary of cancer stage and categories of benign diagnoses are shown in Tables 15 and 16, respectively.

### Performance of Bronchoscopy

A total of 639 patients underwent bronchoscopy for suspected lung cancer. Of those, 272 (43%; 95%CI, 39 to 46) were non-diagnostic, including 120 of 487 patients (25%; 95%CI, 21 to 29) ultimately diagnosed with lung cancer. The sensitivity of bronchoscopy for lung cancer was 74% (95%CI, 68 to 79) and 76% (95%CI, 71 to 81) in AEGIS 1 and AEGIS 2, respectively. Follow-up procedure data was available for 98% (267 of 272) of the patients with a non-diagnostic bronchoscopy. Invasive procedures following non-diagnostic bronchoscopy were performed in 170 of 267 patients (64%; 95%CI, 58 to 69), including 52 of 147 (35%; 95%CI, 24 to 38) with benign lesions and 118 of 120 (98%; 95%CI, 94 to 99) with cancer. SLB was performed in 76 patients, of which 27 (36%; 95%CI, 26 to 47) had benign lesions.

### Performance of Gene Expression Classifier

The classifier alone had an AUC=0.78 (95%CI, 0.73 to 0.83) and accurately identified 194 of 220 patients with cancer (88% sensitivity; 95%CI, 83 to 92), and 37 of 78 patients without cancer (47% specificity; 95%CI, 37 to 58) in AEGIS 1 (Figure 5). In AEGIS 2, the classifier had an AUC=0.74 (95%CI, 0.68 to 0.80) and correctly identified 237 of 267 patients with cancer (89% sensitivity; 95% CI, 84 to 92), and 35 of 74 without cancer (47% specificity; 95%, 36 to 59). The combination of the classifier with bronchoscopy increased the sensitivity to 96% (95%CI, 93 to 98) and 98% (95%CI, 96 to 99) in AEGIS 1 and 2, respectively compared to 74% and 76%, respectively, for bronchoscopy alone (p<0.001).

In patients with a non-diagnostic bronchoscopy, the classifier accurately identified cancer in 49 of 57 patients in AEGIS 1 (86% sensitivity; 95%CI, 74 to 94) and in 62 of 67 patients in AEGIS 2 (92% sensitivity; 95%CI, 82 to 97). As there was no significant difference between patients in the two cohorts with regard to the classifier AUC either across all patients (p = 0.32) or for patients with a non-diagnostic bronchoscopy (p = 0.61) (Figure 5), we combined the two cohorts for subsequent analyses of sub-groups. The sensitivity of bronchoscopy alone was lower in lesions that were < 3 cm (p < 0.001) or peripherally located (p < 0.001) (Table 17). In contrast, the sensitivity of the classifier alone and the classifier combined with bronchoscopy were consistently high and not significantly associated with size or location of the lesion (Table 17), cancer stage (Table 18) or histological subtype (Table 19).

Table 18 - Sensitivity of the classifier and bronchoscopy according to cancer stage.

| Histology | Stage | N | Bronchoscopy | Classifier | Combined |
|---|---|---|---|---|---|
| NSCLC | 1 | 86 | 41% | 88% | 93% |
| | 2 | 34 | 68% | 82% | 94% |
| | 3 | 109 | 83% | 94% | 99% |
| | 4 | 109 | 86% | 93% | 99% |
| | Unknown | 59 | 80% | 92% | 98% |
| Total NSCLC | | 397 | 73% | 89% | 97% |
| SCLC | Limited | 30 | 80% | 83% | 97% |
| | Extensive | 42 | 93% | 100% | 100% |
| | Unknown | 8 | 88% | n/a | 88% |
| Total SCLC | | 80 | 88% | 80% | 98% |
| Unknown | | 10 | 70% | 100% | 100% |
| Total Cancers | | 487 | 74% | 86% | 97% |

Table 19 - Sensitivity of the classifier and bronchoscopy according to histology.

| Histology | N | Bronchoscopy | Classifier | Combined |
|---|---|---|---|---|
| Adenocarcinoma | 169 | 65% | 86% | 95% |
| Squamous | 153 | 80% | 90% | 98% |
| Large Cell | 16 | 75% | 100% | 100% |
| NSCLC-NOS | 59 | 78% | 100% | 100% |
| SCLC | 80 | 88% | 80% | 98% |
| Unknown | 10 | 70% | 100% | 100% |

**Accuracy of the Classifier in Patients With Intermediate Probability of Cancer**

We combined the physician-assessed probability of malignancy (POM) into categories of low (<10%), intermediate (10-60%), and high (>60%) POM (Table 3), to align with guideline recommendations for assessing lung cancer risk [1]. Bronchoscopy was non-diagnostic for cancer in 83% of patients with an intermediate pre-test POM (n=101), despite a 41% cancer prevalence rate. In this group of patients, the classifier achieved a NPV of 91% (95%CI, 75 to 98) among those with a non-diagnostic bronchoscopy and a PPV of 40% (95%CI, 28 to 54) (Table 20).

Table 20 - Performance of bronchoscopy and the classifier stratified pre-test POM.

| | | Pre-test POM Category | | | |
|---|---|---|---|---|---|
| | N | <10% | 10-60% | >60% | Unknown |
| | | Patient population | | | |
| Total Patients[a] | 639 | 62 | 101 | 425 | 51 |
| Lung Cancer | 487 | 3 (5%) | 41 (41%) | 404 (95%) | 39 (76%) |
| Benign | 152 | 59 (95%) | 60 (59%) | 21 (5%) | 12 (24%) |
| | | Bronchoscopy performance | | | |
| Bronchoscopy sensitivity (95%CI) | 272 | 33% (6-80) | 41% (28-57) | 79% (74-82) | 79% (64-89) |
| Patients with non-diagnostic bronchoscopy[b] | | 61 (98%) | 84 (83%) | 107 (25%) | 20 (39%) |

(continued)

| | Classifier performance | | | |
|---|---|---|---|---|
| Classifier sensitivity (95%CI)[c] | 100% (29-100) | 88% (68-96) | 90% (82-95) | 88% (51-100) |
| Classifier specificity (95%CI)[d] | 56% (43-68) | 48% (36-61) | 29% (14-50) | 33% (14-61) |
| Classifier NPV (95%CI)[e] | 100% (88-100) | 91% (75-98) | 40% (20-64) | 80% (36-98) |
| Classifier PPV (95%CI)[f] | 7% (1-24) | 40% (28-54) | 84% (75-90) | 47% (25-70) |
| Combined classifier & bronchoscopy sensitivity | 100% (38-100) | 93% (80-98) | 98% (96-99) | 97% (86-100) |

(a) There were 639 total patients across the POM categories shown.
(b) Bronchoscopy was non-diagnostic for 272 of 639 patients (43%; 95%CI, 39 to 46), including 120 of 487 patients (25%; 95%CI, 21 to 29) diagnosed with lung cancer.
(c) The classifier accurately predicted 107 of 120 total patients overall (89%; 95%CI, 82 to 94) with cancer. Sensitivity is reported in each POM category for patients with non-diagnostic bronchoscopy procedures.
(d) The classifier accurately predicted 72 of 152 patients overall (47%; 95%CI, 40 to 55) without cancer. Specificity is reported in each POM category for patients with non-diagnostic bronchoscopy procedures.
(e) NPV, and (f) PPV is reported for patients with non-diagnostic bronchoscopy procedures.

Although the classifier had a high NPV in patients with a non-diagnostic bronchoscopy, there were 13 patients with a non-diagnostic bronchoscopy who had lung cancer and a negative classifier score (i.e. false negatives). The majority (10 of 13) had a high (>60%) POM with only three patients in the 10-60% pre-test POM group.

The NLR of the classifier in combination with bronchoscopy was calculated to determine the range of pre-test POM in which the post-test probability would be <10%. The NLR of bronchoscopy (0.244; 95%CI, 0.21 to 0.29) improves when combined with the classifier to 0.056 (95%CI, 0.03 to 0.10). As a result, when both bronchoscopy and the classifier are negative, the post-test POM is reduced to <10% for patients with a pre-test POM up to 66% (Figure 6).

**Evaluation of Results**

This study describes the validation of a bronchial genomic classifier that identifies patients without lung cancer among those undergoing bronchoscopy in two independent prospective cohorts. We find that the gene-expression classifier has high sensitivity across different sizes, locations, stages, and cell types of lung cancer in the combined cohorts. The combination of the classifier and bronchoscopy has a sensitivity of 96% and 98% in the AEGIS-1 and AEGIS-2 validation cohorts respectively. We also report several additional findings that support the clinical need for this type of classifier. First, our studies confirm the previously reported observations that non-diagnostic bronchoscopy is common (particularly in patients of intermediate pre-test POM) and leads to further invasive testing including SLB, often in patients ultimately found to not have lung cancer. Second, in contrast to the high sensitivity of the classifier, we find that bronchoscopy performed poorly in small, peripheral or early stage cancers. Third, the classifier has a high NPV in patients with intermediate POM and a non-diagnostic bronchoscopy. These findings suggest that this classifier has the potential to assist clinical decision making in patients with intermediate POM in whom the prevalence of lung cancer is 41% but the sensitivity of bronchoscopy is only 41%. Due to the high NPV, a negative classifier score in patients with a non-diagnostic bronchoscopy and intermediate POM warrants a more conservative diagnostic strategy with active surveillance via imaging.

Although the high NPV of the classifier would help avoid unnecessary invasive procedures in patients with an intermediate POM that are classifier negative, there were a small number of patients in this group who have lung cancer; the negative gene-expression classifier result may delay further invasive testing in these patients. However, this group of patients would undergo active surveillance via imaging, which is the standard practice when an immediate invasive strategy is not employed [1,15]. This would allow for identification of lesion growth, triggering additional invasive testing to establish a definitive diagnosis. In contrast to the high NPV observed in patients with an intermediate POM, the classifier has a modest PPV of 40% in this setting. Thus, a positive result with the classifier does not warrant alteration in the diagnostic strategy; further testing would need to be based on traditional factors used to choose between an invasive versus an imaging surveillance strategy.

This gene expression classifier is measured in proximal BECs and not from cells within the pulmonary lesion. The ability of gene-expression alterations in cytologically-normal proximal airway to detect the presence of lung cancer within the lung parenchyma stems directly from the "field of injury" paradigm [13]. Spira, et al. has previously shown that there is a distinct pattern of gene-expression alterations in cytologically-normal bronchial epithelial cells among current and previous smokers with lung cancer [13,16]. Additionally oncogenic signaling pathways are activated in the proximal airway epithelium of smokers with lung cancer and smokers with premalignant airway lesions [17]. More recently, Kadara, et al. [18] demonstrated that genes whose expression is altered in non-small cell lung cancer itself and the adjacent small airway epithelium are enriched among those genes that are altered in the proximal airway epithelium, suggesting that the gene-expression changes within the proximal airway reflect, in part, the altered transcriptome observed in lung tumors.

A bronchoscopy was considered as "diagnostic" only when the procedure yielded a lung cancer diagnosis. There were a relatively small number of bronchoscopies that were potentially diagnostic of a specific benign etiology, but most of these patients received further invasive testing including patients ultimately diagnosed with lung cancer, suggesting that the concern for lung cancer remained elevated despite the initial benign finding on bronchoscopy. Finally, we did not assess the accuracy of a model incorporating the classifier in combination with clinical variables. Although clinical risk prediction models have been developed for solitary pulmonary nodules [1,20,21], there are no validated models for patients selected to undergo diagnostic bronchoscopy, which includes patients with a broad range of findings, including larger lesions (i.e. >3 cm), infiltrates or other features such as lymphadenopathy. Thus, most patients are selected for bronchoscopy based on the physician’s qualitative assessment of lung cancer probability. Importantly, we demonstrate that our classifier performs well in patients with intermediate POM by physician assessment, a process that incorporates available clinical risk factors.

The potential impact of this work is bolstered by a number of key strengths in its study design. First, these were two independent prospective validation studies in which the classifier was measured in the setting in which the test would be used clinically (i.e. prior to diagnosis). This is a critical step in moving molecular biomarkers from discovery studies to their ultimate clinical application. Second, the large multicenter design enabled inclusion of patients undergoing bronchoscopy from different practice settings and geographic locations. Third, our data demonstrates the high prevalence of lung cancer among patients with a non-diagnostic bronchoscopy and intermediate pre-test POM, and show that the classifier has an NPV of 91% in this setting where there is the greatest uncertainty about cancer status. In this setting, the opportunity to use a classifier with high sensitivity for lung cancer allows for the confident identification of patients without lung cancer that might be followed by active surveillance, thereby avoiding potentially harmful invasive follow up testing.

**Example 3: Derivation of Bronchial Genomic Classifier for Lung Cancer Patients In Patients Undergoing Diagnostic Chronchoscopy**

**Introduction**

Lung cancer remains the leading cause of cancer mortality in the United States, with an estimated 224,000 new diagnoses, and 160,000 deaths in 2014, 90% of which are due to smoking [24]. Recently, the National Lung Cancer Screening Trial showed that low dose Computed Tomography (CT) screening results in a 20% relative mortality reduction in high risk individuals [25]. The mortality reduction, however, was accompanied by a high rate (~96%) of false-positive CT findings, which in turn has generated concern for the overutilization of invasive diagnostic procedures [26].

Patients with suspected lung cancer are often referred for bronchoscopy where the primary aim is to sample a suspicious pulmonary lesion for pathological analysis. It is estimated that 500,000 bronchoscopies are performed per year in the U.S. [27], of which roughly half are for the diagnosis of lung cancer. Bronchoscopy is considered to be safer than other invasive sampling methods, such as transthoracic needle biopsy (TTNB), or surgical techniques. However the diagnostic sensitivity of bronchoscopy is sub-optimal, ranging from 34% (for <2cm peripheral nodules) to 88% (for larger, centrally located lesions) [28]. Adoption of guidance techniques has expanded the applicability of bronchoscopy to more challenging suspicious lesions (i.e., solitary pulmonary nodules which are often peripheral in the lung), but the overall clinical sensitivity of bronchoscopy for lung cancer has not improved substantially [29,30]. When bronchoscopy is non-diagnostic, physicians are often left with the ambiguity of whether to pursue further invasive diagnostic procedures, with associated complications [31,32], or choose imaging surveillance. In current practice when these invasive procedures are performed, approximately a third of patients are determined to have benign disease [33], suggesting that these procedures are avoidable. Methods that reduce this ambiguity by substantially improving the diagnostic yield of bronchoscopy could improve patient care.

It has previously been demonstrated that cigarette smoke creates a molecular field of injury in airway epithelial cells that line the entire respiratory tract [34]. The reversible and irreversible impact of cigarette smoke on the bronchial airway transcriptome has been characterized and a set of gene-expression alterations in the bronchial epithelium have been identified in current and former smokers with lung cancer [35]. These cancer-associated gene expression profiles have previously been shown to yield a sensitive classifier for detecting lung cancer when bronchoscopy is non-diagnostic. The high sensitivity of this classifier, measured in a biospecimen readily accessible during bronchoscopy, results in a very low probability of lung cancer when the test result is negative, and suggests that physicians might be enabled to confidently pursue active surveillance and reduce risky invasive procedures in subjects without lung cancer.

**Materials and Methods**

**Training Set Patient Population**

Patients were enrolled in the AEGIS trials (Airway Epithelium Gene Expression In the DiagnosiS of Lung Cancer), designed as prospective, observational, cohort studies (registered as NCT01309087 and NCT00746759) of current and former cigarette smokers with a suspicion of lung cancer undergoing bronchoscopy as part of their diagnostic workup. A set of patients from one of the cohorts ("AEGIS 1") was selected for the exclusive purpose of training a gene expression classifier. The study was approved by IRB at each of the participating medical centers, and all patients signed an informed consent prior to enrollment. All enrolled patients were followed post-bronchoscopy until a final diagnosis was made, or for 12 months. Patients were diagnosed as having primary lung cancer based on cytopathology obtained at bronchoscopy or upon subsequent lung biopsy (such as TTNB or surgical lung biopsy (SLB) when bronchoscopy did not lead to a diagnosis of lung cancer). Patients were diagnosed as having benign disease based on a review of medical records and follow-up procedures at 12 months post-bronchoscopy (described in more detail in Additional File 1). Bronchoscopy was considered "diagnostic" when clinical samples collected at the time of the bronchoscopy procedure yielded a confirmed lung cancer diagnosis via cytology or pathology.

Patients enrolled in the NIH-registered AEGIS studies (NCT01309087 and NCT00746759) were patients undergoing clinically indicated bronchoscopy for suspicion of lung cancer who were at least 21 years of age and had smoked at least 100 cigarettes in their lifetime. Study exclusions included patients who had previously been diagnosed with primary lung cancer, who had been on a mechanical ventilator for $\geq$ 24 consecutive hours immediately prior to bronchoscopy, or who could not consent or comply with the study. Additional patients were excluded prior to training the classifier to exclude patients with a malignancy other than primary lung cancer. This included the exclusion of patients with a history of any malignancy, confirmed metastatic cancer to the lung, or found to have an active non-lung primary cancer after enrollment. Also, patients without a final definitive diagnosis were excluded. Finally, after specimen processing, those with insufficient yield (<1$\mu$g) or quality (RIN<4) of RNA were excluded from further analysis.

Patients were followed for up to twelve months post bronchoscopy and records were reviewed to confirm or determine a final clinical diagnosis. A diagnosis of cancer was based on cytopathology of cells/tissue collected either during bronchoscopy, or in follow-up procedures when bronchoscopy was non-diagnostic. Follow-up procedures leading to diagnosis consisted of a second bronchoscopy, transthoracic needle aspiration (TTNA), surgical lung biopsy (SLB), or a combination of procedures. Records of patients who were not diagnosed with cancer, and who had been followed for 12 months, underwent an adjudication process by a panel of five pulmonologists. The process consisted of a review of the available medical records and patients were only declared to be cancer-free if the patient met one of the following criteria: diagnosed with an alternative diagnosis that explained the initial suspicious abnormality, the abnormality was determined to be stable, or the abnormality resolved. Patients who did not meet these criteria at the completion of the 12-month follow-up period were labeled as "indeterminate" and were excluded from training, due to lack of diagnostic "truth".

**Sample Collection**

Physicians at each of 25 participating medical centers were instructed to collect normal appearing bronchial epithelial cells (BEC) from the right mainstem bronchus (or the left side if any abnormalities were observed on the right) during bronchoscopy using standard bronchoscopic cytology brushes. Following collection, the cytology brushes were cut and placed in an RNA preservative (Qiagen RNAProtect, Cat. 76526) immediately after collection and stored at 4°C. Specimens were then shipped at 4-20°C to a central laboratory for further processing.

A shipping container was provided to all sites enabling the transport of specimens at 4-20°C within a 48 hour period. Sites were asked to send specimens using 2-day shipping services. Upon receipt in the central laboratory, specimens were inspected and accessioned into a laboratory information system. Accepted specimens were stored at 4°C prior to RNA isolation, which was typically conducted within 7 days of receipt. Records of all storage, and shipping times were retained, and the cumulative time between specimen collection and RNA isolation was less than 30 days (consistent with manufacturer's recommendations for the RNA preservative).

**RNA Isolation**

BECs were separated from cytology brushes using a vortex mixer and were then pelleted and processed using QIAzol lysis reagent (Qiagen). RNA was isolated by phenol/chloroform extractions and purified on a silica membrane spin-column (Qiagen miRNeasy kit, Cat. #217004) according to manufacturer's recommendations. RNA was analyzed on a NanoDrop ND-1000 spectrophotometer (Thermo Scientific) to determine concentration and purity, and RNA integrity (RIN) was measured on a 2100 Bioanalyzer (Agilent Technologies). Each sample was then stored at -80°C until processing further on microarrays.

**cDNA Preparation**

Total RNA was converted to sense strand cDNA, amplified using the Ambion WT Expression kit (Life Technologies Cat. # 4440536) designed for use with Affymetrix microarrays. Starting with 200ng of total RNA, single stranded cDNA was prepared through reverse transcription using T7 promoter primers protocol. Single-strand cDNA was converted to double stranded cDNA using DNA polymerase.

**Microarray Processing**

cDNA obtained from the total RNA was labeled with Affymetrix GeneChip WT terminal labeling kit (Affymetrix Cat. #900671). The labeled cDNA was hybridized to Gene 1.0 ST microarrays (Affymetrix Cat. #901085) and analyzed on an Affymetrix GeneChip Scanner. Individual CEL files for each of the patient samples were normalized using the standard Affymetrix Gene 1.0 ST CDF and RMA [38].

Total RNA was converted to sense-strand cDNA using a commercial kit (Ambion WT; Life Technologies, Cat. # 4440536) designed for use with Affymetrix microarrays. Starting with 200ng of total RNA, single stranded cDNA was prepared through reverse transcription using T7 promoter primers protocol. Single-strand cDNA was converted to double stranded cDNA using DNA polymerase. Double stranded cDNA acts as a template for in vitro transcription of cRNA which was then purified to remove enzymes, salts, inorganic phosphates and unincorporated nucleotides. The yield of cRNA was measured using UV-adsorption and labeled sense-stranded cDNA was then generated using 10 μg of the purified cRNA by reverse transcription with random primers and a mix of dUTP/dNTPs, fragmented, and labeled using the GeneChip WT Terminal labeling kit (Affymetrix, Cat. #900671). The labeled cDNA was hybridized to Gene 1.0 ST microarrays (Affymetrix Cat. #901085) using the Hybridization, Wash and Stain kit (Affymetrix Cat. #900720), and incubated at 45°C for 16 hours. Following hybridization, arrays were washed and stained using standard Affymetrix procedures before being scanned on the Affymetrix GeneChip Scanner, and data was extracted using Expression Console software (Affymetrix). Thus, microarrays described herein are not measuring the expression of natural molecules, rather the microarrays measure the expression of non-naturally occuring cDNA molecules.

**Classifier Development**

A gene expression classifier was derived in a multi-step process. Initial modeling consisted of using the training data to select genes ("gene expression correlates") which were associated with three clinical covariates (gender, tobacco use, and smoking history) to identify gene expression correlates of these clinical variables. Lung cancer-associated genes were then selected, and finally a classifier for predicting the likelihood of lung cancer based on the combination of the cancer genes, the gene expression correlates, and patient age was determined. All aspects of this classifier development procedure were determined using cross validation and using only data from the training set samples.

**Clinical Factor Gene Expression Correlates (CFGC)**

Covariates of lung cancer in this study population, including sex (male/female), smoking status (current/former), and pack years (<10/>10), were modeled to identify gene expression correlates for the clinical factors. Empirical Bayes t-tests were used to identify genes whose expression was significantly associated with each of the clinical factors. Next, genes were selected in a sparse manner [36] that could be used to predict the value of each clinical factor. Finally, the predicted values from the gene expression correlates for gender (GG), smoking status (GS), and pack-years (GPY) were computed for each patient and used in selecting genes with lung-cancer associated gene expression and in the lung cancer classifier described below.

**Selection of Lung Cancer Genes**

A logistic regression model with lung cancer status (1=cancer-positive and 0=cancer-negative) as the dependent variable was fit using the training data, CFGC's, and patient age as predictors. Next an empirical Bayes linear model was fit using gene expression values as the independent variable and the logistic regression model residuals as the dependent variable. This was used to select genes most directly correlated with disease status and independent of clinical covariates. The top lung cancer-associated genes from this analysis were grouped using hierarchical clustering. Genes were selected in an iterative manner to maximize AUC using cross-validation to estimate prediction accuracy. The aim was to select clusters that cumulatively provide the best classifier performance, and specific genes that best represent each of the clusters. A gene titration analysis was also performed to determine the number of genes per cluster providing optimal performance. For the clusters selected, the top genes were averaged, yielding cluster mean estimates for each patient/cluster combination. Functional analysis of genes within each of the cancer clusters was performed using DAVID [37] to identify biological terms describing the cancer-associated genes in the classifier.

**Lung Cancer Classifier**

A lung cancer classifier was developed using lung cancer status as the outcome variable and the cancer gene expression estimates, patient age, and CFGC's for gender (GG), smoking status (GS), pack years (GPY) as predictors. The model was fit using a penalized logistic regression model; the penalization factor (lambda) was 0 for the clinical/ gene expression correlates and 10 for each of the gene expression cluster estimates. The resulting score is on a 0 to 1 scale. A score threshold for predicting lung cancer status was established to achieve a sensitivity of approximately 90% for patients with a non-diagnostic bronchoscopy. An evaluation of the benefit of the gene expression classifier to predict lung cancer compared to clinical factors alone was performed by generating a "clinical model" that included age, gender, smoking status, and pack-years (determined clinically) in a logistic regression model to predict lung cancer status. The difference in performance between the complete gene expression classifier and the clinical factors classifier to predict lung cancer status was assessed by comparing the AUC's of each model in the training set.

**Analysis of an Independent Test Set**

Data from a prior study [35] were used as an independent test set to assess the performance of the locked classifier derived in this study. In that study BECs were collected at bronchoscopy from patients undergoing bronchoscopy for suspicion of lung cancer, and RNA was analyzed on microarrays (Affymetrix HG-U133A). CEL files from that study (n=163) were re-normalized to produce gene-level expression values using Robust Multiarray Average (RMA) [38] in the Bioconductor R package (version 1.28.1). This processing used the Entrez Gene-specific probeset chip definition file (CDF) [39] in place of the standard U133A CDF provided by Affymetrix in order to facilitate cross-platform analyses. Analyses were performed using the R environment for statistical computing (version 2.9.2).

The classifier was applied to patients in the test set with two modifications to account for the difference in microarray platforms. First, the HG-U133A RMA expression values were adjusted by a gene-wise constant which shifted the mean of each gene's expression levels in the test set to the mean observed in the training set. Second, for the classifier genes where a corresponding HG-U133A probeset was not available (LYPD2 and RNF150), the gene's mean expression value in the training set was used for all of the test set samples.

**Statistical Methods**

Classifier accuracy was assessed using standard measures of prediction accuracy: the area under the curve (AUC), sensitivity, specificity, NPV and PPV. Cross-validation, using a 10% sample hold-out set, was used in the training set to estimate the performance of the prediction classifiers generated using these approaches [40]. These performance estimates were used to guide the development of the classifier discovery procedure. A final model was set prior to performing a one-time analysis of the test set. Fisher's exact test was used to calculate statistical significance of all categorical variables and a t-test was used for continuous variables.

**Results**

**Study Populations**

A set of 299 patients from AEGIS 1 consisting of 223 patients diagnosed with lung cancer and 76 patients diagnosed with benign disease (Table 1) were used to derive our gene expression classifier. Characteristics of the independent test set have been previously described **[Error! Bookmark not defined.],** and are summarized here. Although the study design was similar to the one described here, there were some differences in the study populations. The patients were older on average in the training set compared to the test set ($p<0.001$) (although there was no significant difference in age ($p=0.959$) for patients diagnosed with lung cancer). The training set also consisted of fewer current smokers ($p=0.050$); and a lower proportion of patients with < 3cm lesions ($p<0.001$). In addition, the prevalence of lung cancer was higher in the training set (75% versus 48%; $p<0.001$).

Table 21 - Clinical and demographic characteristics of the patients used to train the classifier.

| Category | Sub-category | Lung cancer | Benign disease | p |
|---|---|---|---|---|
| N | | 223 | 76 | |

(continued)

| Category | Sub-category | Lung cancer | Benign disease | p |
|---|---|---|---|---|
| Sex | Female | 97 | 26 | 0.178 |
| | Male | 126 | 50 | |
| Age (median years) | | 65 | 56 | <0.0001 |
| Race | Caucasian | 168 | 59 | 0.757 |
| | African-American | 47 | 13 | |
| | Other | 5 | 3 | |
| | Unknown | 3 | 1 | |
| Smoking Status | Current | 101 | 26 | 0.107 |
| | Former | 122 | 50 | |
| Smoking History (median PY) | | 43 | 30 | <0.0001 |
| Mass size | <2cm | 46 | 23 | |
| | >2 to <3 cm | 30 | 12 | |
| | ≥3 cm | 122 | 19 | |
| | ill-defined infiltrate | 10 | 13 | |
| | Unknown | 15 | 9 | |
| Mass Location | Central | 86 | 16 | |
| | Peripheral | 60 | 30 | |
| | Central & peripheral | 60 | 18 | |
| | Unknown | 17 | 12 | |
| **Histology** | **Sub-type** | | | |
| SCLC | | 40 | | |
| NSCLC | | 180 | | |
| | Adenocarcinoma | 83 | | |
| | Squamous | 73 | | |
| | Large cell | 6 | | |
| | Mixed/undefined | 18 | | |
| Unknown | | 3 | | |
| **Histology** | **Stage** | | | |
| SCLC | Limited | 16 | | |
| | Extensive | 18 | | |
| | Unknown | 6 | | |
| NSCLC | 1 | 28 | | |
| | 2 | 16 | | |
| | 3 | 42 | | |
| | 4 | 62 | | |
| | Unknown | 32 | | |
| Benign disease | Sub-category | | | |
| Alternative Diagnosis | | | 54 | |
| | Infection | | 23 | |
| | Sarcoid | | 14 | |
| | Inflammation | | 7 | |
| | Fibrosis | | 4 | |
| | Other | | 4 | |

(continued)

| Benign disease | Sub-category | |
|---|---|---|
| Alternative Diagnosis | | 54 |
| | Benign growths | 2 |
| Resolution/Stability | | 22 |

**Derivation of the Classifier and Evaluation of Performance**

Gene expression was associated with current smoking status for a large fraction of the genes on the array (6477 genes with p<0.001; top 10 genes reported in Table 22). Three of the top ranked genes (SLC7A11, TKT, and CLND10) were selected to serve as a logistic regression-based smoking status classifier based on cross-validation. This smoking status classifier had an AUC of 0.93 within the training set. An additional CFGC was derived for smoking history, independent of smoking status, and was based on cumulative smoke exposure, measured in pack-years. Smoking history (< 10 PY vs > 10 PY) was significantly associated with the expression of 531 genes (p<0.001; top 10 genes reported in Table 23). Two of the top genes were selected to serve as a logistic regression-based smoking history classifier (RUNX1T1, AKR1C2) which had an AUC of 0.78 within the training set. Sex was significantly associated with 339 genes (p<0.001; top 10 genes reported in Table 24). The top ranked gene (RPS4Y1) was a perfect classifier (AUC=1) of sex within the training set.

Table 22: Top differentially expressed genes associated with smoking status

| ID | Symbol | logFC | AveExpr | T | P.Value | GS term |
|---|---|---|---|---|---|---|
| 8102800 | SLC7A11 | -2.31513 | 7.80246 | -19.0302 | 1.44E-53 | YES |
| 8088106 | TKT | -0.70341 | 9.137779 | -18.3095 | 7.46E-51 | YES |
| 8084630 | NA | -1.39188 | 6.897858 | -18.2898 | 8.86E-51 | |
| 8136336 | AKR1B10 | -2.27454 | 6.839364 | -18.2346 | 1.43E-50 | |
| 7969640 | CLDN10 | -0.94118 | 9.579322 | -18.1835 | 2.23E-50 | YES |
| 8171435 | PIR | -0.80298 | 8.924225 | -18.0114 | 9.97E-50 | |
| 7937465 | TALDO1 | -0.62614 | 10.07378 | -17.8271 | 4.95E-49 | |
| 8051583 | CYP1B1 | -2.8955 | 8.179293 | -17.7765 | 7.69E-49 | |
| 8020653 | CABYR | -1.19744 | 8.009791 | -17.6885 | 1.65E-48 | |
| 7979658 | GPX2 | -1.10719 | 10.62466 | -17.524 | 6.93E-48 | |

Table 23: Top differentially expressed genes associated with smoking history

| ID | Symbol | logFC | AveExpr | T | P.Value | GPY term |
|---|---|---|---|---|---|---|
| 8151768 | RUNX1T1 | 0.435653 | 5.905711 | 8.547091 | 6.44E-16 | Yes |
| 8077989 | TPRXL | -0.36913 | 8.733733 | -6.22283 | 1.63E-09 | |
| 7994058 | SCNN1G | 0.685624 | 8.450023 | 5.90033 | 9.75E-09 | |
| 8069764 | NA | -0.32511 | 7.23309 | -5.82162 | 1.49E-08 | |
| 8145470 | DPYSL2 | 0.260084 | 7.62917 | 5.749689 | 2.19E-08 | |
| 7931832 | AKR1C2 | -0.81612 | 10.93402 | -5.72526 | 2.50E-08 | Yes |
| 8039674 | ZNF154 | 0.372911 | 7.366637 | 5.724589 | 2.51E-08 | |
| 8150978 | CA8 | 0.415392 | 6.182963 | 5.638727 | 3.95E-08 | |
| 8129497 | EPB41L2 | 0.4248 | 6.895569 | 5.589681 | 5.10E-08 | |
| 8039672 | NA | 0.437196 | 4.729487 | 5.515997 | 7.48E-08 | |

Table 24: Top differentially expressed genes associated with gender

| ID | Symbol | logFC | AveExpr | T | P.Value | GS term |
|---|---|---|---|---|---|---|
| 8176375 | RPS4Y1 | -3.16276 | 7.851579 | -84.6047 | 6.46E-212 | YES |
| 8176624 | DDX3Y | -4.05255 | 7.702569 | -84.0882 | 3.79E-211 | |

(continued)

| ID | Symbol | logFC | AveExpr | T | P.Value | GS term |
|---|---|---|---|---|---|---|
| 8177232 | KDM5D | -2.23316 | 7.443775 | -80.4804 | 1.17E-205 | |
| 8176578 | USP9Y | -3.35997 | 7.485794 | -79.3437 | 7.01E-204 | |
| 8177137 | UTY | -3.38728 | 7.655898 | -78.9665 | 2.76E-203 | |
| 8176698 | TXLNG2P | -2.82024 | 6.904035 | -70.7168 | 1.36E-189 | |
| 8176709 | CYorf15B | -2.63878 | 7.07696 | -68.9368 | 1.87E-186 | |
| 8176719 | EIF1AY | -3.02926 | 7.079489 | -66.6741 | 2.34E-182 | |
| 8176384 | ZFY | -1.6795 | 6.679083 | -59.2385 | 5.47E-168 | |
| 8176460 | PRKY | -1.33643 | 7.761388 | -52.0418 | 1.48E-152 | |

As described in the methods, we identified genes whose expression is significantly associated with the residuals from the CFGC model for lung cancer. A total of 232 cancer associated genes (Table 25) met the significance criteria (T score>2.7). A pairwise correlation of the 232 genes followed by hierarchical clustering was examined to identify genes with similar expression patterns and partitioned the genes into 11 clusters (Figure 7). Since genes were correlated within each cluster, we hypothesized that the mean of a small set of genes within each cluster could be used to represent the cluster in a sparse manner. We optimized the classifier, using cross validation to estimate the AUC. We selected genes to represent the gene clusters whose expression was most strongly associated with lung cancer and determined that inclusion of clusters 1, 2, 4, 7, 9 and 10 gave the best AUC. We also determined that beyond 2-4 genes per cluster the performance of the test did not improve. In cross-validation, AUC = 0.80 (95% CI 0.75 - 0.84) for all patients in the training set (n=299); for the subset of patients with non-diagnostic bronchoscopy (n=134) the performance was similar (AUC=0.81; 95% CI 0.74 - 0.87).

Table 25: Genes associated with cancer which are included in the classifier

| ID | Symbol | T | p.value | FC | Cluster | Final Model |
|---|---|---|---|---|---|---|
| 8094228 | BST1 | -4.29031 | 2.41E-05 | 0.89208 | 1 | Yes |
| 8037298 | CD177 | -3.85704 | 0.00014 | 0.715357 | 1 | Yes |
| 8029280 | CD177 | -3.68455 | 0.000272 | 0.840725 | 1 | Yes |
| 7918857 | TSPAN2 | -3.92967 | 0.000106 | 0.845904 | 2 | Yes |
| 7968062 | ATP12A | -3.49107 | 0.000553 | 0.794623 | 2 | Yes |
| 8124654 | GABBR1 | 2.881256 | 0.004247 | 1.071879 | 4 | Yes |
| 8147461 | SDC2 | 2.847433 | 0.004712 | 1.089453 | 4 | Yes |
| 7978391 | NOVA1 | 2.729315 | 0.006721 | 1.094912 | 4 | Yes |
| 7952205 | MCAM | 2.70666 | 0.007186 | 1.06072 | 4 | Yes |
| 8175531 | CDR1 | 4.307308 | 2.24E-05 | 1.468199 | 7 | Yes |
| 8103877 | CLDN22 | 3.502336 | 0.000531 | 1.329189 | 7 | Yes |
| 8051001 | CGREF1 | 3.275505 | 0.001178 | 1.056672 | 7 | Yes |
| 8149811 | NKX3-1 | 2.92659 | 0.003689 | 1.175825 | 7 | Yes |
| 8034974 | EPHX3 | -3.73504 | 0.000225 | 0.898923 | 9 | Yes |
| 8153342 | LYPD2 | -2.93177 | 0.00363 | 0.887468 | 9 | Yes |
| 8102938 | RNF150 | -4.32839 | 2.05E-05 | 0.880745 | 10 | Yes |
| 8028924 | MIA | -3.23844 | 0.001337 | 0.906368 | 10 | Yes |

The final lung cancer classifier was then determined using the finalized classifier discovery procedure on the entire training set.. The classifier consisted of a combination of the six cancer gene clusters (represented by 17 genes in total), patient age, and the gene expression correlates (GG, GS, GPY) (Table 26) as predictors. Dichotomous classification was performed using a score threshold of 0.65 (patients with scores >/= 0.65 were predicted as cancer-positive and <0.65, cancer-negative). The classifier had a sensitivity of 93% and specificity of 57% in the training set and there was no difference in the AUC of the classifier for the entire training set (0.78; 95% CI, 0.73-0.82), compared with the subset of patients whose bronchoscopy was non-diagnostic for lung cancer (AUC=0.78; 95% 0.71-0.85), (see, Fig. 9).

Table 26 - Description of the gene expression classifier [a]

| **Feature**[b], ($x_i$) | **Coefficient**, ($b_i$) | **Informative Genes within features** | | | |
|---|---|---|---|---|---|
| Age | 0.0623 | | | | |
| GG | 0.5450 | RPS4Y1 | | | |
| GS | 0.1661 | SLC7A11 | CLDN10 | TKT | |
| GPY | 3.0205 | RUNX1T1 | AKR1C2 | | |
| CA (1) | -0.4406 | BST1 | CD177.1 | CD177.2 | |
| CA (2) | -0.3402 | ATP12A | TSPAN2 | | |
| CA (4) | 0.1725 | GABBR1 | MCAM | NOVA1 | SDC2 |
| CA (7) | 0.5670 | CDR1 | CGREF1 | CLDN22 | NKX3-1 |
| CA (9) | -0.3160 | EPHX3 | LYPD2 | | |
| CA (10) | -0.3791 | MIA | RNF150 | | |
| Intercept ($b_0$) | 3.3173 | | | | |

a) Genomic gender was defined as GG = 1 (female) if RPS4Y1<7.5, 0 (male) otherwise. The predicted genomic smoking (GS) value was derived, where x= 40.8579-0.4462*SLC7A11-2.1298*CLND10-1.8256*TKT, and genomic smoking GS = $e^x/(1+ e^x)$. The predicted genomic pack years (GPY) value was derived, where x=-5.1429+2.1891*RUNX1T1 - 0.9506*AKR1C2, and genomic pack years GPY = exp(x)/(1+exp(x)). The generalized equation for the prediction classifier was: Score= $e^y/(1+ e^y)$, where, y= $b_0 + \sum(b_i{*}x_i)$, where bo is the intercept, $b_i$ is the coefficient, and $x_i$ is the feature (as shown).

b) Features include patient age (as reported), GG, GS, GPY as described in the methods, and CA (*i*), the lung cancer gene clusters (shown in Figure 7).

The gene expression classifier performed significantly better (AUC = 0.78; 95% CI, 0.73-0.82) than a model using clinical factors alone (AUC = 0.72; 95% CI, 0.67-0.77) in the training set (p < 0.001). Functional analysis of the 17 cancer genes is summarized separately (Table 27). Nine of the genes are down-regulated and 8 are up-regulated in association with cancer.

Table 27: Biological characterization of classifier genes.

| **Cluster** | **Direction in Cancer** | **Biomarker genes** | **Biological themes** |
|---|---|---|---|
| 1 | Down | BST1, CD177.1, CD177.2 | Innate immune response |
| 2 | Down | ATP12A, TSPAN2 | Mitotic cell cycle |
| 4 | Up | GABBR1, MCAM, NOVA1, SDC2 | Response to retinoic acid, cell cycle |
| 7 | Up | CGREF1, CDR1, CLDN22, NKX3-1 | Submucosal gland markers |
| 9 | Down | EPHX3, LYPD2 | Xenobiotic detoxification |
| 10 | Down | MIA, RNF150 | Cartilaginous markers |

**Validation in an Independent Test Set**

In the patients with non-diagnostic bronchoscopy (n=123) of the independent test set, the AUC of the classifier was 0.81 (95% CI, 0.73 - 0.88), (Figure 8) which was similar to the performance in patients with non-diagnostic bronchoscopy in the training set (AUC=0.78; 95% 0.71-0.85; p = 0.495). The sensitivity was 92% and with a specificity of 55%, the NPV was 94% (95% CI, 83-99%), (see Table 28). Interestingly we did not observe any effect of cancer histology or stage (Table 29, or lesion size (Table 30) on the classifier's sensitivity for cancer. Moreover, in the test set the classifier had an AUC of 0.79 in current smokers and 0.82 in former smokers, suggesting that smoking status does not have a dramatic effect on classifier performance (p = 0.710). When compared with bronchoscopy alone, the combination of the gene expression classifier with bronchoscopy improved the sensitivity from 51% to 95% (p <0.001).

Table 28 - Performance of bronchoscopy, classifier, and the combined procedures in the test set.

| Category [a] | Bronchoscopy | Classifier [b] | Classifier & bronchoscopy combined |
|---|---|---|---|
| N, total | 163 | 123 | 163 |
| N, Lung cancer | 78 | 38 | 78 |

(continued)

| Category [a] | Bronchoscopy | Classifier [b] | Classifier & bronchoscopy combined |
|---|---|---|---|
| N, Benign disease | 85 | 85 | 85 |
| Sensitivity (95% CI) | 51% (40-62%) | 92% (78-98%) | 96% (89-99%) |
| Specificity (95% CI) | 100% (95-100%) | 53% (42-63%) | 53% (42-63%) |
| NPV (95% CI) | 69% (60-77%) | 94% (83-98%) | 94% (83-98%) |
| PPV (95% CI) | 100% (90-100%) | 47% (36-58%) | 65% (56-73%) |

a) Patients diagnosed with cancer = CA+ and benign disease = CA-
b) The performance of the classifier was evaluated in patients in which bronchoscopy did not result in a finding of cancer (n=123).

Table 29 - Sensitivity of bronchoscopy, the classifier, and the combined procedures for patients with lung cancer in the test set.

| Histology | Sub-type | N | Bronchoscopy Sensitivity | Classifier Sensitivity | Combined Sensitivity |
|---|---|---|---|---|---|
| All Cancers | | 78 | 51%[a] | 92%[b] | 96%[c] |
| SCLC | | 14 | 64% | 100% | 100% |
| NSCLC | | 64 | 48% | 91% | 95% |
| | Adenocarcinoma | 18 | 33% | 83% | 89% |
| | Squamous | 27 | 56% | 92% | 96% |
| | Large Cell | 4 | 25% | 100% | 100% |
| | Undefined | 15 | 60% | 83% | 93% |
| Histology | Stage | | | | |
| SCLC | | | | | |
| | Limited | 9 | 78% | 100% | 100% |
| | Extensive | 5 | 40% | 100% | 100% |
| NSCLC | | | | | |
| | 1 | 14 | 36% | 100% | 100% |
| | 2 | 2 | 50% | 100% | 100% |
| | 3 | 25 | 52% | 92% | 96% |
| | 4 | 22 | 55% | 80% | 91% |
| | Unknown | 1 | 0% | 100% | 100% |

Of 163 patients who underwent a diagnostic bronchoscopy procedure for suspicion of lung cancer, 78 were diagnosed with cancer. A lung cancer diagnosis was made at bronchoscopy (a) in 40 patients (51%; 95%CI, 40-62%), and in the remaining lung cancer patients where no diagnosis was made at bronchoscopy, the classifier correctly predicted 34 (b) of them (89%; 95%CI, 75-96%). The classifier combined with bronchoscopy yielded a detection of 74 of 78 (95%; 95%CI, 87-98%) patients with lung cancer (c). The sensitivities of bronchoscopy, the classifier, and the combined procedures are also shown for lung cancers according to sub-type and stage.

Table 30 - Sensitivity of bronchoscopy, the classifier, and the combined procedures in the test set stratified by size of suspicious lesions.

| Mass size [a] | N | Bronchoscopy Sensitivity | Classifier Sensitivity | Combined Sensitivity |
|---|---|---|---|---|
| <3cm | 99 | 44% | 87% | 93% |
| >3cm | 48 | 58% | 94% | 98% |
| Ill-def Infiltrate | 16 | 38% | 100% | 100% |

Includes patients diagnosed with lung cancer and those with benign disease.

Table 26 recites multiple gene expression classifiers of interest. The following table, Table 31, provides a Gene ID, as available on NCBI, providing descriptive support for the gene expression classifiers. Gene classifier CD177 is depicted in Table 31 with two designations, CD177.1 and CD177.2. The .1 and .2 designations identify that two different probe sets are used in the arrays which detect differential expression of the genes represented by the gene classifiers. Figure 10A discloses 19 probes utilized in hybridizing to CD177, accounting for CD177.1. Figure 10B discloses 4 probes utilized in hybridizing to CD177, accounting for CD177.2.

Table 31 - NCBI Gene ID Numbers corresponding to gene expression classifiers.

| Gene Classifier | Gene ID Number | Gene Classifier | Gene ID Number |
|---|---|---|---|
| RPS4Y1 | 6192 | MCAM | 4162 |
| SLC7A11 | 23657 | NOVA1 | 4857 |
| CLDN10 | 9071 | SDC1 | 6382 |
| TKT | 7086 | CDR1 | 1038 |
| AKR1C2 | 1646 | CGREF1 | 10669 |
| BST1 | 683 | CLDN22 | 53842 |
| CD177.1 | 57126 (See Fig. 10A) | NKX3-1 | 4824 |
| CD177.2 | 57126 (See Fig. 10B) | EPHX3 | 79852 |
| ATP12A | 479 | LYPD2 | 137797 |
| TSPAN2 | 10100 | MIA | 8190 |
| GABBR1 | 2550 | RNF150 | 57484 |
| RUNX1T1 | 862 | | |

**Evaluation of Results**

Work has demonstrated that there are persistent gene-expression alterations in normal epithelial cells from the bronchial airway that are associated with exposure to cigarette smoke and the presence of lung cancer in current and former smokers [32,41,42,43]. These cancer-associated differences can be used to derive classifiers capable of accurately detecting lung cancer in these relatively non-invasively collected biospecimens obtained during bronchoscopy [35]. In current practice it is challenging to rule out lung cancer when bronchoscopy does not lead to a finding of malignancy, and the false-negative rate can range from 20-70% [28]. Current guidelines suggest that patients with elevated risk of disease should be pursued with more invasive follow-up diagnostic procedures [28], which carry increased risk of complications [31]. However due to uncertainty these procedures often performed in patients found to have benign disease [33]. Therefore our goal was to derive a gene-expression classifier from the proximal airway during bronchoscopy to increase the overall sensitivity, minimize ambiguity when bronchoscopy is non-diagnostic, and reduce the need for unnecessary invasive procedures.

In this study, we leveraged a cohort of current and former smokers undergoing bronchoscopy for suspected lung cancer from a larger multicenter study to derive a gene-expression classifier for lung cancer. The classifier is a multivariate logistic regression model that has high sensitivity and high NPV. Importantly, we have validated the performance of the classifier in an independent cohort, using data from a previously published study of airway samples collected from smokers undergoing bronchoscopy for suspected lung cancer. The sensitivity is 92% in patients whose bronchoscopy is non-diagnostic in the test set with a specificity of 55%. The NPV is 94% in the test set compared to an NPV of 69% for bronchoscopy alone suggesting that the classifier could help physicians reliably identify patients unlikely to have lung cancer after a non-diagnostic bronchoscopy.

The functions of the differentially expressed genes in the normal appearing airway epithelium in current and former smokers with lung cancer provide insight into the biology underlying the field of injury. Among genes that are suppressed, there are a number involved in the immune response, including CD177 and BST1, suggesting an impaired immune response in the airway of smokers with lung cancer. The gene TSPAN2, whose expression is depressed by p53 knockdown and is associated with poor prognosis in lung adenocarcinomas [44] was also expressed at lower levels in patients with cancer. Also EPHX3, a gene involved in xenobiotic metabolism, processing of carcinogens in tobacco smoke, and carcinogenesis in other epithelial cancers is down-regulated [45]. Among the classifier genes that are up-regulated in lung cancer, NOVA1 and CDR1 are predominantly expressed in neurons, but are also expressed in tumors and are associated with para-neoplastic antibodies in several malignancies, including small-cell lung cancer [46,47,48,49,50]. Furthermore, MCAM which is up-regulated in lung cancer, is expressed in basal bronchial epithelial cells [51]. MCAM is also strongly and transiently up-regulated in tracheal epithelium during repair [52], is required for tracheal epithelial regeneration [53], and is up-regulated in the bronchial epithelium of patients with COPD [54] and asthma [55]. A number of classifier genes that regulate cell growth and proliferation are up-regulated in patients with lung cancer, including SDC2, and NKX3-1 as well as the cell-cycle-arrest mediator CGREF1. Finally the CFGC genes selected to predict smoking status (SLC7A11, CLDN10, TKT) and smoking history (RUNX1T1, AKR1C2) in our classifier have been previously reported as being altered by tobacco smoke exposure, confirming the robust effect of smoking on airway epithelium biology [11,18,33].

Our discovery approach extends earlier work on gene-expression based lung cancer diagnostics [35] primarily in the explicit modeling of clinical covariates as components of the predictive model prior to selection of features with lung cancer-associated expression. It is known that the response to environmental insults and other clinical factors can vary substantially between individuals. Therefore our approach was to use gene expression to capture the patient-level physiological response to an environmental insult (e.g., cumulative smoke exposure), as this response may be more reflective of disease risk than the actual reported values [57]. Another component of our approach was selecting genes whose expression is associated with cancer after accounting for the modeled clinical factors. We hypothesized that this approach would help ensure that the information about the likelihood of cancer captured by the genes with cancer-associated gene expression is independent from the information about cancer captured by the modeled clinical factors. An additional important aspect of our classifier discovery approach was our methodology to identify patterns of independent cancer-associated gene expression through clustering and then to model cancer as the additive effects of each of the cancer-associated gene expression modules. This is in contrast to selecting only genes that are globally top-ranked according to their association with cancer which could potentially result in selecting an entire panel of genes that reflect a single cancer-associated molecular process. Previous studies to derive a gene expression classifier to predict risk of lung cancer in normal appearing airway epithelial cells have described similar results with high sensitivity and NPV when bronchoscopy is non-diagnostic [35]. While there are no common genes in that classifier compared to the one described here, we believe that our new classifier represents similar mechanisms of action given the strong performance in the independent test set. However, the differences in the specific genes selected this may be due to differences in the feature selection process.

We have derived a gene expression classifier for lung cancer using cells from the proximal airway that can be used in conjunction with bronchoscopy for suspected lung cancer. We have validated the performance of this classifier in an independent test set. The classifier adds substantial sensitivity to the bronchoscopy procedure resulting in high NPV. This classifier can be used to aid in decision-making when bronchoscopy is non-diagnostic by identifying patients who are at low risk of having lung cancer.

Having thus described several aspects of at least one embodiment of this disclosure, it is to be appreciated that various alterations, modifications, and improvements will readily occur to those skilled in the art. Such alterations, modifications, and improvements are intended to be part of this disclosure, and are intended to be within the spirit and scope of the disclosure. Accordingly, the foregoing description and drawings are by way of example only and the disclosure is described in detail by the claims that follow.

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

## INCORPORATION BY REFERENCE

All references, articles, publications, patents, patent publications, and patent applications cited herein are incorporated by reference in their entireties for all purposes.

## References

**1.** Gould, M. K., Donington, J., Lynch, W. R., et al. Evaluation of individuals with pulmonary nodules: When is it lung cancer?: Diagnosis and management of lung cancer: American College of Chest Physicians evidence-based clinical practice guidelines. CHEST Journal 2013; 143(5_suppl): e93S-e120S.

**2.** Tukey, M. H., & Wiener, R. S.. Population-based estimates of transbronchial lung biopsy utilization and complications. Respiratory medicine 2012; 106(11): 1559-1565.

**3.** Ernst A, Silvestri G, Johnstone D. Interventional Pulmonary Procedures: Guidelines from the American College of Chest Physicians. Chest 2003;123;1693-1717

**4.** Rivera MP, Mehta AC, Wahidi MM. Establishing the Diagnosis of Lung Cancer: Diagnosis and Management of Lung Cancer, 3rd ed: American College of Chest Physicians Evidence-Based Clinical Practice Guidelines. Chest 2013;143:e142S-65S.

**5.** Memoli, J. S. W., Nietert, P. J., & Silvestri, G. A. Meta-analysis of guided bronchoscopy for the evaluation of the pulmonary nodule. CHEST Journal 2012; 142(2): 385-393.

**6.** Ost, D., Fein, A. M., & Feinsilver, S. H. (2003). The solitary pulmonary nodule. New England Journal of Medicine, 348(25), 2535-2542.

**7.** Grogan, E. L., Weinstein, J. J., Deppen, S. A., et al. Thoracic operations for pulmonary nodules are frequently not futile in patients with benign disease. Journal of thoracic oncology: official publication of the International Association for the Study of Lung Cancer 2011, 6(10): 1720.

**8.** Detterbeck, F. C., Mazzone, P. J., Naidich, D. P., & Bach, P. B. Screening for lung cancer: diagnosis and management of lung cancer: American College of Chest Physicians evidence-based clinical practice guidelines. CHEST Journal 2013; 143(5_suppl): e78S-e92S.

**9.** Wiener, R. S., Wiener, D. C., & Gould, M. K. Risks of Transthoracic Needle Biopsy: How High? Clinical pulmonary medicine 2013; 20(1): 29.

**10.** Covey, A. M., Gandhi, R., Brody, L. A., Getrajdman, G., Thaler, H. T., & Brown, K. T. Factors associated with pneumothorax and pneumothorax requiring treatment after percutaneous lung biopsy in 443 consecutive patients. Journal of vascular and interventional radiology 2004; 15(5): 479-483.

**11.** Geraghty, P. R., Kee, S. T., McFarlane, G., Razavi, M. K., Sze, D. Y., & Dake, M. D. CT-guided Transthoracic Needle Aspiration Biopsy of Pulmonary Nodules: Needle Size and Pneumothorax Rate 1. Radiology 2003; 229(2): 475-481.

**12.** Golub, T. R., Slonim, D. K., Tamayo, P., et al. Molecular classification of cancer: class discovery and class prediction by gene expression monitoring. Science 1999; 286(5439): 531-537.

**13.** Spira A, Beane JE, Shah V, et al. Airway epithelial gene expression in the diagnostic evaluation of smokers with suspect lung cancer. Nature medicine 2007;13:361-6.

**14.** Hanley JA, McNeil BJ. The meaning and use of the area under a receiver operating characteristic (ROC) curve. Radiology 1982;143:29-36.

**15.** MacMahon, H., Austin, J. H., Gamsu, G., et al. Guidelines for management of small pulmonary nodules detected on CT scans: a statement from the Fleischner Society 1. Radiology 2005, 237(2): 395-400..

**16.** Beane, J., Sebastiani, P., Whitfield, T. H., et al. A prediction model for lung cancer diagnosis that integrates genomic and clinical features. Cancer Prevention Research 2008; 1(1): 56-64.

**17.** Gustafson, A. M., Soldi, R., Anderlind, C., et al. Airway PI3K pathway activation is an early and reversible event in lung cancer development. Science translational medicine 2010; 2(26): 26ra25-26ra25.

**18.** Kadara, H., Fujimoto, J., Yoo, S. Y., Maki, et al. Transcriptomic architecture of the adjacent airway field cancerization in non-small cell lung cancer. Journal of the National Cancer Institute 2014; 106(3): dju004.

**19.** Alexander, E. K., Kennedy, G. C., Baloch, et al. Preoperative diagnosis of benign thyroid nodules with indeterminate cytology. New England Journal of Medicine 2012, 367(8):705-715.

**20.** McWilliams A, Tammemagi MC, Mayo JR, et al. Probability of cancer in pulmonary nodules detected on first screening CT. N Engl J Med 2013;369:910-9.

**21.** Ost DE, Gould MK. Decision making in patients with pulmonary nodules. American journal of respiratory and critical care medicine 2012;185:363-72.

**22.** Irizarry, R. A., Bolstad, B. M., Collin, F., Cope, L. M., Hobbs, B., & Speed, T. P. (2003). Summaries of Affymetrix GeneChip probe level data. Nucleic acids research, 31(4), e15-e15.

**23.** Johnson WE, Li C, Rabinovic A. Adjusting batch effects in microarray expression data using empirical Bayes methods. Biostatistics 2007; 8: 118-127.

**24.** Howlader N, Noone AM, Krapcho M, et al. SEER stat fact sheets: lung and bronchus. Available online: http://seer.cancer.gov/statfacts/html/lungb.html.

**25.** The National Lung Screening Trial Research Team. Reduced Lung-Cancer Mortality with Low-Dose Computed Tomographic Screening. N Engl J Med 2011; 365:395-409

**26.** Tanoue LT, Tanner NT, Gould MK, Silvestri GA. Lung Cancer Screening. Am J Respir Crit Care Med. published online 04 Nov 2014

**27.** Ernst A, Silvestri G, Johnstone D. Interventional Pulmonary Procedures: Guidelines from the American College of Chest Physicians. Chest 2003;123;1693-1717

**28.** Rivera, M. P., Mehta, A. C., & Wahidi, M. M. (2013). Establishing the Diagnosis of Lung Cancer Diagnosis and Management of Lung Cancer, 3rd ed: American College of Chest Physicians Evidence-Based Clinical Practice Guidelines. CHEST Supplement. Chest, 143, e143S.

**29.** Silvestri, G. A., Feller-Kopman, D., Chen, A., Wahidi, M., Yasufuku, K., & Ernst, A. (2012). Latest Advances in Advanced Diagnostic and Therapeutic Pulmonary Procedures Update on Pulmonary Procedures. CHEST Journal, 142(6), 1636-1644.

**30.** Gildea, T. R., Mazzone, P. J., Karnak, D., Meziane, M., & Mehta, A. C. (2006). Electromagnetic navigation diagnostic bronchoscopy: a prospective study. American journal of respiratory and critical care medicine, 174(9), 982-989.

**31.** Wiener, R. S., Schwartz, L. M., Woloshin, S., & Welch, H. G. (2011). Population-based risk for complications after transthoracic needle lung biopsy of a pulmonary nodule: an analysis of discharge records. Annals of internal medicine, 155(3), 137-144.

**32.** Fontaine-Delaruelle, C., Ferretti, G., Gamondes, D., Pradat, E., Souquet, P. J., & Couraud, S. (2014). Is transthoracic core needle biopsy under CT scan a good deal for benign diseases' diagnosis?. European Respiratory Journal, 44(Suppl 58), P679.

**33.** Smith MA, Battafarano RJ, Meyers BF, Zoole JB, Cooper JD, Patterson GA. Prevalence of benign disease in patients undergoing resection for suspected lung cancer. The Annals of thoracic surgery 2006;81:1824-1828; discussion 1828-1829

**34.** Beane, J., Sebastiani, P., Liu, G., Brody, J. S., Lenburg, M. E., & Spira, A. (2007). Reversible and permanent effects of tobacco smoke exposure on airway epithelial gene expression. Genome Biol, 8(9), R201.

**35.** Spira A, Beane JE, Shah V, Steiling K, Liu G, Schembri F, Gilman S, Dumas YM, Calner P, Sebastiani P, Sridhar S, Beamis J, Lamb C, Anderson T, Gerry N, Keane J, Lenburg ME, Brody J. Airway epithelial gene expression in the diagnostic evaluation of smokers with suspect lung cancer. Nature Medicine 2007; 13(3):361-6

**36.** Tibshirani, R. (1996). Regression shrinkage and selection via the lasso. Journal of the Royal Statistical Society. Series B (Methodological), 267-288.

**37.** Da Wei Huang, B. T. S., & Lempicki, R. A. (2008). Systematic and integrative analysis of large gene lists using DAVID bioinformatics resources. Nature protocols, 4(1), 44-57.

**38.** Irizarry, R. A., Hobbs, B., Collin, F., Beazer-Barclay, Y. D., Antonellis, K. J., Scherf, U., & Speed, T. P. (2003). Exploration, normalization, and summaries of high density oligonucleotide array probe level data. Biostatistics, 4(2), 249-264.

**39.** Dai, M., Wang, P., Boyd, A. D., Kostov, G., Athey, B., Jones, E. G., et al., (2005). Evolving gene/transcript definitions significantly alter the interpretation of GeneChip data. Nucleic acids research, 33(20), e175-e175.

**40.** Golub, T. R., Slonim, D. K., Tamayo, P., Huard, C., Gaasenbeek, M., Mesirov, J. P., et al., (1999). Molecular classification of cancer: class discovery and class prediction by gene expression monitoring. Science, 286(5439), 531-537.

**41.** Kadara, H., Fujimoto, J., Yoo, S. Y., Maki, Y., Gower, A. C., Kabbout, M., et al., (2014). Transcriptomic Architecture of the Adjacent Airway Field Cancerization in Non-Small cell lung cancer. Journal of the National Cancer Institute, 106(3)

**42.** Steiling, K., Ryan, J., Brody, J. S., & Spira, A. (2008). The field of tissue injury in the lung and airway. Cancer prevention research, 1(6), 396-403.

**43.** Bossé, Y., Postma, D. S., Sin, D. D., Lamontagne, M., Couture, C., Gaudreault, N., et al., (2012). Molecular signature of smoking in human lung tissues. Cancer research, 72(15), 3753-3763.

**44.** Otsubo, C., Otomo, R., Miyazaki, M., Matsushima-Hibiya, Y., Kohno, T., Iwakawa, R., et al., (2014). TSPAN2 Is Involved in Cell Invasion and Motility during Lung Cancer Progression. Cell reports, 7(2), 527-538.

**45.** Øster, B., Thorsen, K., Lamy, P., Wojdacz, T. K., Hansen, L. L., Birkenkamp-Demtröder, K., et al., (2011). Identification and validation of highly frequent CpG island hypermethylation in colorectal adenomas and carcinomas. International journal of cancer, 129(12), 2855-2866.

**46.** Knudsen, A., Monstad, S. E., Dørum, A., Lønning, P. E., Salvesen, H. B., Drivsholm, L., et al., (2006). Ri antibodies in patients with breast, ovarian or small cell lung cancer determined by a sensitive immunoprecipitation technique. Cancer Immunology, Immunotherapy, 55(10), 1280-1284.

**47.** Buckanovich, R. J., Posner, J. B., & Darnell, R. B. (1993). Nova, the paraneoplastic Ri antigen, is homologous to an RNA-binding protein and is specifically expressed in the developing motor system. Neuron, 11(4), 657-672.

**48.** Salemi, M., Fraggetta, F., Galia, A., Pepe, P., Cimino, L., Condorelli, R. A., & Calogero, A. E. (2013). Cerebellar degeneration-related autoantigen 1 (CDR1) gene expression in prostate cancer cell lines. The International journal of biological markers, 30;29(3):e288-90

**49.** Tanaka, M., Tanaka, K., Onodera, O., & Tsuji, S. (1995). Trial to establish an animal model of paraneoplastic cerebellar degeneration with anti-Yo antibody: 1. Mouse strains bearing different MHC molecules produce antibodies on immunization with recombinant Yo protein, but do not cause Purkinje cell loss. Clinical neurology and neurosurgery, 97(1), 95-100.

**50.** Furneaux, H. M., Rosenblum, M. K., Dalmau, J., Wong, E., Woodruff, P., Graus, F., & Posner, J. B. (1990). Selective expression of Purkinje-cell antigens in tumor tissue from patients with paraneoplastic cerebellar degeneration. New England Journal of Medicine, 322(26), 1844-1851.

**51.** Shih, I. M., Nesbit, M., Herlyn, M., & Kurman, R. J. (1998). A new Mel-CAM (CD146)-specific monoclonal antibody, MN-4, on paraffin-embedded tissue. Modern pathology: an official journal of the United States and Canadian Academy of Pathology, Inc, 11(11), 1098-1106.
**52.** Tsukamoto, Y., Taira, E., Miki, N., & Sasaki, F. (2001). The role of gicerin, a novel cell adhesion molecule, in development, regeneration and neoplasia. Histol Histopathol. 2001; 16(2):563-71.
**53.** Tsukamoto, Y., Taira, E., Kotani, T., Yamate, J., Wada, S., Takaha, N., et al., (1996). Involvement of gicerin, a cell adhesion molecule, in tracheal development and regeneration. Cell Growth Differ. 7(12), 1761-1767.
**54.** Schulz, C., Petrig, V., Wolf, K., Krätzel, K., Kohler, M., Becker, B., & Pfeifer, M. (2003). Upregulation of MCAM in primary bronchial epithelial cells from patients with COPD. Eur Respir J. 2003; 22(3):450-6.
**55.** Simon, G. C., Martin, R. J., Smith, S., Thaikoottathil, J., Bowler, R. P., Barenkamp, S. J., & Chu, H. W. (2011). Up-regulation of MUC18 in airway epithelial cells by IL-13: implications in bacterial adherence. Am J Resp Cell Mol Biol, 44(5), 606-613.
**56.** Penning, T. M., & Lerman, C. (2008). Genomics of smoking exposure and cessation: lessons for cancer prevention and treatment. Cancer Prevention Research, 1(2), 80-83.
**57.** Lampe, J. W., Stepaniants, S. B., Mao, M., Radich, J. P., Dai, H., Linsley, P. S., et al., (2004). Signatures of environmental exposures using peripheral leukocyte gene expression: tobacco smoke. Cancer Epidemiology Biomarkers & Prevention, 13(3), 445-453.CLAIMS

The invention includes the following aspects.

1. A method of determining the likelihood that a subject has lung cancer, the method comprising subjecting a biological sample obtained from a subject to a gene expression analysis, wherein the gene expression analysis comprises

(a) determining mR A expression levels in the biological sample of one or more informative-genes that relate to lung cancer status, and

(b) determining mRNA expression levels in the biological sample of one or more genomic correlate genes that relate to one or more self-reportable characteristics of the subject; and

determining a lung cancer risk-score, based on the expression levels determined in (a) and (b), that is indicative of the likelihood that the subject has lung cancer.

2. The method of aspect 1, wherein the one or more self-reportable characteristics of the subject are selected from: smoking pack years, smoking status, age and gender.

3. The method of aspect 1 or 2, wherein the informative-genes are selected from Table 11.

4. The method of any one of aspects 1 to 3, wherein the informative-genes comprise at least two genes selected from the set of genes identified as cluster 1 in Table 11.

5. The method of any one of aspects 1 to 4, wherein the informative-genes comprise at least two genes selected from the set of genes identified as cluster 2 in Table 11.

6. The method of any one of aspects 1 to 5, wherein the informative-genes comprise at least two genes selected from the set of genes identified as cluster 3 in Table 11.

7. The method of any one of aspects 1 to 6, wherein the informative-genes comprise at least two genes selected from the set of genes identified as cluster 4 in Table 11.

8. The method of any one of aspects 1 to 7, wherein the informative-genes comprise at least two genes selected from the set of genes identified as cluster 5 in Table 11.

9. The method of any one of aspects 1 to 8, wherein the informative-genes comprise at least two genes selected from the set of genes identified as cluster 6 in Table 11.

10. The method of any one of aspects 1 to 9, wherein the informative-genes comprise at least two genes selected from the set of genes identified as cluster 7 in Table 11.

11. The method of any one of aspects 1 to 10, wherein the informative-genes comprise at least two genes selected from the set of genes identified as cluster 8 in Table 11.

12. The method of any one of aspects 1 to 11, wherein the informative-genes comprise at least two genes selected from the set of genes identified as cluster 9 in Table 11.

13. The method of any one of aspects 1 to 12, wherein the informative-genes comprise at least two genes selected from the set of genes identified as cluster 10 in Table 11.

14. The method of any one of aspects 1 to 13, wherein the informative-genes comprise MYOT.

15. The method of any one of aspects 1 to 14, wherein the lung cancer risk-score is determined based on the sum of weighted expression levels.

16. The method of any one of aspects 1 to 15, wherein the lung cancer risk-score is determined according to the follow model:

$$x^{score\ 1} = Wo + Wi \times GG + W_2 \times GS + W_3 \times GPY + W_4 \times Reported\ Age + W_5 \times CIA + W_6 \times CIB + W_7 \times C2 + W_s \times C3 + W_9 \times C4A + W_w \times CAB,$$

and

$$x^{score\ 2} = Wo + Wi \times GG + W_2 \times GS + W_3 \times GPY + W_4 \times GA + W_5 \times CIA + W_6 \times CIB + W_7 \times C2 + W_s \times C3 + W_9 \times C4A + W_w \times CAB$$

and wherein GG, GS, GA, GPY, CIA, CIB, C2, C3, C4A and C4B are determined according to the equations disclosed herein.

17. The method of any one of aspects 1 to 16, wherein the lung cancer-risk score is determined according to a model having a Negative Predictive Value (NPV) of greater than 90% for ruling out lung cancer in an intended use population.

18. The method of any one of aspects 1 to 17, wherein the lung cancer-risk score is determined according to a model having a Negative Predictive Value (NPV) of greater than 85% for subjects diagnosed with COPD.

19. A method of determining the likelihood that a subject has lung cancer, the method comprising:

subjecting a biological sample obtained from a subject to a gene expression analysis, wherein the gene expression analysis comprises determining mRNA expression levels in the biological sample of at least 2 to at least 10 genes selected from Table 11; and

determining the likelihood that the subject has lung cancer by determining a statistical significance on the mRNA expression levels.

20. The method of aspect 19, wherein the step of determining the statistical significance comprises transforming the expression levels into a lung cancer risk-score that is indicative of the likelihood that the subject has lung cancer.

21. The method of aspect 20, wherein the lung cancer risk-score is the combination of weighted expression levels.

22 The method of aspect 21, wherein the lung cancer risk-score is the sum of weighted expression levels.

23. The method of aspect 21 or 22, wherein the expression levels are weighted by their relative contribution to predicting increased likelihood of having lung cancer.

24. A method for determining a treatment course for a subject, the method comprising:

subjecting a biological sample obtained from the subject to a gene expression analysis, wherein the gene expression analysis comprises determining mRNA expression levels in the biological sample of at least 2 to at least 10 genes selected from Table 11;

determining a treatment course for the subject based on the expression levels.

25. The method of aspect 24, wherein the treatment course is determined based on a lung cancer risk-score derived from the expression levels.

26. The method of aspect 25, wherein the subject is identified as a candidate for a lung cancer therapy based on a lung cancer risk-score that indicates the subject has a relatively high likelihood of having lung cancer.

27. The method of aspect 25, wherein the subject is identified as a candidate for an invasive lung procedure based on a lung cancer risk-score that indicates the subject has a relatively high likelihood of having lung cancer.

28. The method of aspect 27, wherein the invasive lung procedure is a transthoracic needle aspiration, mediastinoscopy or thoracotomy.

29. The method of aspect 25, wherein the subject is identified as not being a candidate for a lung cancer therapy or an invasive lung procedure based on a lung cancer risk-score that indicates the subject has a relatively low likelihood of having lung cancer.

30. The method of any one of aspects 19 to 29 further comprising creating a report summarizing the results of the gene expression analysis.

31 The method of any one of aspects 20, 21, 25-27, and 29 further comprising creating a report that indicates the lung cancer risk-score.

32. The method of any one of aspects 19 to 31, wherein the biological sample is obtained from the respiratory epithelium of the subject.

33. The method of aspect 32, wherein the respiratory epithelium is of the mouth, nose, pharynx, trachea, bronchi, bronchioles, or alveoli.

34. The method of one of aspects 19 to 33, wherein the biological sample is obtained using bronchial brushings, broncho-alveolar lavage, or a bronchial biopsy.

35. The method of one of aspects 19 to 34, wherein the subject exhibits one or more symptoms of lung cancer and/or has a lesion that is observable by computer-aided tomography or chest X-ray.

36. The method of aspect 35, wherein, prior to subjecting the biological sample to the gene expression analysis, the subject has not be diagnosed with primary lung cancer.

37. The method of one of aspects 19 to 36, wherein the genes comprise at least two genes selected from the set of genes identified as cluster 1 in Table 11.

38. The method of one of aspects 19 to 37, wherein the genes comprise at least two genes selected from the set of genes identified as cluster 2 in Table 11.

39. The method of one of aspects 19 to38, wherein the genes comprise at least two genes selected from the set identified as cluster 3 in Table 11.

40. The method of one of aspects 19 to 39, wherein the genes comprise at least two genes selected from the set identified as cluster 4 in Table 11.

41. The method of one of aspects 19 to 40, wherein the genes comprise at least two genes selected from the set identified as cluster 5 in Table 11.

42. The method of one of aspects 19 to 41, wherein the genes comprise at least two genes selected from the set identified as cluster 6 in Table 11.

43. The method of one of aspects 19 to 42, wherein the genes comprise at least two genes selected from the set identified as cluster 7 in Table 11.

44. The method of one of aspects 19 to 43, wherein the genes comprise at least two genes selected from the set identified as cluster 8 in Table 11.

45. The method of one of aspects 19 to 44, wherein the genes comprise at least two genes selected from the set identified as cluster 9 in Table 11.

46. The method of one of aspects 19 to 45, wherein the genes comprise at least two genes selected from the set identified as cluster 10 in Table 11.

47. The method of one of aspects 19 to 46, wherein the genes comprise MYOT.

48. The method of any one of aspects 19 to 47, wherein the gene expression analysis comprises determining the expression levels of at least 10 genes selected from Table 11.

49. The method of any one of aspects 19 to 48, wherein the gene expression analysis comprises determining the expression levels of at least 15 genes selected from Table 11.

50. The method of one of aspects 19 to 49, wherein the expression levels are determined using a quantitative reverse transcription polymerase chain reaction, a bead-based nucleic acid detection assay or a oligonucleotide array assay.

51. A method of determining the likelihood that a subject has lung cancer, the method comprising: subjecting a biological sample obtained from a subject to a gene expression analysis, wherein the gene expression analysis comprises determining an mRNA expression level in the biological sample of at least 2 to at least 10 genes selected from Table 11; and determining the likelihood that the subject has lung cancer based at least in part on the expression levels.

52. A method of determining the likelihood that a subject has lung cancer, the method comprising:

subjecting a biological sample obtained from the respiratory epithelium of a subject to a gene expression analysis, wherein the gene expression analysis comprises determining an expression level in the biological sample of at least 2 to at least 10 genes selected from Table 11; and

determining the likelihood that the subject has lung cancer based at least in part on the expression level.

53. The method of any one of aspects 19 to52, wherein the lung cancer is a adenocarcinoma, squamous cell carcinoma, small cell cancer or non-small cell cancer.

54. The method of any one of aspects 19 to 53, wherein the expression level of each of the 15 genes in Table 11 is determined.

55. The method of any one of aspects 91 to 54, wherein the expression levels of at least 2 genes are evaluated.

56. The method of any one of aspects 19 to 55, wherein the expression levels of at least 3 genes are evaluated.

57. The method of any one of aspects 19 to 56, wherein the expression levels of at least 4 genes are evaluated.

58. The method of any one of aspects 19 to 57, wherein the expression levels of at least 5 genes are evaluated.

59. A computer implemented method for processing genomic information, the method comprising:

obtaining data representing expression levels in a biological sample of at least 2 to at least 10 genes selected from Table 11, wherein the biological sample was obtained of a subject; and using the expression levels to assist in determining the likelihood that the subject has lung cancer.

60. The computer implemented method of aspect 59, wherein the step of determining comprises calculating a risk-score indicative of the likelihood that the subject has lung cancer.

61. The computer implemented method of aspect 60, wherein computing the risk-score involves determining the combination of weighted expression levels, wherein the expression levels are weighted by their relative contribution to predicting increased likelihood of having lung cancer.

62. The computer implemented method of aspect 59 furthering comprising generating a report that indicates the risk-score.

63. The computer implemented method of aspect 62 further comprising transmitting the report to a health care provider of the subject.

64. The computer implemented method of any one aspects 59 to 63, wherein the genes comprise at least two genes selected from the set of genes identified as cluster 1 in Table 11.

65. The computer implemented method of any one of aspects 59 to 64, wherein the genes comprise at least two genes selected from the set of genes identified as cluster 2 in Table 11.

66. The computer implemented method of any one of aspects 59 to 65, wherein the genes comprise at least two genes selected from the set of genes identified as cluster 3 in Table 11.

67. The computer implemented method of any one of aspects 59 to 66, wherein the genes comprise at least two genes selected from the set identified as cluster 4 in Table 11.

68. The computer implemented method of any one of aspects 59 to 67 wherein the genes comprise at least two genes selected from the set identified as cluster 5 in Table 11.

69. The computer implemented method of any one of aspects 59 to 68, wherein the genes comprise at least two genes selected from the set identified as cluster 6 in Table 11.

70. The computer implemented method of any one of aspects 59 to 69, wherein the genes comprise at least two genes selected from the set identified as cluster 7 in Table 11.

71. The computer implemented method of any one of aspects 59 to 70, wherein the genes comprise at least two genes selected from the set identified as cluster 8 in Table 11.

72. The computer implemented method of any one of aspects 59 to 71, wherein the genes comprise at least two genes selected from the set identified as cluster 9 in Table 11.

73. The computer implemented method of any one of aspects 59 to 72, wherein the genes comprise at least two genes selected from the set identified as cluster 10 in Table 11.

74. The computer implemented method of any one of aspects 59 to 73, wherein the genes comprise MYOT.

75. The computer implemented method of any one of aspects 59 to 74, wherein the gene expression analysis comprises determining mRNA expression levels in an RNA sample of at least 10 genes selected from Table 11.

76. The computer implemented method of any one of aspects 59 to 75, wherein the gene expression analysis comprises determining mRNA expression levels in an RNA sample of at least 15 genes selected from Table 11.

77. The computer implemented method of any one of aspects 59 to 76, wherein the biological sample was obtained from the respiratory epithelium of the subject.

78. A composition consisting essentially of at least 1-10 nucleic acid probes, wherein each of the at least 2 to at least 10 nucleic acids probes specifically hybridizes with an mRNA expressed from a different gene selected from the genes of Table 11.

79. A composition comprising up to 5, up to 10, up to 25, up to 50, up to 100, or up to 200 nucleic acid probes, wherein each of at least 2 to at least 10 of the nucleic acid probes specifically hybridizes with an mRNA expressed from a different gene selected from the genes of Table 11.

80. The composition of aspect 78 or 79, wherein the genes comprise at least two genes selected from the set of genes identified as cluster 1 in Table 11.

81. The composition of any one of aspects 78 to 80, wherein the genes comprise at least two genes selected from the set of genes identified as cluster 2 in Table 11.

82. The composition of any one of aspects 78 to 81, wherein the genes comprise at least two genes selected from the set of genes identified as cluster 3 in Table 11.

83. The composition of any one of aspects 78 to 82, wherein the genes comprise at least two genes selected from the set identified as cluster 4 in Table 11.

84. The composition of any one of aspects 78 to 83, wherein the genes comprise at least two genes selected from the set identified as cluster 5 in Table 11.

85. The composition of any one of aspects 78 to 84, wherein the genes comprise at least two genes selected from the set identified as cluster 6 in Table 11.

85. The composition of any one of aspects 78 to 85, wherein the genes comprise at least two genes selected from the set identified as cluster 7 in Table 11.

87. The composition of any one of aspects 78 to 86, wherein the genes comprise at least two genes selected from the set identified as cluster 8 in Table 11.

88. The composition of any one of aspects 78 to 87, wherein the genes comprise at least two genes selected from the set identified as cluster 9 in Table 11.

89. The composition of any one of aspects 78 to 88, wherein the genes comprise at least two genes selected from the set identified as cluster 10 in Table 11.

90. The composition of any one of aspects 78 to 89, wherein the genes comprise MYOT.

91. The composition of any one of aspects 78 to 90, wherein each of at least 10 of the nucleic acid probes specifically hybridizes with an m NA expressed from a gene selected from Table 1 or 11 or with a nucleic acid having a sequence complementary to the mRNA.

92. The composition of any one of aspects 78 to 91, wherein each of at least 15 of the nucleic acid probes specifically hybridizes with an mRNA expressed from a gene selected from Table 1 or 11 or with a nucleic acid having a sequence complementary to the mRNA.

93. The composition of any of aspects 78 to 92, wherein the nucleic acid probes are conjugated directly or indirectly to a bead.

94. The composition of any of aspects 78 to 92, wherein the bead is a magnetic bead.

95. The composition of any of aspects 78 to 93, wherein the nucleic acid probes are immobilized to a solid support.

96. The composition of aspect 95, wherein the solid support is a glass, plastic or silicon chip.

97 The composition of any of aspects 78 to 96, wherein the nucleic acid probes comprises a sequence as set forth in Table 1.1 or a reverse complementary sequence of any one of them.

98. A kit comprising at least one container or package housing the composition of any one of aspects 78 to 97.

99. A method of processing an RNA sample, the method comprising

(a) obtaining an RNA sample;

(b) determining the expression level of a first mRNA in the RNA sample; and

(c) determining the expression level of a second mRNA in the RNA sample, wherein the expression level of the first mRNA and the second mRNA are determined in biochemically separate assays, and wherein the first mRNA and second mRNA are expressed from genes selected from Table 1 or 11.

100. The method of aspect 99 further comprising determining the expression level of at least one other mRNA in the RNA sample, wherein the expression level of the first mRNA, the second mRNA, and the at least one other mRNA are determined in biochemically separate assays, and wherein the at least one other mRNA is expressed from a gene selected from Table 1 or 11.

101. The method of aspect 99 or 100, wherein the expression levels are determined using a quantitative reverse transcription polymerase chain reaction.

102. A method of processing an RNA sample, the method comprising

(a) obtaining an RNA sample;

(b) determining the expression level of a first mRNA in the RNA sample; and

(c) determining the expression level of a second mRNA in the RNA sample, wherein the expression level of the first mRNA and the second mRNA are determined in biochemically separate assays, and wherein the first mRNA and second mRNA are expressed from genes selected from Table 26.

103. The method of aspect 102 further comprising determining the expression level of at least one other mRNA in the RNA sample, wherein the expression level of the first mRNA, the second mRNA, and the at least one other mRNA are determined in biochemically separate assays, and wherein the at least one other mRNA is expressed from a gene selected from Table 26.

104. The method of aspect 102 or 103, wherein the expression levels are determined using a quantitative reverse transcription polymerase chain reaction.

105. A method of determining the likelihood that a subject has lung cancer, the method comprising
subjecting a biological sample obtained from a subject to a gene expression analysis, wherein the gene expression analysis comprises

(a) determining mR A expression levels in the biological sample of one or more informative-genes that relate to lung cancer status, and

(b) determining mRNA expression levels in the biological sample of one or more genomic correlate genes that relate to one or more self-reportable characteristics of the subject; and

determining a lung cancer risk-score, based on the expression levels determined in (a) and (b), that is indicative of the likelihood that the subject has lung cancer.

106. The method of aspect 105, wherein the one ore more self-reportably characteristics of the subject are selected from : smoking pack years, smoking status, age, and gender.

107. The method of aspect 104 or 105, wherein the informative genes are selected from table 26.

108. The method of any one of aspects 105 to 107, wherein the informative genes comprise at least two genes selected from table 26.

109. The method of any one of aspects 105 to 108, wherein the informative genes comprise at least three genes selected from table 26.

110. The method of any one of aspects 105 to 109, wherein the informative genes comprise at least four genes selected from table 26.

111. The method of any one of aspects 105 to 110, wherein the informative genes comprise at least five genes selected from table 26.

112. The method of any one of aspects 105 to 111, wherein the lung cancer risk-score is determined according to the follow model:

$$x^{score\ 1=} Wo + Wi \times GG + W_2 \times GS + W_3 \times GPY + W_4 \times \text{Reported Age} + W_5 \times CIA + W_6 \times CIB + W_7 \times C2 + W_s \times C3 + W_9 \times C4A + W_w \times CAB,$$

and

$$x^{score\ 2} = W_0 + Wi \times GG + W_2 \times GS + W_3 \times GPY + W_4 \times GA + W_5 \times CIA + W_6 \times CIB + W_7 \times C2 + W_s \times C3 + Wg \times C4A + W_w \times CAB$$

and wherein GG, GS, GPY, CIA, C1B, C2, C3, C4A and C4B are determined according to the equations disclosed herein.

113. The method of any one of aspects 105 to 112, wherein the lung cancer-risk score is determined according to a model having a Negative Predictive Value (NPV) of greater than 90% for ruling out lung cancer in an intended use population.

114. The method of any one of aspects 105 to 113, wherein the lung cancer-risk score is determined according to a model having a Negative Predictive Value (NPV) of greater than 85% for subjects diagnosed with COPD.

115. A method of determining the likelihood that a subject has lung cancer, the method comprising:

subjecting a biological sample obtained from a subject to a gene expression analysis, wherein the gene expression analysis comprises determining mRNA expression levels in the biological sample of at least 2 to at least 10 genes selected from Table 26; and
determining the likelihood that the subject has lung cancer by determining a statistical significance on the mRNA expression levels.

116. The method of aspect 115, wherein the step of determining the statistical significance comprises transforming the expression levels into a lung cancer risk-score that is indicative of the likelihood that the subject has lung cancer.

117. The method of aspect 116, wherein the lung cancer risk-score is the combination of weighted expression levels.

118 The method of aspect 117, wherein the lung cancer risk-score is the sum of weighted expression levels.

119. The method of aspect 116 or 117, wherein the expression levels are weighted by their relative contribution to predicting increased likelihood of having lung cancer

120. A method for determining a treatment course for a subject, the method comprising:

subjecting a biological sample obtained from the subject to a gene expression analysis, wherein the gene expression analysis comprises determining mRNA expression levels in the biological sample of at least 2 to at least 10 genes selected from Table 26;

determining a treatment course for the subject based on the expression levels.

121. The method of aspect 120, wherein the treatment course is determined based on a lung cancer risk-score derived from the expression levels.

122. The method of aspect 121, wherein the subject is identified as a candidate for a lung cancer therapy based on a lung cancer risk-score that indicates the subject has a relatively high likelihood of having lung cancer.

123. The method of aspect 121, wherein the subject is identified as a candidate for an invasive lung procedure based on a lung cancer risk-score that indicates the subject has a relatively high likelihood of having lung cancer.

124. The method of aspect 123, wherein the invasive lung procedure is a transthoracic needle aspiration, mediastinoscopy or thoracotomy.

125. The method of aspect 121, wherein the subject is identified as not being a candidate for a lung cancer therapy or an invasive lung procedure based on a lung cancer risk-score that indicates the subject has a relatively low likelihood of having lung cancer.

126. The method of any one of aspects 115 to 125 further comprising creating a report summarizing the results of the gene expression analysis.

127. The method of any one of aspects 116, 117, 121-123, and 125 further comprising creating a report that indicates the lung cancer risk-score.

128. The method of any one of aspects 116 to 127, wherein the biological sample is obtained from the respiratory epithelium of the subject.

129. The method of aspect 128, wherein the respiratory epithelium is of the mouth, nose, pharynx, trachea, bronchi, bronchioles, or alveoli.

130. The method of one of aspects 116 to 129, wherein the biological sample is obtained using bronchial brushings, broncho-alveolar lavage, or a bronchial biopsy.

131. The method of one of aspects 116 to 130, wherein the subject exhibits one or more symptoms of lung cancer and/or has a lesion that is observable by computer-aided tomography or chest X-ray.

132. The method of aspect 131, wherein, prior to subjecting the biological sample to the gene expression analysis, the subject has not be diagnosed with primary lung cancer.

133. The method of one of aspects 116 to 132, wherein the genes comprise at least two genes selected from the set of genes identified in Table 26.

134. The method of one of aspects 105 to 133, wherein the biological sample obtained from a subject is obtained with a bronchoscopic procedure.

135. The method of one of aspects 105 to 134, wherein the biological sample obtained from a subject is obtained with the cytobrush.

136. A method of determining the likelihood that a subject has lung cancer, the method comprising
subjecting a biological sample obtained from a subject to a gene expression analysis, wherein the gene expression analysis comprises

(a) measuring cDNA levels of one or more informative-genes that relate to lung cancer status, and

(b) measuring cDNA levels of one or more genomic correlate genes that relate to one or more self-reportable characteristics of the subject; and

determining a lung cancer risk-score, based on the cDNA levels determined in (a) and (b), that is indicative of the likelihood that the subject has lung cancer;
wherein the cDNA is prepared from mRNA from the biological sample.

137. The method of one of aspects 1-58, 75-77, or 99-136, wherein the mRNA is converted to cDNA.

138. The method of aspect 137, wherein the cDNA is amplified.

## Claims

1. A method, comprising:

   (a) assaying gene expression levels in a sample obtained from a subject, wherein said subject is suspected of having lung cancer based at least in part on an imaging analysis of said subject showing a suspicious lesion; and
   (b) processing said gene expression levels to determine that said suspicious lesion is associated with or is likely to be associated with said lung cancer in said subject;

   wherein said sample comprises respiratory epithelial cells from a mouth, nose, pharynx, trachea, or bronchi of said subject.

2. The method of claim 1, wherein said imaging analysis comprises computer-aided tomography, magnetic resonance imaging, ultrasonography, or chest X-ray.

3. The method of claim 1, wherein said lung cancer is adenocarcinoma, squamous cell carcinoma, small cell lung cancer, or non-small cell lung cancer.

4. The method of claim 1, wherein said gene expression levels comprise informative gene expression levels that relate to lung cancer status, and genomic correlate gene expression levels that relate to one or more self-reportable characteristics of said subject.

5. The method of claim 4, wherein said one or more self-reportable characteristics of said subject comprise smoking pack years, smoking status, age, or gender.

6. The method of claim 1, wherein said sample is obtained by a process comprising bronchial brushing, broncho-alveolar lavage, bronchial biopsy, oral washing, or sputum collection.

7. The method of claim 1, further comprising creating a report that indicates a lung cancer risk score.

8. The method of claim 7, wherein said subject is identified as a candidate for an invasive lung procedure based at least in part on said lung cancer risk score, wherein said lung cancer risk score indicates that said subject is at risk of having said lung cancer.

9. The method of claim 8, wherein said invasive lung procedure is a transthoracic needle aspiration, mediatinoscopy, or thoracotomy.

10. The method of claim 7, wherein said lung cancer risk score is a combination of weighted expression levels.

11. The method of claim 10, wherein said expression levels are weighted by a relative contribution to predicting an increased likelihood of having said lung cancer.

12. The method of claim 7, wherein said lung cancer risk score is determined using a model having a Negative Predictive Value (NPV) of greater than 90% for ruling out said lung cancer.

13. The method of claim 7, wherein said lung cancer risk score is determined using a model having a Negative Predictive Value (NPV) of greater than 85% for ruling out said lung cancer in subjects diagnosed with chronic obstructive pulmonary disease (COPD).

14. The method of claim 1, wherein said sample is obtained from said nose of said subject.

15. The method of claim 1, wherein said subject is suspected of having lung cancer based at least in part on said subject exhibiting one or more symptoms of said lung cancer, wherein said one or more symptoms of said lung cancer comprise a persistent cough, worsening of an existing cough, blood in sputum of said subject, persistent bronchitis, chest pain, unexplained weight loss, shortness of breath, or wheezing.

FIGURE 1

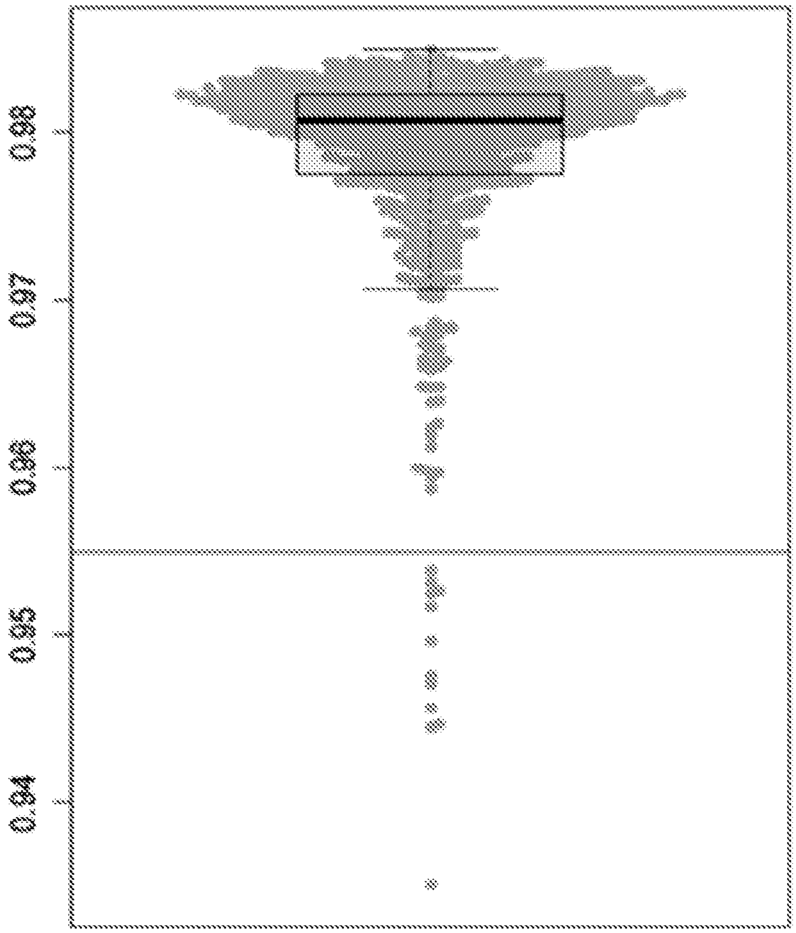

FIGURE 2

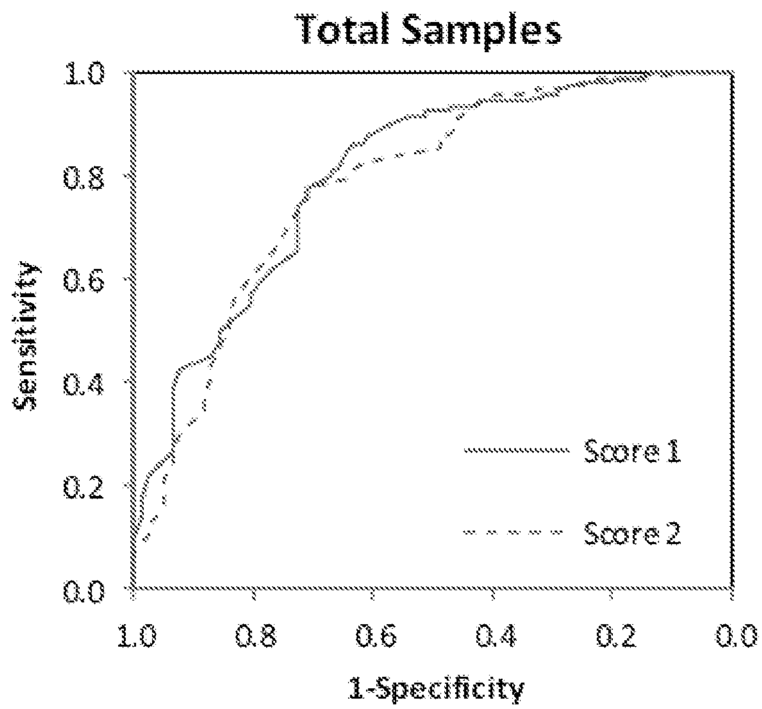
Total Samples
1.0
0.8
0.6
0.4
0.2
0.0
Sensitivity
Score 1
Score 2
1.0
0.8
0.6
0.4
0.2
0.0
1-Specificity

FIGURE 3

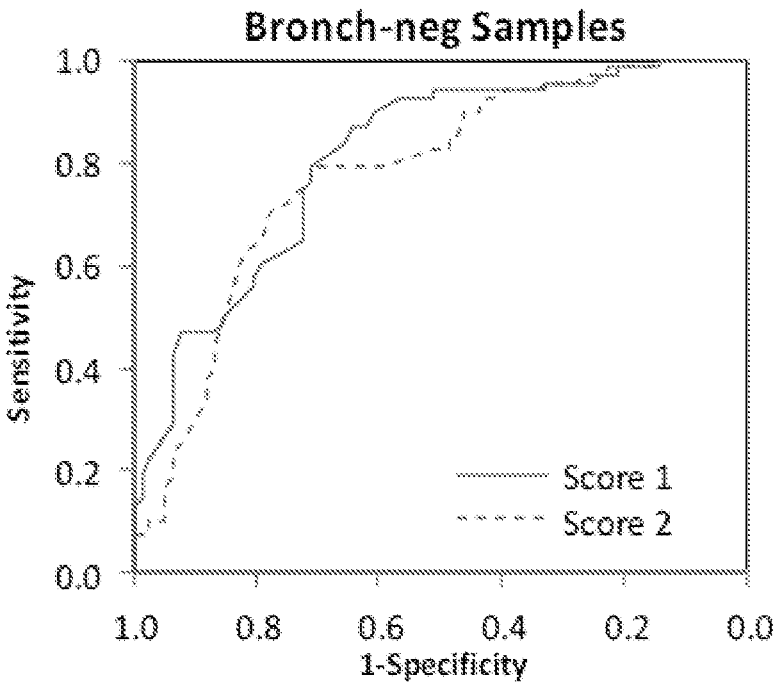
Bronch-neg Samples
Sensitivity
1.0
0.8
0.6
0.4
0.2
0.0
1-Specificity
Score 1
Score 2

FIGURE 4

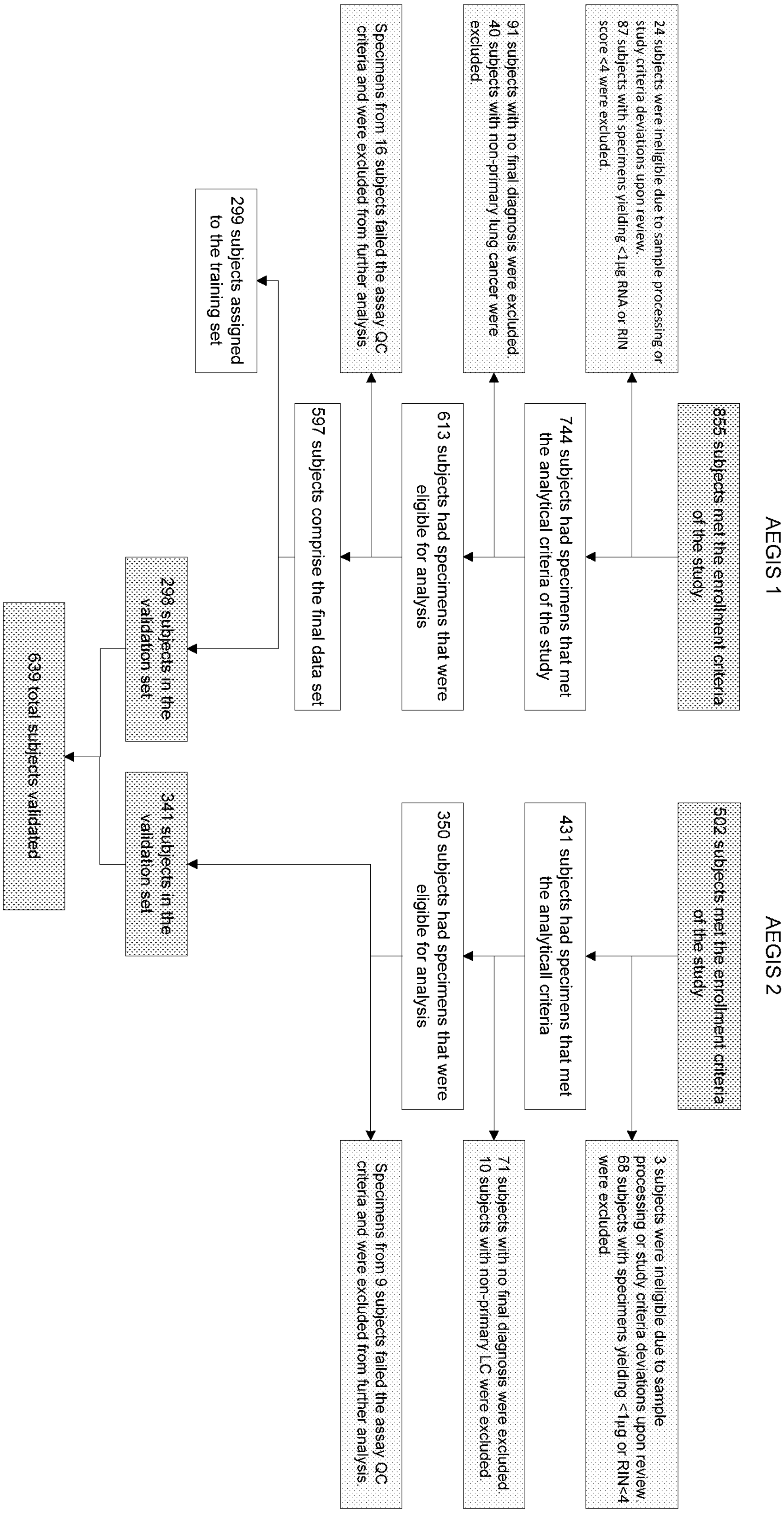
AEGIS 1
855 subjects met the enrollment criteria of the study
24 subjects were ineligible due to sample processing or study criteria deviations upon review. 87 subjects with specimens yielding <1μg RNA or RIN score <4 were excluded.
744 subjects had specimens that met the analytical criteria of the study
91 subjects with no final diagnosis were excluded. 40 subjects with non-primary lung cancer were excluded.
613 subjects had specimens that were eligible for analysis
Specimens from 16 subjects failed the assay QC criteria and were excluded from further analysis.
597 subjects comprise the final data set
299 subjects assigned to the training set
298 subjects in the validation set
AEGIS 2
502 subjects met the enrollment criteria of the study
3 subjects were ineligible due to sample processing or study criteria deviations upon review. 68 subjects with specimens yielding <1μg or RIN<4 were excluded.
431 subjects had specimens that met the analyticall criteria
71 subjects with no final diagnosis were excluded. 10 subjects with non-primary LC were excluded.
350 subjects had specimens that were eligible for analysis
Specimens from 9 subjects failed the assay QC criteria and were excluded from further analysis.
341 subjects in the validation set
639 total subjects validated

FIGURE 5

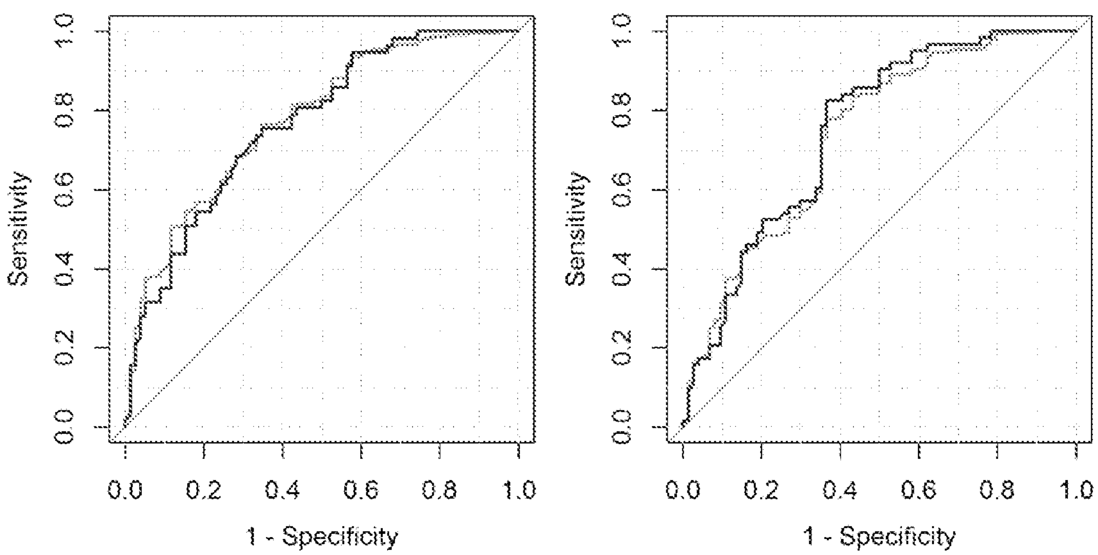
Sensitivity
1 - Specificity
0.0
0.2
0.4
0.6
0.8
1.0
Sensitivity
1 - Specificity
0.0
0.2
0.4
0.6
0.8
1.0

FIGURE 6A

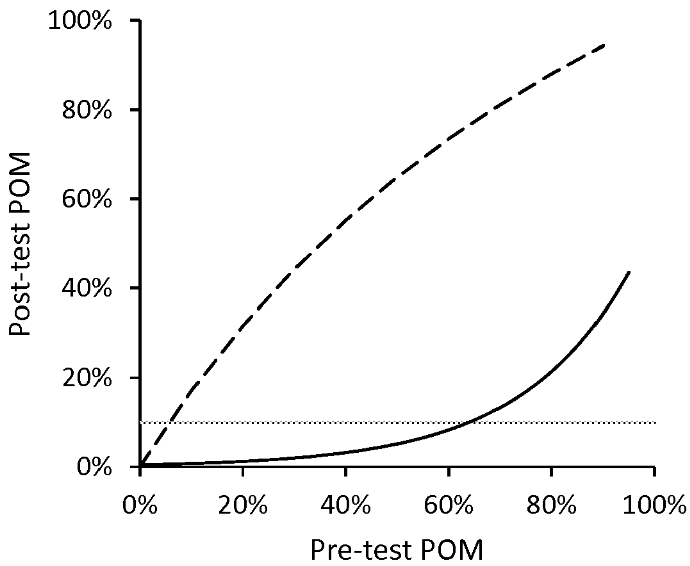

FIGURE 6B

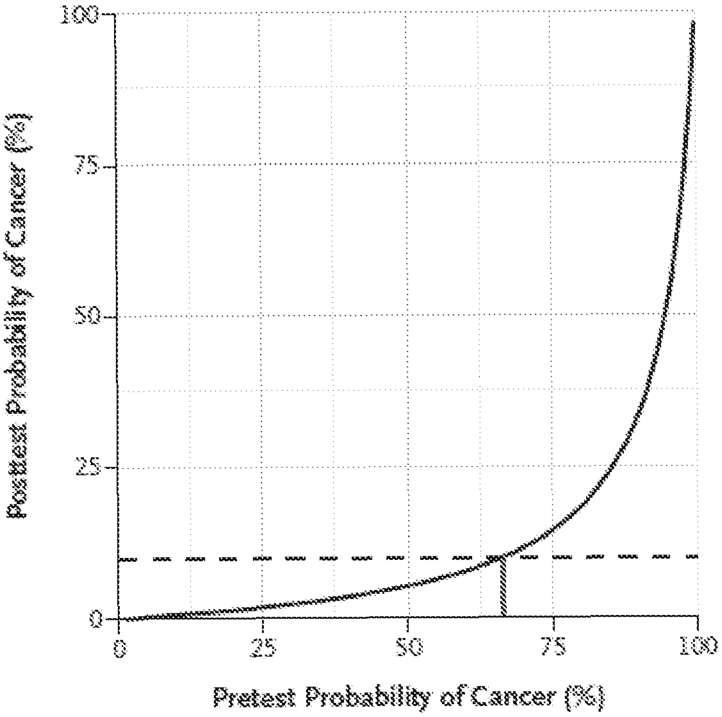
100
75
50
25
0
0
25
50
75
100
Posttest Probability of Cancer (%)
Pretest Probability of Cancer (%)

FIGURE 7

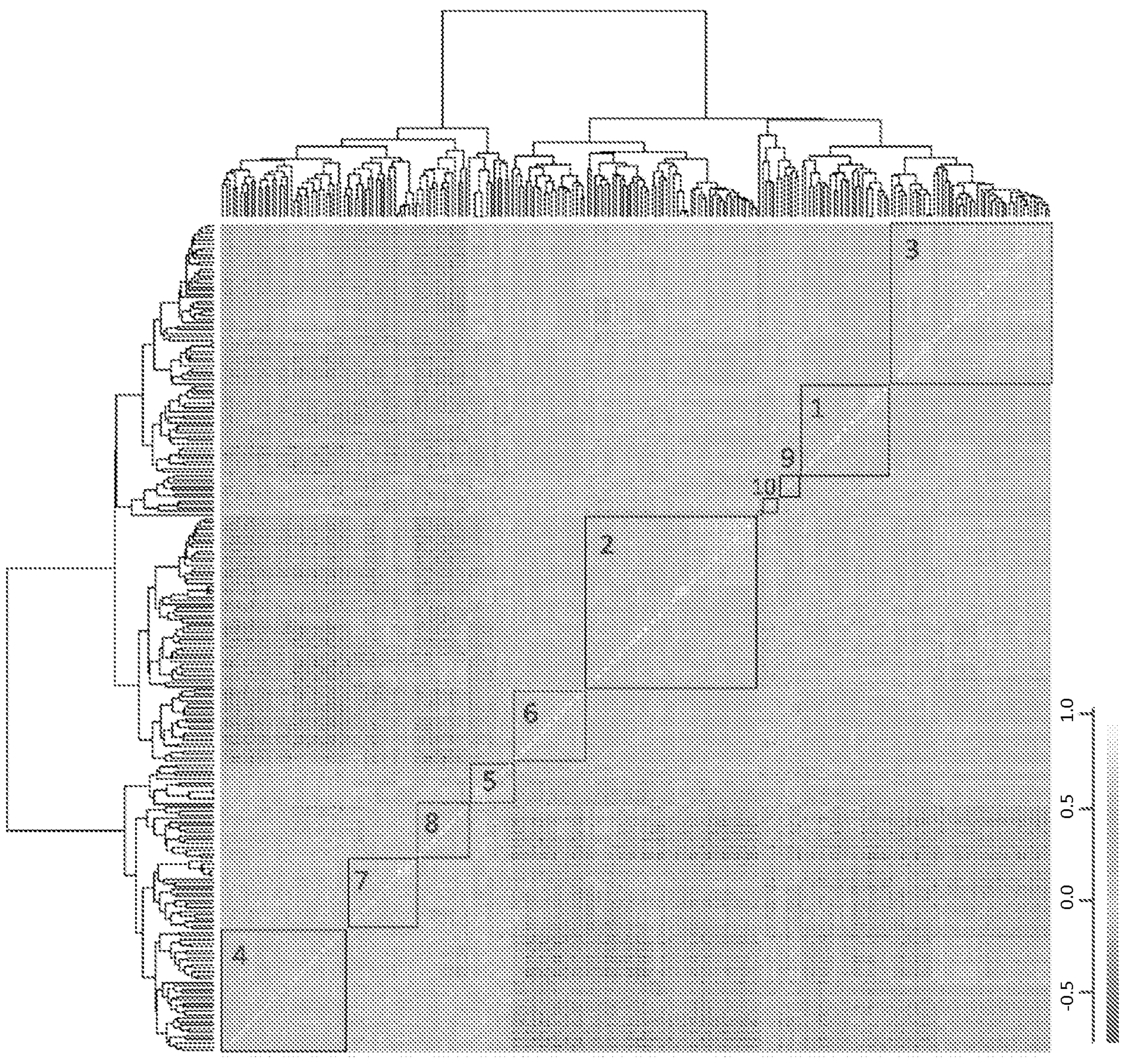

FIGURE 8

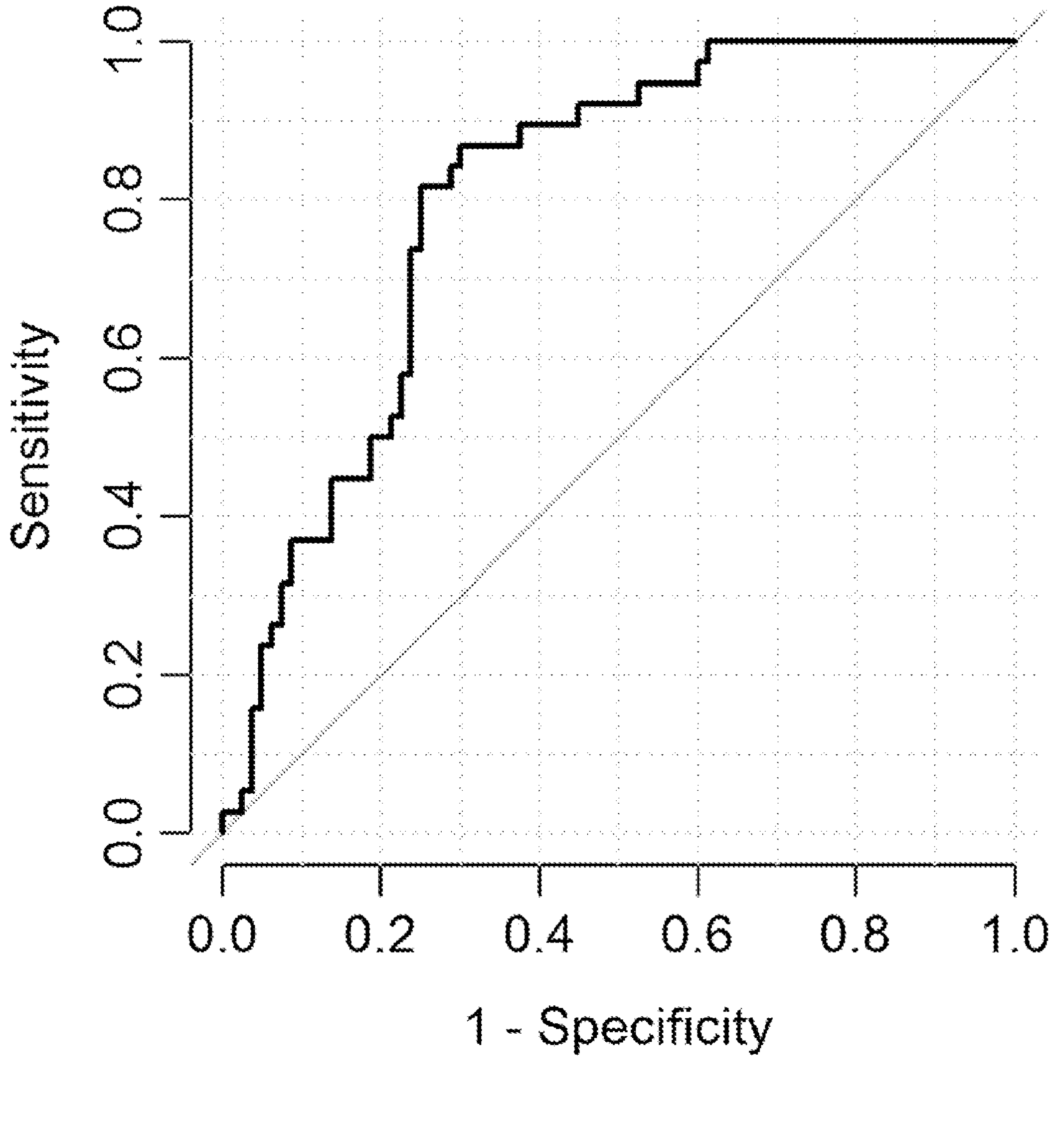

FIGURE 9

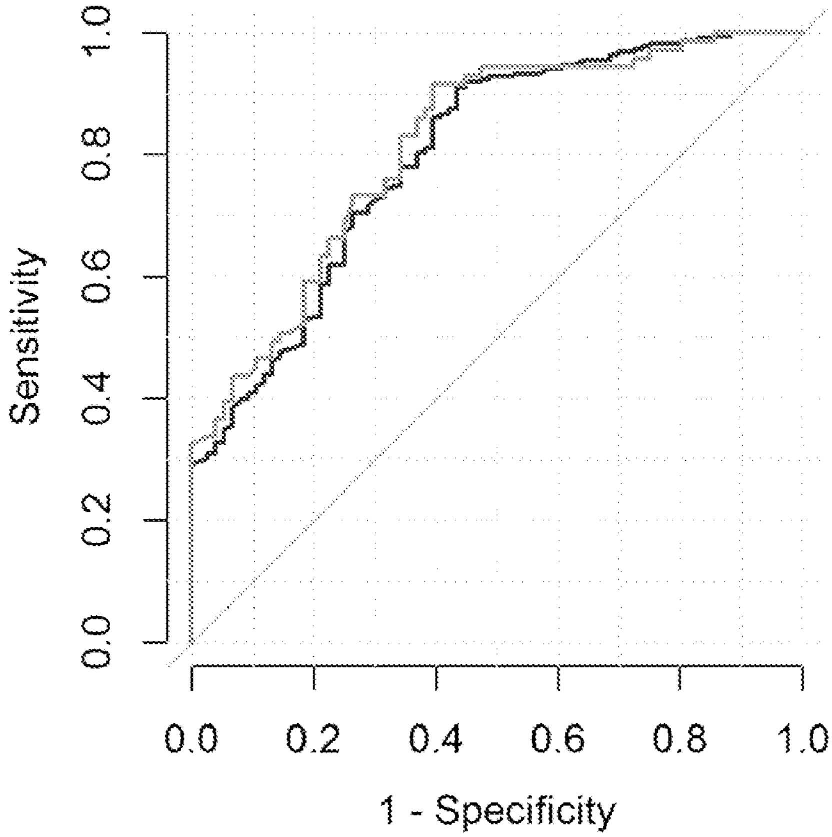

# FIGURE 10A

| Probe | Location |
|---|---|
| gacgtgaaccagaccaatgaagggc | chr19:43866831-43866855 (+) |
| tcccatagggcaagtccggatgctc | chr19:43866648-43866672 (+) |
| agcaggaagggcaaaccactcccca | chr19:43866446-43866470 (+) |
| ccaagtgagagactccaggccctca | chr19:43866388-43866412 (+) |
| aggaggctgcacatcacgcttctca | chr19:43866337-43866361 (+) |
| ggaggctgcacatcacgcttctcac | chr19:43866336-43866360 (+) |
| acaagaagctggaaggttgggccac | chr19:43866275-43866299 (+) |
| aatgctcattttggtggacagccca | chr19:43866241-43866265 (+) |
| ctacatctaggagcagcagcgtctc | chr19:43864533-43864557 (+) |
| agcagcgtctcctgacacacctgcc | chr19:43864519-43864543 (+) |
| tcttgagccaagtttccgtgtgtca | chr19:43864450-43864474 (+) |
| tccgtgtgtcataatggtggtcccc | chr19:43864436-43864460 (+) |
| gttaacgaggttgttgcagaagtcc | chr19:43858486-43858510 (+) |
| ttggagagcaccaggctcacttggg | chr19:43858362-43858386 (+) |
| tctcaatgagcatcaacgtgtcctg | chr19:43858118-43858142 (+) |
| gcaggtcggacaccttccacacatg | chr19:43858043-43858067 (+) |
| agggccagcagtaataccgcgctca | chr19:43857868-43857892 (+) |
| gggccagcagtaataccgcgctcat | chr19:43857867-43857891 (+) |
| gacccgtctgtggctggtaatctct | chr19:43857842-43857866 (+) |

FIGURE 10B

| Probe | Location |
|---|---|
| ttcctgtgtcccattgagcaggttg | chr19:43882867-43882891 (-) |
| tcccattgagcaggttgcaaactgg | chr19:43882875-43882899 (-) |
| cctgaggaggccatcataacagtgt | chr19:43883163-43883187 (-) |
| aggccatcataacagtgtgtggtcc | chr19:43883170-43883194 (-) |

Europäisches Patentamt
European Patent Office
Office européen des brevets

# EUROPEAN SEARCH REPORT

Application Number
EP 21 18 5798

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/000098 A2 (UNIV BOSTON [US]; BRODY JEROME S [US]; SPIRA AVRUM [US]) 6 January 2005 (2005-01-06) * paragraph [0029] - paragraph [0030]; claims 1, 16,17 * | 1-15 | INV. C12Q1/6886 G01N33/574 C40B40/08 |
| X | US 2013/196868 A1 (LEBOWITZ MICHAEL [US] ET AL) 1 August 2013 (2013-08-01) * claims 13,14 * | 1-15 | |
| X | WO 2009/006323 A2 (ABBOTT LAB [US]; COLPITTS TRACEY [US]; RUSSELL ERIC L [US]; FROST STEP) 8 January 2009 (2009-01-08) * claims 1,7,11 * | 1-15 | |
| X | S. P. SHRIVER ET AL: "Sex-Specific Expression of Gastrin-Releasing Peptide Receptor: Relationship to Smoking History and Risk of Lung Cancer", JOURNAL OF THE NATIONAL CANCER INSTITUTE, vol. 92, no. 1, 5 January 2000 (2000-01-05), pages 24-33, XP055445678, GB ISSN: 0027-8874, DOI: 10.1093/jnci/92.1.24 * left-hand column, last paragraph - page 32 * | 1-15 | TECHNICAL FIELDS SEARCHED (IPC) C12Q G01N |

The present search report has been drawn up for all claims

1

| Place of search | Date of completion of the search | Examiner |
|---|---|---|
| The Hague | 21 October 2021 | Gabriels, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 18 5798

This annex lists the patent family members relating to the patent documents cited in the above-mentioned European search report. The members are as contained in the European Patent Office EDP file on
The European Patent Office is in no way liable for these particulars which are merely given for the purpose of information.

21-10-2021

| Patent document cited in search report | Publication date | Patent family member(s) | Publication date |
|---|---|---|---|
| WO 2005000098 A2 | 06-01-2005 | CA 2528572 A1 | 06-01-2005 |
| | | CA 3084542 A1 | 06-01-2005 |
| | | EP 1633892 A2 | 15-03-2006 |
| | | EP 2316973 A1 | 04-05-2011 |
| | | EP 2327795 A1 | 01-06-2011 |
| | | EP 2327796 A1 | 01-06-2011 |
| | | EP 3305919 A1 | 11-04-2018 |
| | | US 2006154278 A1 | 13-07-2006 |
| | | US 2017226591 A1 | 10-08-2017 |
| | | US 2020115763 A1 | 16-04-2020 |
| | | WO 2005000098 A2 | 06-01-2005 |
| US 2013196868 A1 | 01-08-2013 | CA 2799163 A1 | 18-06-2013 |
| | | US 2013196868 A1 | 01-08-2013 |
| | | US 2017276689 A1 | 28-09-2017 |
| | | US 2019178895 A1 | 13-06-2019 |
| WO 2009006323 A2 | 08-01-2009 | CA 2691852 A1 | 08-01-2009 |
| | | CA 2995531 A1 | 08-01-2009 |
| | | EP 2171464 A2 | 07-04-2010 |
| | | EP 2392925 A1 | 07-12-2011 |
| | | EP 2650683 A1 | 16-10-2013 |
| | | EP 3270164 A1 | 17-01-2018 |
| | | ES 2533832 T3 | 15-04-2015 |
| | | ES 2596807 T3 | 12-01-2017 |
| | | ES 2633596 T3 | 22-09-2017 |
| | | HK 1163250 A1 | 07-09-2012 |
| | | JP 5611821 B2 | 22-10-2014 |
| | | JP 6030101 B2 | 24-11-2016 |
| | | JP 2010532480 A | 07-10-2010 |
| | | JP 2015007645 A | 15-01-2015 |
| | | JP 2017049263 A | 09-03-2017 |
| | | US 2008160546 A1 | 03-07-2008 |
| | | US 2012071334 A1 | 22-03-2012 |
| | | US 2015168412 A1 | 18-06-2015 |
| | | US 2016320393 A1 | 03-11-2016 |
| | | WO 2009006323 A2 | 08-01-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62024456
- US 62160403 B
- US 2007148650 A
- US 2009311692 A
- US 88471410
- US 2006154278 A
- US 2010035244 A
- US 86952510
- US 23436808
- US 905897
- US 2009186951 A
- US 2009061454 A
- US 94084010
- US 2010055689 A
- US 1338449 W


### Non-patent literature cited in the description


- **FROHMAN, M.A. et al.** *Proc. Natl. Acad. Sci. USA,* 1988, vol. 85, 8998-9002
- **BELYAYSKY, A. et al.** *Nucleic Acids Research,* 1989, vol. 17, 2919-2932
- **PETRUCCELLI ; CHEN ; NANDRAM.** Applied Statistics for Engineers and Scientists. 1999
- **RICHARD ARNOLD JOHNSON ; DEAN W. WICHERN.** Applied Multivariate Statistical Analysis. Prentice Hall, 02 April 2007
- Nucleic Acid Arrays. Current Protocols In Molecular Biology. John Wiley, 2000
- **LIU CG et al.** An oligonucleotide microchip for genome-wide microRNA profiling in human and mouse tissues. *Proc Nall Acad Sci USA,* 29 June 2004, vol. 101 (26), 9740-4
- **GOULD, M. K. ; DONINGTON, J. ; LYNCH, W. R. et al.** Evaluation of individuals with pulmonary nodules: When is it lung cancer?: Diagnosis and management of lung cancer: American College of Chest Physicians evidence-based clinical practice guidelines. *CHEST Journal,* 2013, vol. 143 (5), e93S-e120S
- **TUKEY, M. H. ; WIENER, R. S.** Population-based estimates of transbronchial lung biopsy utilization and complications. *Respiratory medicine,* 2012, vol. 106 (11), 1559-1565
- **ERNST A ; SILVESTRI G ; JOHNSTONE D.** Interventional Pulmonary Procedures: Guidelines from the American College of Chest Physicians. *Chest,* 2003, vol. 123, 1693-1717
- **RIVERA MP ; MEHTA AC ; WAHIDI MM.** Establishing the Diagnosis of Lung Cancer: Diagnosis and Management of Lung Cancer. *American College of Chest Physicians Evidence-Based Clinical Practice Guidelines. Chest,* 2013, vol. 143, e142S-65S
- **MEMOLI, J. S. W. ; NIETERT, P. J. ; SILVESTRI, G. A.** Meta-analysis of guided bronchoscopy for the evaluation of the pulmonary nodule. *CHEST Journal,* 2012, vol. 142 (2), 385-393
- **OST, D. ; FEIN, A. M. ; FEINSILVER, S. H.** The solitary pulmonary nodule. *New England Journal of Medicine,* 2003, vol. 348 (25), 2535-2542
- **GROGAN, E. L. ; WEINSTEIN, J. J. ; DEPPEN, S. A et al.** Thoracic operations for pulmonary nodules are frequently not futile in patients with benign disease. *Journal of thoracic oncology: official publication of the International Association for the Study of Lung Cancer,* 2011, vol. 6 (10), 1720
- **DETTERBECK, F. C. ; MAZZONE, P. J. ; NAIDICH, D. P. ; BACH, P. B.** Screening for lung cancer: diagnosis and management of lung cancer: American College of Chest Physicians evidence-based clinical practice guidelines. *CHEST Journal,* 2013, vol. 143 (5), e78S-e92S
- **WIENER, R. S. ; WIENER, D. C. ; GOULD, M. K.** Risks of Transthoracic Needle Biopsy: How High?. *Clinical pulmonary medicine,* 2013, vol. 20 (1), 29
- **COVEY, A. M. ; GANDHI, R. ; BRODY, L. A. ; GETRAJDMAN, G. ; THALER, H. T. ; BROWN, K. T.** Factors associated with pneumothorax and pneumothorax requiring treatment after percutaneous lung biopsy in 443 consecutive patients. *Journal of vascular and interventional radiology,* 2004, vol. 15 (5), 479-483
- **GERAGHTY, P. R. ; KEE, S. T. ; MCFARLANE, G. ; RAZAVI, M. K. ; SZE, D. Y. ; DAKE, M. D.** CT-guided Transthoracic Needle Aspiration Biopsy of Pulmonary Nodules: Needle Size and Pneumothorax Rate 1. *Radiology,* 2003, vol. 229 (2), 475-481

- **GOLUB, T. R. ; SLONIM, D. K. ; TAMAYO, P. et al.** Molecular classification of cancer: class discovery and class prediction by gene expression monitoring. *Science,* 1999, vol. 286 (5439), 531-537
- **SPIRA A ; BEANE JE ; SHAH V et al.** Airway epithelial gene expression in the diagnostic evaluation of smokers with suspect lung cancer. *Nature medicine,* 2007, vol. 13, 361-6
- **HANLEY JA ; MCNEIL BJ.** The meaning and use of the area under a receiver operating characteristic (ROC) curve. *Radiology,* 1982, vol. 143, 29-36
- **MACMAHON, H. ; AUSTIN, J. H. ; GAMSU, G. et al.** Guidelines for management of small pulmonary nodules detected on CT scans: a statement from the Fleischner Society 1. *Radiology,* 2005, vol. 237 (2), 395-400
- **BEANE, J. ; SEBASTIANI, P. ; WHITFIELD, T. H. et al.** A prediction model for lung cancer diagnosis that integrates genomic and clinical features. *Cancer Prevention Research,* 2008, vol. 1 (1), 56-64
- **GUSTAFSON, A. M. ; SOLDI, R. ; ANDERLIND, C. et al.** Airway PI3K pathway activation is an early and reversible event in lung cancer development. *Science translational medicine,* 2010, vol. 2 (26), 26ra25-26ra25
- **KADARA, H. ; FUJIMOTO, J. ; YOO, S. Y. ; MAKI et al.** Transcriptomic architecture of the adjacent airway field cancerization in non-small cell lung cancer. *Journal of the National Cancer Institute,* 2014, vol. 106 (3), dju004
- **ALEXANDER, E. K. ; KENNEDY, G. C. ; BALOCH et al.** Preoperative diagnosis of benign thyroid nodules with indeterminate cytology. *New England Journal of Medicine,* 2012, vol. 367 (8), 705-715
- **MCWILLIAMS A ; TAMMEMAGI MC ; MAYO JR et al.** Probability of cancer in pulmonary nodules detected on first screening CT. *N Engl J Med,* 2013, vol. 369, 910-9
- **OST DE ; GOULD MK.** Decision making in patients with pulmonary nodules. *American journal of respiratory and critical care medicine,* 2012, vol. 185, 363-72
- **IRIZARRY, R. A. ; BOLSTAD, B. M. ; COLLIN, F. ; COPE, L. M. ; HOBBS, B. ; SPEED, T. P.** Summaries of Affymetrix GeneChip probe level data. *Nucleic acids research,* 2003, vol. 31 (4), e15-e15
- **JOHNSON WE ; LI C ; RABINOVIC A.** Adjusting batch effects in microarray expression data using empirical Bayes methods. *Biostatistics,* 2007, vol. 8, 118-127
- **HOWLADER N ; NOONE AM ; KRAPCHO M et al.** *SEER stat fact sheets: lung and bronchus, http://seer.cancer.gov/statfacts/html/lungb.html*
- The National Lung Screening Trial Research Team. Reduced Lung-Cancer Mortality with Low-Dose Computed Tomographic Screening. *N Engl J Med,* 2011, vol. 365, 395-409
- **TANOUE LT ; TANNER NT ; GOULD MK ; SILVESTRI GA.** Lung Cancer Screening. *Am J Respir Crit Care Med.,* 04 November 2014
- **RIVERA, M. P. ; MEHTA, A. C. ; WAHIDI, M. M.** Establishing the Diagnosis of Lung Cancer Diagnosis and Management of Lung Cancer, 3rd ed: American College of Chest Physicians Evidence-Based Clinical Practice Guidelines. CHEST Supplement. *Chest,* 2013, vol. 143, e143S
- **SILVESTRI, G. A. ; FELLER-KOPMAN, D. ; CHEN, A. ; WAHIDI, M. ; YASUFUKU, K. ; ERNST, A.** Latest Advances in Advanced Diagnostic and Therapeutic Pulmonary Procedures Update on Pulmonary Procedures. *CHEST Journal,* 2012, vol. 142 (6), 1636-1644
- **GILDEA, T. R. ; MAZZONE, P. J. ; KARNAK, D. ; MEZIANE, M. ; MEHTA, A. C.** Electromagnetic navigation diagnostic bronchoscopy: a prospective study. *American journal of respiratory and critical care medicine,* 2006, vol. 174 (9), 982-989
- **WIENER, R. S. ; SCHWARTZ, L. M. ; WOLOSHIN, S. ; WELCH, H. G.** Population-based risk for complications after transthoracic needle lung biopsy of a pulmonary nodule: an analysis of discharge records. *Annals of internal medicine,* 2011, vol. 155 (3), 137-144
- **FONTAINE-DELARUELLE, C. ; FERRETTI, G. ; GAMONDES, D. ; PRADAT, E. ; SOUQUET, P. J. ; COURAUD, S.** Is transthoracic core needle biopsy under CT scan a good deal for benign diseases' diagnosis?. *European Respiratory Journal,* 2014, vol. 44 (58), 679
- **SMITH MA ; BATTAFARANO RJ ; MEYERS BF ; ZOOLE JB ; COOPER JD ; PATTERSON GA.** Prevalence of benign disease in patients undergoing resection for suspected lung cancer. *The Annals of thoracic surgery,* 2006, vol. 81, 1824-1828
- **BEANE, J. ; SEBASTIANI, P. ; LIU, G. ; BRODY, J. S. ; LENBURG, M. E. ; SPIRA, A.** Reversible and permanent effects of tobacco smoke exposure on airway epithelial gene expression. *Genome Biol,* 2007, vol. 8 (9), R201
- **SPIRA A ; BEANE JE ; SHAH V ; STEILING K ; LIU G ; SCHEMBRI F ; GILMAN S ; DUMAS YM ; CALNER P ; SEBASTIANI P.** Airway epithelial gene expression in the diagnostic evaluation of smokers with suspect lung cancer. *Nature Medicine,* 2007, vol. 13 (3), 361-6
- **TIBSHIRANI, R.** Regression shrinkage and selection via the lasso. *Journal of the Royal Statistical Society. Series B (Methodological),* 1996, 267-288
- **DA WEI HUANG, B. T. S. ; LEMPICKI, R. A.** Systematic and integrative analysis of large gene lists using DAVID bioinformatics resources. *Nature protocols,* 2008, vol. 4 (1), 44-57

- **IRIZARRY, R. A. ; HOBBS, B. ; COLLIN, F. ; BEAZER-BARCLAY, Y. D. ; ANTONELLIS, K. J. ; SCHERF, U. ; SPEED, T. P.** Exploration, normalization, and summaries of high density oligonucleotide array probe level data. *Biostatistics,* 2003, vol. 4 (2), 249-264
- **DAI, M. ; WANG, P. ; BOYD, A. D. ; KOSTOV, G. ; ATHEY, B. ; JONES, E. G. et al.** Evolving gene/transcript definitions significantly alter the interpretation of GeneChip data. *Nucleic acids research,* 2005, vol. 33 (20), e175-e175
- **GOLUB, T. R. ; SLONIM, D. K. ; TAMAYO, P. ; HUARD, C. ; GAASENBEEK, M. ; MESIROV, J. P. et al.** Molecular classification of cancer: class discovery and class prediction by gene expression monitoring. *Science,* 1999, vol. 286 (5439), 531-537
- **KADARA, H. ; FUJIMOTO, J. ; YOO, S. Y. ; MAKI, Y. ; GOWER, A. C. ; KABBOUT, M. et al.** Transcriptomic Architecture of the Adjacent Airway Field Cancerization in Non-Small cell lung cancer. *Journal of the National Cancer Institute,* 2014, vol. 106 (3
- **STEILING, K. ; RYAN, J. ; BRODY, J. S. ; SPIRA, A.** The field of tissue injury in the lung and airway. *Cancer prevention research,* 2008, vol. 1 (6), 396-403
- **BOSSÉ, Y. ; POSTMA, D. S. ; SIN, D. D. ; LAMONTAGNE, M. ; COUTURE, C. ; GAUDREAULT, N. et al.** Molecular signature of smoking in human lung tissues. *Cancer research,* 2012, vol. 72 (15), 3753-3763
- **OTSUBO, C. ; OTOMO, R. ; MIYAZAKI, M. ; MATSUSHIMA-HIBIYA, Y. ; KOHNO, T. ; IWAKAWA, R. et al.** TSPAN2 Is Involved in Cell Invasion and Motility during Lung Cancer Progression. *Cell reports,* 2014, vol. 7 (2), 527-538
- **ØSTER, B. ; THORSEN, K. ; LAMY, P. ; WOJDACZ, T. K. ; HANSEN, L. L. ; BIRKENKAMP-DEMTRÖDER, K. et al.** Identification and validation of highly frequent CpG island hypermethylation in colorectal adenomas and carcinomas. *International journal of cancer,* 2011, vol. 129 (12), 2855-2866
- **KNUDSEN, A. ; MONSTAD, S. E. ; DØRUM, A. ; LØNNING, P. E. ; SALVESEN, H. B. ; DRIVSHOLM, L. et al.** Ri antibodies in patients with breast, ovarian or small cell lung cancer determined by a sensitive immunoprecipitation technique. *Cancer Immunology, Immunotherapy,* 2006, vol. 55 (10), 1280-1284
- **BUCKANOVICH, R. J. ; POSNER, J. B. ; DARNELL, R. B.** Nova, the paraneoplastic Ri antigen, is homologous to an RNA-binding protein and is specifically expressed in the developing motor system. *Neuron,* 1993, vol. 11 (4), 657-672
- **SALEMI, M. ; FRAGGETTA, F. ; GALIA, A. ; PEPE, P. ; CIMINO, L. ; CONDORELLI, R. A. ; CALOGERO, A. E.** Cerebellar degeneration-related autoantigen 1 (CDR1) gene expression in prostate cancer cell lines. *The International journal of biological markers,* 2013, vol. 29 (3), e288-90
- **TANAKA, M. ; TANAKA, K. ; ONODERA, O. ; TSUJI, S.** Trial to establish an animal model of paraneoplastic cerebellar degeneration with anti-Yo antibody: 1. Mouse strains bearing different MHC molecules produce antibodies on immunization with recombinant Yo protein, but do not cause Purkinje cell loss. *Clinical neurology and neurosurgery,* 1995, vol. 97 (1), 95-100
- **FURNEAUX, H. M. ; ROSENBLUM, M. K. ; DALMAU, J. ; WONG, E. ; WOODRUFF, P. ; GRAUS, F. ; POSNER, J. B.** Selective expression of Purkinje-cell antigens in tumor tissue from patients with paraneoplastic cerebellar degeneration. *New England Journal of Medicine,* 1990, vol. 322 (26), 1844-1851
- **SHIH, I. M. ; NESBIT, M. ; HERLYN, M. ; KURMAN, R. J.** A new Mel-CAM (CD146)-specific monoclonal antibody, MN-4, on paraffin-embedded tissue. *Modern pathology: an official journal of the United States and Canadian Academy of Pathology,* 1998, vol. 11 (11), 1098-1106
- **TSUKAMOTO, Y. ; TAIRA, E. ; MIKI, N. ; SASAKI, F.** The role of gicerin, a novel cell adhesion molecule, in development, regeneration and neoplasia. *Histol Histopathol. 2001,* 2001, vol. 16 (2), 563-71
- **TSUKAMOTO, Y. ; TAIRA, E. ; KOTANI, T. ; YAMATE, J. ; WADA, S. ; TAKAHA, N. et al.** Involvement of gicerin, a cell adhesion molecule, in tracheal development and regeneration. *Cell Growth Differ.,* 1996, vol. 7 (12), 1761-1767
- **SCHULZ, C. ; PETRIG, V. ; WOLF, K. ; KRÄTZEL, K. ; KOHLER, M. ; BECKER, B. ; PFEIFER, M.** Upregulation of MCAM in primary bronchial epithelial cells from patients with COPD. *Eur Respir J.,* 2003, vol. 22 (3), 450-6
- **SIMON, G. C. ; MARTIN, R. J. ; SMITH, S. ; THAIKOOTTATHIL, J. ; BOWLER, R. P. ; BARENKAMP, S. J. ; CHU, H. W.** Up-regulation of MUC18 in airway epithelial cells by IL-13: implications in bacterial adherence. *Am J Resp Cell Mol Biol,* 2011, vol. 44 (5), 606-613
- **PENNING, T. M. ; LERMAN, C.** Genomics of smoking exposure and cessation: lessons for cancer prevention and treatment. *Cancer Prevention Research,* 2008, vol. 1 (2), 80-83
- **LAMPE, J. W. ; STEPANIANTS, S. B. ; MAO, M. ; RADICH, J. P. ; DAI, H. ; LINSLEY, P. S. et al.** Signatures of environmental exposures using peripheral leukocyte gene expression: tobacco smoke. *Cancer Epidemiology Biomarkers & Prevention,* 2004, vol. 13 (3), 445-453